# EUROPEAN PATENT SPECIFICATION

(11) **EP 1 555 259 B1**
(45) Date of publication and mention of the grant of the patent: **02.01.2008**
(21) Application number: 05000594.1
(22) Date of filing: 13.01.2005
(51) Int. Cl.: C07D 239/26, C07D 239/38, C07D 241/12, C07D 237/08, C07D 239/28, C07D 239/34, C07D 237/12, C07D 239/30, C07D 237/18, C07D 241/16, C07D 241/18, C07D 253/06, A01N 43/54, A01N 43/58, A01N 43/60, A01N 43/707

(54) **Malononitrile compounds as pesticides**
Malononitrilverbindungen als Pestizide
Dérives de malononitrile comme pesticides

(30) Priority: 16.01.2004 JP 2004009151
(43) Date of publication of application: 20.07.2005
(73) Proprietor: Sumitomo Chemical Company, Limited, Tokyo 104-8260 (JP)
(72) Inventor: Ohshita, Jun, Toyonaka-shi Osaka (JP)
(74) Representative: Vossius & Partner

(56) References cited:
- WO-A-02/089579
- WO-A-20/04006677
- PATENT ABSTRACTS OF JAPAN vol. 1998, no. 06, 30 April 1998 (1998-04-30) -& JP 10 029966 A (MITSUBISHI CHEM CORP), 3 February 1998 (1998-02-03)

## Description

The present invention relates a malononitrile compound having a six-membered ring containing nitrogen atoms and a use thereof.

Compounds having pesticidal activity have been developed and practically used for pest control.

For example, malononitrile derivatives as compounds having pesticidal activity are disclosed in WO 02/089579 and in WO 2004/006677. Malononitrile derivatives having herbicidal activity are disclosed in JP 10-029966.

The object of the present invention is to provide a compound having excellent pesticidal activity, a pesticide composition comprising said compound as an active ingredient and a method for controlling pests by applying said compound.

The present invention is a malononitrile compound represented by the formula (I) : 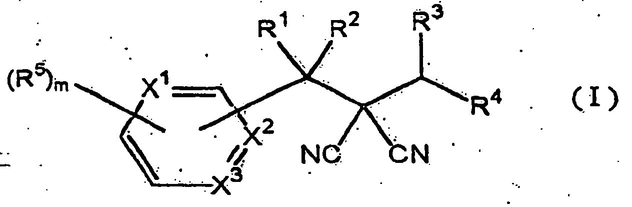 wherein, in the formula,
R¹ represents a C1-C5 alkyl group optionally substituted by one or more halogen atoms, a C2-C5 alkenyl group optionally substituted by one or more halogen atoms, a C2-C5 alkynyl group optionally substituted by one or more halogen atoms or a hydrogen atom;
R² represents a C1-C5 alkyl group optionally substituted by one or more halogen atoms, a C1-C5 alkoxy group optionally substituted by one or more halogen atoms, a C2-C5 alkenyl group optionally substituted by one or more halogen atoms, a C2-C5 alkynyl group optionally substituted by one or more halogen atoms, a cyano group or a hydrogen atom;
each of R³ and R⁴ represents a C1-C5 alkyl group optionally substituted by one or more halogen atoms, a C2-C5 alkenyl group optionally substituted by one or more halogen atoms, a C2-C5 alkynyl group optionally substituted by one or more halogen atoms, a C3-C5 cycloalkyl group optionally substituted by one or more halogen atoms, a C5 -C6 cycloalkenyl group optionally substituted by one or more halogen atoms or a hydrogen atom,
or represents a C2-C6 alkanediyl group optionally substituted by one or more halogen atoms or a C4-C6 alkenediyl group optionally substituted by one or more halogen atoms in each of which R³ and R⁴ couple one another at the ends thereof;
each of X¹, X² and X³ represents a nitrogen atom or CR⁶, with the proviso that two or three of X¹, X² and X³ represent a nitrogen atom;
R⁶ represents a hydrogen atom or R⁵;
each of R⁵s represents a halogen atom, a cyano group, a nitro group, a hydroxyl group, a mercapto group, a formyl group, a SF₅ group, a carboxyl group, a C1-C5 alkyl group optionally substituted by one or more halogen atoms, a C2-C5 alkenyl group optionally substituted by one or more halogen atoms, a C2-C5 alkynyl group optionally substituted by one or more halogen atoms, a C3-C5 cycloalkyl group optionally substituted by one or more halogen atoms, a C1-C5 alkoxy group optionally substituted by one or more halogen atoms, a C3-C6 alkenyloxy group optionally substituted by one or more halogen atoms, a C3-C6 alkynyloxy group optionally substituted by one or more halogen atoms, a C1-C5 alkylthio group optionally substituted by one or more halogen atoms, a C3-C5 alkenylthio group optionally substituted by one or more halogen atoms, a C3-C5 alkynylthio group optionally substituted by one or more halogen atoms, a C1-C5 alkylsulfinyl group optionally substituted by one or more halogen atoms, a C1-C5 alkylsulfonyl group optionally substituted by one or more halogen atoms, a C2-C6 alkylcarbonyl group optionally substituted by one or more halogen atoms, a C2-C5 alkoxycarbonyl group optionally substituted by one or more halogen atoms, a group designated by ―NR¹⁰R¹¹, a group designated by ―C(=X⁵)NR¹²R¹³ or a group designated by ―C(=NOR¹⁴)R¹⁵, or
where m is 2 and two R⁵s are coupled to adjacent carbon atoms. may represent a C3-C6 alkanediyl group or a C3-C6 alkenediyl group each of which is formed by coupling of the two R⁵s at the ends thereof, furthermore, in this condition, a methylene group contained in said C3-C6 alkanediyl group or said C3-C6 alkenediyl group may be replaced by O, S(O)ᵣ or NR¹⁶ and a carbon atom contained in said C3-C6 alkanediyl group or said C3-C6 alkenediyl group may be coupled with a halogen atom or a C1-C3 alkoxy group optionally substituted by one or more halogen atoms instead of hydrogen atom(s);
each of R¹⁰ and R¹¹ represents a C1-C5 alkyl group optionally substituted by one or more halogen atoms, a C3-C5 alkenyl group optionally substituted by one or more halogen atoms, a C3-C5 alkynyl group optionally substituted by one or more halogen atoms, a C3-C5 cycloalkyl group optionally substituted by one or more halogen atoms, a (C1-C5 alkoxy optionally substituted by one or more halogen atoms) C1-C3 alkyl group, a C1-C5 alkylsulfonyl group optionally substituted by one or more halogen atoms, a C2-C6 alkylcarbonyl group optionally substituted by one or more halogen atoms, a C2-C5 alkoxycarbonyl group optionally substituted by one or more halogen atoms or a hydrogen atom, or represents a C2-C6 alkanediyl group optionally substituted by one or more halogen atoms or a C4-C6 alkenediyl group optionally substituted by one or more halogen atoms in each of which R¹⁰ and R¹¹ are coupled with one another at the ends thereof; each of R¹² and R¹³ represents a C1-C5 alkyl group optionally substituted by one or more halogen atoms, a C3-C5 alkenyl group optionally substituted by one or more halogen atoms, a C3-C5 alkynyl group optionally substituted by one or more halogen atoms , a C3-C5 cycloalkyl group optionally substituted by one or more halogen atoms, a group designated by - ( CH₂) ₙQ¹ or a hydrogen atom,
or represents a C2-C6 alkanediyl group optionally substituted by one or more halogen atoms or a C4 -C6 alkenediyl group optionally substituted by one or more halogen atoms in each of which R¹² and R¹³ are coupled with one another at the ends thereof;

each of R¹⁴ and R¹⁵ represents a C1-C5 alkyl group optionally substituted by one or more halogen atoms, a C3-C5 alkenyl group optionally substituted by one or more halogen atoms, a C3-C5 alkynyl group optionally substituted by one or more halogen atoms, a C3-C5 cycloalkyl group optionally substituted by one or more halogen atoms, a group designated by -(CH₂)ₙQ² or a hydrogen atom;
each of Q¹ and Q² represents a phenyl group which may be substituted by a group selected from the group consisting of a C1-C5 alkyl group optionally substituted by one or more halogen atoms, a C1-C5 alkoxy group optionally substituted by one or more halogen atoms, a C1-C5 alkylthio group optionally substituted by one or more halogen atoms and a halogen atom;
R¹⁶ represents a C1-C3 alkyl group; m represents an integer of from 0 to 2; n represents an integer of from 0 to 3; r represents an integer of from 0 to 2; and
X⁵ represents an oxygen atom or a sulfur atom.

Said malononitrile compound is referred to as "the compound of the present invention" hereinafter. The present invention further providesa pesticide composition comprising the compound of the present invention as an active ingredient, a method for controlling pests comprising applying an effective amount of the compound of the present invention to pests or at a habitat of pests and use of the compound of the present invention for pest control.

In the present invention, "alkanediyl group" represents a group having a free valency on two different carbon atoms contained in a saturated hydrocarbon chain, and "alkenediyl group" represents a group having a free valency on two different carbon atoms contained in a hydrocarbon chain having one or two double bonds.

In the compound of the present invention:
a C1-C5 alkyl group optionally substituted by one or more halogen atoms represented by R¹, R², R³ , R⁴ , R⁵ , R¹⁰, R¹¹, R¹² , R¹³ , R¹⁴ and R¹⁵ includes, for example, a methyl group, an ethyl group, a propyl group, an isopropyl group, a t-butyl group, a 2, 2-dimethylpropyl group, a chloromethyl group, a fluoromethyl group, a difluoromethyl group, a trifluoromethyl group, a 2,2,2-trifluoroethyl group and a 1,1,2,2-tetrafluoroethyl group;
a C2-C5 alkenyl group optionally substituted by one or more halogen atoms represented by R¹, R², R³, R⁴ and R⁵ includes, for example, a vinyl group, a 2,2-difluorovinyl group, a 1,2,2-trifluorovinyl group, a 1-propenyl group and an allyl group;
a C2-C5 alkynyl group optionally substituted by one or more halogen atoms represented by R¹, R², R³, R⁴ and R⁵ includes, for example, an ethynyl group, a 1-propynyl group, a propargyl group and a 3,3,3-trifluoro-1-propynyl group;
a C1-C5 alkoxy group optionally substitutedby one or more halogen atoms represented by R² and R⁵ includes, for example, a methoxy group, an ethoxy group, a 1-methylethoxy group, a trifluoromethoxy group, a difluoromethoxy group, a
2,2,2-trifluoroethoxy group and a 1,1,2,2-tetrafluoroethoxy group;
a C3-C5 cycloalkyl group optionally substituted by one or more halogen atoms represented by R³ , R⁴ , R⁵ , R¹⁰, R¹¹, R¹², R¹³ , R¹⁴ and R¹⁵ includes, for example, a cyclopropyl group, a 2,2-dichlorocyclopropyl group, a2,2-difluorocyclopropylgroup, a 2,2,3,3-tetrafluorocyclopropyl group, a 2,2-dichlorocyclobutyl group, a 2,2-difluorocyclobutyl group, a 2 , 2, 3, 3-tetrafluorocyclobutyl group, a cyclobutyl group and a cyclopentyl group;
a C5-C6 cycloalkenyl group optionally substituted by one or more halogen atoms represented by R³ and R⁴ includes, for example, a 2-fluoro-2-cyclopentenyl group;
a C2-C6 alkanediyl group optionally substituted by one or more halogen atoms represented by the combination of R³ and R⁴, that of R¹⁰ and R¹¹, and that of R¹² and R¹³ includes, for example, an ethylene group, a propylene group, a trimethylene group and a tetramethylene group;
a C4-C6 alkenediyl group optionally substituted by one or more halogen atoms represented by the combination of R³ and R⁴, that of R¹⁰ and R¹¹, and that of R¹² and R¹³ includes, for example, a 2-butene-1,4-diyl group and a 2-pentene-1,5-diyl group; a C3-C6 alkenyloxy group optionally substituted by one or more halogen atoms represented by R⁵ includes, for example, an allyloxy group, a 1-methyl-2-propenyloxy group, a 1,1-dimethyl-2-propenyloxy group, a 2-methyl-2-propenyloxy group, a1-butenyloxygroup, a2-butenyloxygroup, a3-butenyloxy group, a1-chloro-2-propenyloxy group, a1-bromo-2-propenyloxy group, a 2-chloro-2-propenyloxy group and a 3,3-dichloro-2-propenyloxy group;
a C3-C6 alkynyloxy group optionally substituted by one or more halogen atoms represented by R⁵ includes, for example, a propargyloxy group, a 2-butynyloxy group, a 3-butynyloxy group, a 2-pentynyloxy group, a 3-pentynyloxy group, a 3-chloro-2-propynyloxy group, a 3-bromo-2-propynyloxy group, a 1-methyl-2-propynyloxy group, a 4-chloro-2-butynyloxy group, a 4,4-difluoro-2-butynyloxy group, a 4,4,4-trifluoro-2-butynyloxy group and 1,1-dimethyl-2-propynyloxy group;
a C1-C5 alkylthio group optionally substituted by one or more halogen atoms represented by R⁵ includes, for example, a methylthio group, a trifluoromethylthio group, a 2,2,2-trifluoroethylthio group and a 1,1,2,2-tetrafluoroethyltio group;
a C3-C5 alkenylthio group optionally substituted by one or more halogen atoms represented by R⁵ includes, for example, a 1-propenylthio group, a 2-propenylthio group and a 2,2-difluoro-2-propenylthio group;
a C3-C5 alkynylthio group optionally substituted by one or more halogen atoms represented by R⁵ includes, for example, a propargylthio group, a 2-butynylthio group and a 3,3,3-trifluoro-1-propynylthio group;
a C1-C5 alkylsulfinyl group optionally substituted by one or more halogen atoms represented by R⁵ includes, for example, a methylsulfinyl group and a trifluoromethylsulfinyl group;
a C1-C5 alkylsulfonyl group optionally substituted by one or more halogen atoms represented by R⁵, R¹⁰ and R¹¹ includes, for example, a methylsulfonyl group and a trifluoromethylsulfonyl group;
a C2-C6 alkylcarbonyl group optionally substituted by one or more halogen atoms represented by R⁵, R¹⁰ and R¹¹ includes, for example, an acetyl group, a propionyl group and a trifluoroacetyl group;
a C2-C5 alkoxycarbonyl group optionally substituted by one or more halogen atoms represented by R⁵, R¹⁰ and R¹¹ includes, for example, a methoxycarbonyl group and a 2,2,2-trifluoroethoxycarbonyl group;
a C3-C5 alkenyl group optionally substituted by one or more halogen atoms represented by R¹⁰, R¹¹, R¹² , R¹³, R¹⁴ and R¹⁵ includes, for example, a 1-propenyl group and an allyl group;
a C3-C5 alkynyl group optionally substituted by one or more halogen atoms represented by R¹⁰, R¹¹, R¹², R¹³, R¹⁴ and R¹⁵ includes, for example, a 1-propynyl group, a propargyl group and a 3,3,3-trifluoro-1-propynyl group;
a (C1-C5 alkoxy optionally substituted by one or more halogen atoms) C1-C3 alkyl group represented by R¹⁰ and R¹¹ includes, for example, a methoxymethyl group, an ethoxymethyl group, a 1-methoxyethyl group, a 1-ethoxyethyl group and a trifluoromethoxymethyl group;
where two R⁵s are coupled to two adjacent carbon atoms, a C3-C6 alkanediyl group formed by coupling said two R⁵s includes, for example, a trimethylene group, a propylene group, a 2,2-dimethylpropane-1,4-diyl group, a 1,1-dimethylpropane-1,3-diyl group and a tetramethylene group; where two R⁵s are coupled to two adjacent carbon atoms, a C3-C6 alkenediyl group formed by coupling said two R⁵s includes, for example, a 2-butene-1,4-diyl group and a 2-pentene-1,5-diyl group; and
a C1-C3 alkyl group represented by R¹⁶ includes, for example, a methyl group, an ethyl group and a propyl group.

A method for producing the compound of the present invention is described hereinafter.

The compound of the present invention can be produced, for example, according to the following (Production Method 1) to (Production Method 3).

### (Production Method 1)

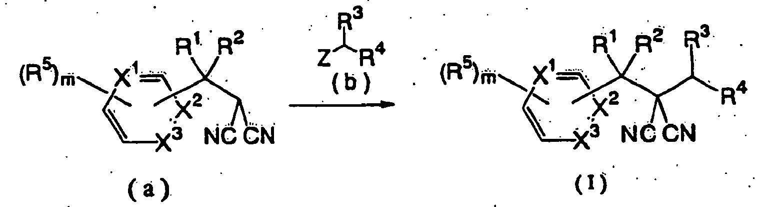

[In the formula, R¹ , R² , R³ , R⁴ , R⁵ , X¹ , X² and X⁵ have the same meaning as described above, and Z represents a halogen atom, a methanesulfonyloxy group, a trifluoromethanesulfonyloxy group or a toluenesulfonyloxy group.]

The reaction is usually carried out in a solvent under the presence of a base.
The solvent used for the reaction includes, for example, amides such as N,N-dimethylformamide and the like, ethers such as diethylether, tetrahydrofuran and the like, organic sulfurs such as dimethylsulfoxide, sulfolane and the like, halogenated hydrocarbons such as 1,2-dichloroethane, chlorobenzene and the like, aromatic hydrocarbons such as toluene, xylene and the like, and mixtures thereof.
The base used for the reaction includes, for example, inorganic bases such as sodium hydride, sodium carbonate, potassium carbonate and the like, alkali metal alkoxides such as potassium-t-butoxide and the like, and organic bases such as 4-(dimethylamino)pyridine, 1,4-diazabicyclo[2.2.2]octane, 1,8-diazabicyclo[5.4.0]-7-undecene and the like.

The amount of the base used for the reaction is usually 1 to 10 moles per 1 mole of the compound (a).

The amount of the compound (b) used for the reaction is usually 1 to 10 moles per 1 mole of the compound (a).

The reaction temperature of the reaction is usually in the range of -20 to 100 °C, and the reaction time is usually in the range of 1 to 24 hours.

After the reaction has finished, the compound of the present invention represented by the formula (I) can be isolated by subjecting the reaction mixture to ordinary post-treatment such as pouring the reaction mixture into water, extracting with an organic solvent, followed by concentrating the extract. The isolated compound represented by the formula (I) of the present invention may be, if required, purified by chromatography, recrystallization and the like.

### (Production Method 2)

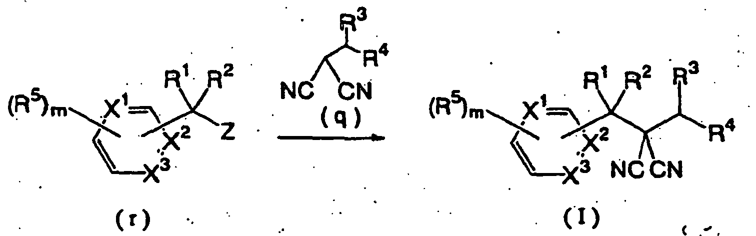

[In the formula, R¹ , R² , R³ , R⁴ , R⁵ , X¹ , X² and X⁵ have the same meaning as described above, and Z represents a halogen atom, a methanesulfonyloxy group, a trifluoromethanesulfonyloxy group or a toluenesulfonyloxy group.]

The reaction is usually carried out in a solvent under the presence of a base.
The solvent used for the reaction includes, for example, amides such as N,N-dimethylformamide and the like, ethers such as diethylether, tetrahydrofuran and the like, organic sulfurs such as dimethylsulfoxide, sulfolane and the like, halogenated hydrocarbons such as 1,2-dichloroethane, chlorobenzene and the like, aromatic hydrocarbons such as toluene, xylene and the like, and mixtures thereof.

The base used for the reaction includes, for example, inorganic bases such as sodium hydride, sodium carbonate, potassium carbonate and the like, alkali metal alkoxides such as potassium-t-butoxide and the like, and organic bases such as 4-(dimethylamino)pyridine, 1,4-diazabicyclo[2.2.2]octane, 1,8-diazabicyclo[5.4.0]-7-undecene and the like.

The amount of the base used for the reaction is usually 1 to 10 moles per 1 mole of the compound (q).

The amount of the compound (r) used for the reaction is usually 1 to 10 moles per 1 mole of the compound (q).

The reaction temperature of the reaction is usually in the range of -20 to 100 °C, and the reaction time is usually in the range of 1 to 24 hours.

After the reaction has finished, the compound of the present invention represented by the formula (I) can be isolated by subjecting the reaction mixture to ordinary post-treatment such as pouring the reaction mixture into water, extracting with an organic solvent, followed by concentrating the extract. The isolated compound represented by the formula (I) of the present invention may be, if required, purified by chromatography, recrystallization and the like.

### (Production Method 3)

Among the compounds of the present invention, the compound (I-a) may be also produced by the following method. 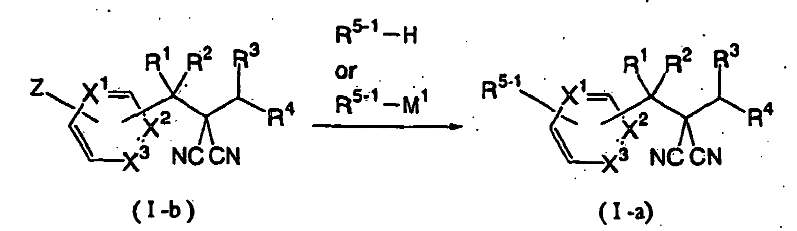 [In the formula, R¹, R², R³ , R⁴, X¹ , X² , X³ and Z have the same meaning as described above; R⁵⁻¹ represents a cyano group, a C1-C5 alkoxy group optionally substituted by one or more halogen atoms, a C3-C5 alkenyloxy group optionally substituted by one or more halogen atoms, a C3-C5 alkynyloxy group optionally substituted by one or more halogen atoms, a C1-C5 alkylthio group optionally substituted by one or more halogen atoms, a C3-C5 alkenylthio group optionally substituted by one or more halogen atoms, a C3-C5 alkynylthio group optionally substituted by one or more halogen atoms or a group represented by -NR¹⁰R¹¹; R¹⁰, and R¹¹ have the same meaning described above; and M¹ represents a sodium atom or potassium atom.]

The reaction is usually carried out in a solvent with or without the presence of a base.

The solvent used for the reaction includes, for example, amides such as N,N-dimethylformamide and the like, ethers such as diethylether, tetrahydrofuran and the like, organic sulfurs such as dimethylsulfoxide, sulfolane and the like, halogenated hydrocarbons such as 1, 2-dichloroethane , chlorobenzene and the like, aromatic hydrocarbons such as toluene, xylene and the like, and the mixture thereof.

The base used for the reaction includes, for example, inorganic bases such as sodium hydride, sodium carbonate, potassium carbonate and the like, alkali metal alkoxides such as potassium-t-butoxide and the like, and organic bases such as 4-(dimethylamino)pyridine, 1,4 - diazabicyclo[2.2.2]octane, 1,8-diazabicyclo[5.4.0]-7-undecene and the like. The amount of the base used for the reaction is usually 1 to 10 moles per 1 mole of the compound (I-b).

The amount of R⁵⁻¹-H or R⁵⁻¹-M¹ used for the reaction is usually 1 to 10 moles per 1 mole of the compound (I-b).

The reaction temperature of the reaction is usually in the range of -20 to 100 °C, and the reaction time is usually in the range of 1 to 24 hours.

After the reaction has finished, the compound of the present invention represented by the formula (I-a) can be isolated by subjecting the reaction mixture to ordinary post-treatment such as pouring the reaction mixture into water, extracting with an organic solvent, followed by concentrating the extract. The isolated compound (I-a) may be, if required, purified by chromatography, recrystallization and the like.

The compound (a) can be produced, for example, by the following method.

### (Referential Production Method 1)

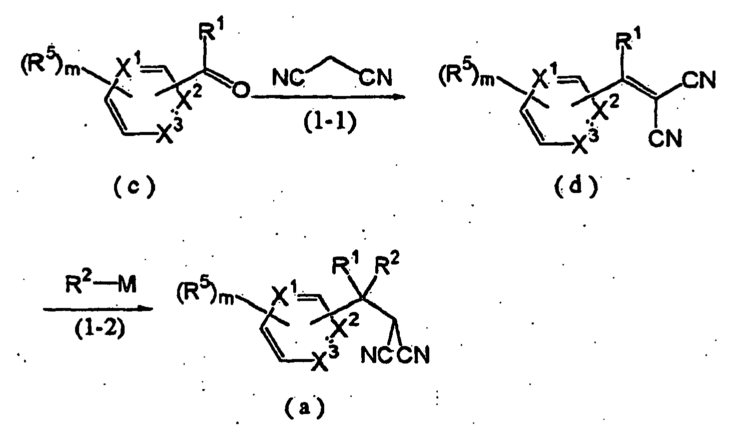

[In the formula, R¹, R², R⁵ , X¹ , X², X³ and m have the same meaning as described above, and M represents MgI, MgBr, MgCl, Li, K, Na or Cu.]

### Step (1-1)

The compound (d) can be produced by subjecting the compound (c) to reaction with malononitrile.

The reaction is usually carried out in a solvent. The solvent used for the reaction includes, for example, amides such as N,N-dimethylformamide and the like, ethers such as diethylether, tetrahydrofuran and the like, halogenated hydrocarbons such as chloroform, 1,2-dichloroethane, chlorobenzene and the like, aromatic hydrocarbons such as toluene, xylene and the like, alcohols such as methanol, ethanol, isopropanol and like, and the mixture thereof.

The reaction is, if required, carried out under the presence of a base, and the base used includes, for example, tetrabutyl ammonium hydroxide and the like.

The amount of the base used for the reaction is usually 0.01 to 0.5 moles per 1 mole of the compound (c).

The amount of the malononitrile used for the reaction is usually 1 to 10 moles per 1 mole of the compound (c).

The reaction temperature of the reaction is usually in the range of -20 to 200 °C, and the reaction time is usually in the range of 1 to 24 hours.

The reaction may be, if required, performed along with removing the water generated by the reaction out of the reaction system.

After the reaction has finished, the compound (d) can be isolated by subjecting the reaction mixture to ordinary post-treatment such as pouring the reaction mixture into water, extracting with an organic solvent, followed by concentrating the extract. The isolated compound (d) may be, if required, purified by chromatography, recrystallization and the like.

### Step (1-2)

(1) In the case that R² represents a C1-C5 alkyl group optionally substituted by one or more halogen atoms, a C2-C5 alkenyl group optionally substituted by one or more halogen atoms, a C2-C5 alkynyl group optionally substituted by one or more halogen atoms.
   The compound (a) can be produced by subjecting the compound (d) to reaction with an organometallic compound (R²-M : wherein M represents MgI, MgBr, MgCl or Li.).
   The reaction is usually carried out in a solvent, if required, under the presence of a copper salt.
   The solvent used for the reaction includes, for example, ethers such as diethylether, tetrahydrofuran and the like, aromatic hydrocarbons such as toluene, xylene and the like, and mixtures thereof.
   R²-M used for the reaction includes, for example, an organic magnesium compound such as methyl magnesium iodide, ethyl magnesium bromide, isopropyl magnesium bromide, vinyl magnesium bromide, ethynyl magnesium bromide, dimethyl magnesium and the like, an organic lithium compound such as methyl lithium and the like, an organic zinc compound such as diethyl zinc and the like, and an organic copper compound such as trifluoromethyl copper and the like.
   The amount of the organometallic compound (R²-M) used for the reaction is usually 1 to 10 moles per 1 mole of the compound (d).
   The copper salt used for the reaction includes, for example, copper iodide (I), copper bromide (I) and the like.
   The amount of the copper salt used for the reaction is usually 1 mole or less per 1 mole of the compound (d).
   The reaction temperature of the reaction is usually in the range of -20 to 100 °C, and the reaction time is usually in the range of 1 to 24 hours.
   After the reaction has finished, the compound (a) can be isolated by subjecting the reaction mixture to ordinary post-treatment such as pouring the reaction mixture into water, extracting with an organic solvent, followed by concentrating the extract. The isolated compound (a) may be, if required, purified by chromatography, recrystallization and the like.
(2) In the case that R² represents a hydrogen atom.
   The compound (a) can be produced by subjecting the compound (d) to reductive reaction.
   The reduction reaction is usually carried out in a solvent.
   The solvent used for the reaction includes, for example, ethers such as diethylether, tetrahydrofuran and the like, aromatic hydrocarbons such as toluene, xylene and the like, alcohols such as methanol, ethanol, isopropanol and like, and mixtures thereof.
   The reducing agent used for the reaction includes, for example, sodium borohydride.
   The amount of the reducing agent used for the reaction is usually 0.25 to 2 moles per 1 mole of the compound (d).
   The reaction temperature of the reaction is usually in the range of 0 to 50 °C, and the reaction time is usually in the range of an instant to 24 hours.
   After the reaction has finished, the compound (a) can be isolated by subjecting the reaction mixture to ordinary post-treatment such as pouring the reaction mixture into water, extracting with an organic solvent, followed by concentrating the extract. The isolated compound (a) may be, if required, purified by chromatography, recrystallization and the like.
(3) In the case that R² represents a cyano group.

The compound (a) can be produced by subjecting the compound (d) to reaction with a metal cyanide compound.

The reaction is usually carried out in a solvent. The solvent used for the reaction includes, for example, ethers such as diethylether, tetrahydrofuran and the like, aromatic hydrocarbons such as toluene, xylene and the like, and the mixture thereof.

The metal cyanide compound used for the reaction includes, for example, sodium cyanide, potassium cyanide and copper cyanide.

The amount of the metal cyanide compound used for the reaction is usually 1 to 10 moles per 1 mole of the compound (d).

The reaction temperature of the reaction is usually in the range of -20 to 100 °C, and the reaction time is usually in the range of 1 to 24 hours.

After the reaction has finished, the compound (a) can be isolated by subjecting the reaction mixture to ordinary post-treatment such as pouring the reaction mixture into water, extracting with an organic solvent, followed by concentrating the extract. The isolated compound (a) may be, if required, purified by chromatography, recrystallization and the like.

### (Referential Production Method 2)

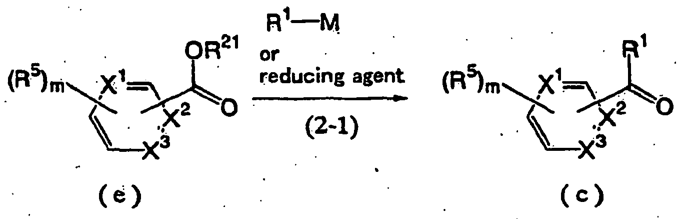

[In the formula, R¹, R⁵, X¹, X², X³, M and m have the same meaning as described above, and R²¹ represents a methyl group or an ethyl group.]

The compound (c) can be produced, for example, by subjecting the compound (e) to reaction with an organometallic compound (R¹ - M : wherein M represents MgI, MgBr, MgCl or Li) or with a reducing agent.

### (1) In the case of subjecting to reaction with R¹ - M

The organometallic compound represented by R¹-M used for the reaction includes,for example,an organic magnesium compound such as methyl magnesium iodide, ethyl magnesium bromide, isopropyl magnesium bromide, vinyl magnesium bromide, ethynyl magnesium bromide, dimethyl magnesium and the like, and an organic lithium compound such as methyl lithium and the like.

The amount of R¹- M used for the reaction is usually 1 to 10 moles per 1 mole of the compound (e).

The solvent used for the reaction includes, for example, ethers such as diethylether, tetrahydrofuran and the like, aromatic hydrocarbons such as toluene, xylene and the like, and mixtures thereof.

The reaction temperature of the reaction is usually in the range of -20 to 100 °C, and the reaction time is usually in the range of the instant to 24 hours.

After the reaction has finished, the compound (c) can be isolated by subjecting the reaction mixture to ordinary post-treatment such as pouring the reaction mixture into water, extracting with an organic solvent, followed by concentrating the extract. The isolated compound (c) may be, if required, purified by chromatography, recrystallization and the like.

### (2) In the case of subjecting to reaction with a reducing agent

The reducing agent used for the reaction includes lithium aluminum hydride, diisobutyl aluminum hydride and the like.

The amount of the reducing agent used for the reaction is usually 0.25 to 5 moles per 1 mole of the compound (e).

The solvent used for the reaction includes, for example, ethers such as diethylether, tetrahydrofuran and the like, aromatic hydrocarbons such as toluene, xylene and the like, and the mixture thereof.

The reaction temperature of the reaction is usually in the range of 0 to 50 °C, and the reaction time is usually in the range of 1 to 24 hours.

After the reaction has finished, the compound (c) can be isolated by subjecting the reaction mixture to ordinary post-treatment such as pouring the reaction mixture into water, extracting with an organic solvent, followed by concentrating the extract. The isolated compound (c) may be, if required, purified by chromatography, recrystallization and the like.

### (Referential Production Method 3)

The compound (e-4) among the compound (e) can be produced, for example, according to the method for synthesizing 5-pyrimidinecarboxylates disclosed in Synthesis, 2002, p.720-722. 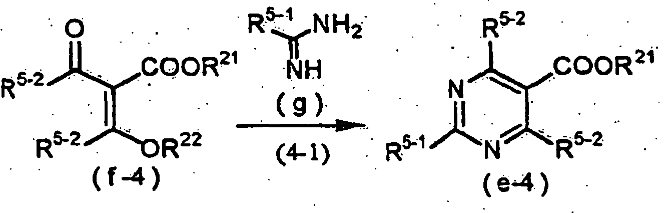 [In the formula, R²¹, m and n have the same meaning as described above; R⁵⁻¹ represents a hydroxyl group, a mercapto group, a C1-C5 alkyl group optionally substituted by one or more halogen atoms, a C3-C5 cycloalkyl group optionally substituted by one or more halogen atoms, a C2-C5 alkenyl group optionally substituted by one or more halogen atoms, a C2-C5 alkynyl group optionally substituted by one or more halogen atoms, a C1-C5 alkoxy group optionally substituted by one or more halogen atoms, a C1-C5 alkylthio group optionally substituted by one or more halogen atoms, a group designated by -COOR²¹ or a hydrogen atom; each of the groups designated by R⁵⁻² represents a C1-C5 alkyl group optionally substituted by one or more halogen atoms, a C3-C5 cycloalkyl group optionally substituted by one or more halogen atoms, a C2-C5 alkenyl group optionally substituted by one or more halogen atoms, a C2-C5 alkynyl group optionally substituted by one or more halogen atoms, a group designated by -COOR²¹ or a hydrogen atom; with the proviso that any one of R⁵⁻¹ and R⁵⁻² is a group designated by ―COOR²¹; R²² represents a methyl group, an ethyl group, a sodium atom or potassium atom.]

### (Referential Production Method 4)

The compound (e-7) among the compound (e) can be produced, for example, by subjecting a compound (1) obtained according to the method disclosed in Eur. J. Med. Chem, Vol. 15, p.157-163 to esterification. 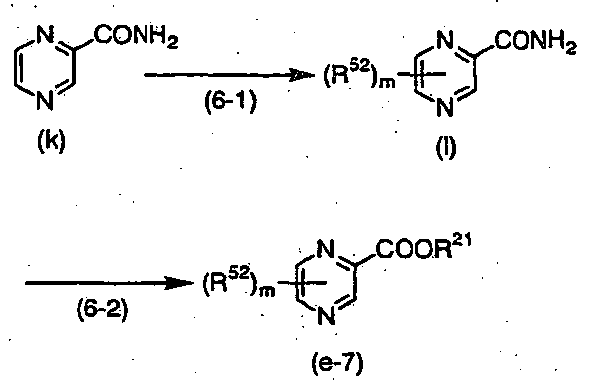 [In the formula, R²¹ and m have the same meaning as described above, and R⁵² represents a C1-C5 alkyl group optionally substituted by one or more halogen atoms.]
The reaction of Step (6-2) can be carried out, for example, by subjecting the compound (1) to dissolution in an alcohol compound represented by R²¹OH, and then addition of an acid (sulfuric acid and the like), followed by heating under reflux.

### (Referential Production Method 5)

The compound (e-8) among the compound (e) can be produced, for example, by the following method. 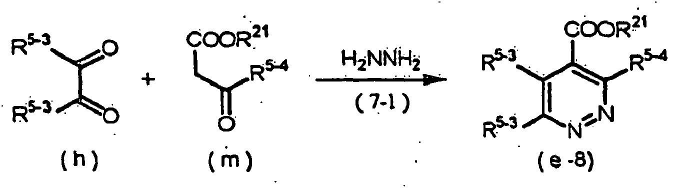 [In the formula, R⁵⁻³ and R²¹ and m have the same meaning as described above, and R⁵⁻⁴ represents a C1-C5 alkyl group optionally substituted by one or more halogen atoms, a C3-C5 cycloalkyl group optionally substituted by one or more halogen atoms, a C2-C5 alkenyl group optionally substituted by one or more halogen atoms, a C2-C5 alkynyl group optionally substituted by one or more halogen atoms, a hydroxyl group or a hydrogen atom.]

### (Referential Production Method 6)

The compound shown by the formula (e-9) among the compound (e) can be produced, for example, according to the following method, using the compound (n) produced according to the method disclosed in J. Med. Chem, Vol. 30, p.239-249. 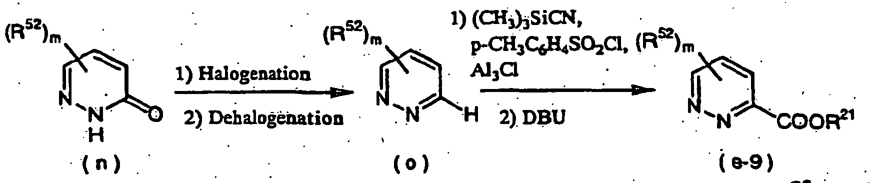 [In the formula, R²¹ , R⁵² and m have the same meaning as described above.]

### (Referential Production Method 7)

The compound (c-1) among the compound (c) can be produced, for example, according to the method disclosed in Chem. Ber., Vol. 97, p.3407-3417. 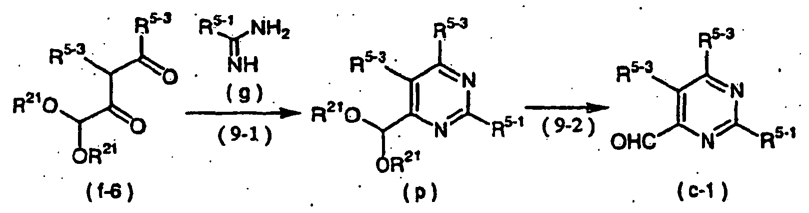 [In the formula, R⁵⁻¹, R⁵⁻³ and R²¹ have the same meaning described above.]

### (Referential Production Method 8)

The compound (q) can be produced, for example, by the following method. 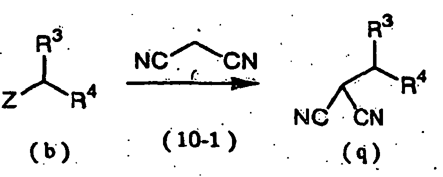 [In the formula, R³, R⁴ and Z have the same meaning as described above.]

The reaction is usually carried out in a solvent under the presence of a base.

The solvent used for the reaction includes, for example, amides such as N,N-dimethylformamide and the like, ethers such as diethylether, tetrahydrofuran and the like, organic sulfurs such as dimethylsulfoxide, sulfolane and the like, halogenated hydrocarbons such as 1,2-dichloroethane, chlorobenzene and the like, aromatic hydrocarbons such as toluene, xylene and the like, and mixtures thereof.

The base used for the reaction includes, for example, inorganic bases such as sodium hydride, sodium carbonate, potassium carbonate and the like, alkali metal alkoxides such as potassium-t-butoxide and the like, and organic bases such as 4-(dimethylamino)pyridine, 1,4-diazabicyclo[2.2.2]octane, 1,8-diazabicyclo[5.4:0]-7-undecene and the like.

The amount of the base used for the reaction is usually 1 to 10 moles per 1 mole of the compound (b).

The amount of the malononitrile used for the reaction is usually 1 to 10 moles per 1 mole of the compound (b).

The reaction temperature of the reaction is usually in the range of -20 to 100 °C, and the reaction time is usually in the range of 1 to 24 hours.

After the reaction has finished, the compound (q) can be isolated by subjecting the reaction mixture to ordinary post-treatment such as pouring the reaction mixture into water, extracting with an organic solvent, followed by concentrating the extract. The isolated compound (q) may be, if required, purified by chromatography, recrystallization and the like.

Among the compound (q), the compound in which R⁴ is a C1-C5 alkyl group optionally substituted by one or more halogen atoms, a C2-C5 alkenyl group optionally substituted by one or more halogen atoms or a C2-C5 alkynyl group optionally substituted by one or more halogen atoms, may be produced according to the method disclosed in J. Chem. Soc. PERKIN. TRANS. 1, 1991, p.2589-2592.

### (Referential Production Method 9)

The compound (r) can be produced, for example, from the compound (c) or the compound (e) according the scheme described below. Namely, the compound (s) which is produced by subjecting the compound (c) or the compound (e) to reaction with an organometallic compound represented by R²-M (illustrated as example are Grignard reagent and an organolithium compound) or a reducing agent (illustrated as example are aluminum lithium hydride and diisobutyl aluminum hydride), is subjected to reaction with halogenating agent (illustrated as example are thionyl chloride and phosphorus oxychloride), or the compound (s) is subjected to reaction with a trifluoromethanesulfonic anhydride, a methanesulfonyl chloride or a toluenesulfonyl chloride under the presence of a base. 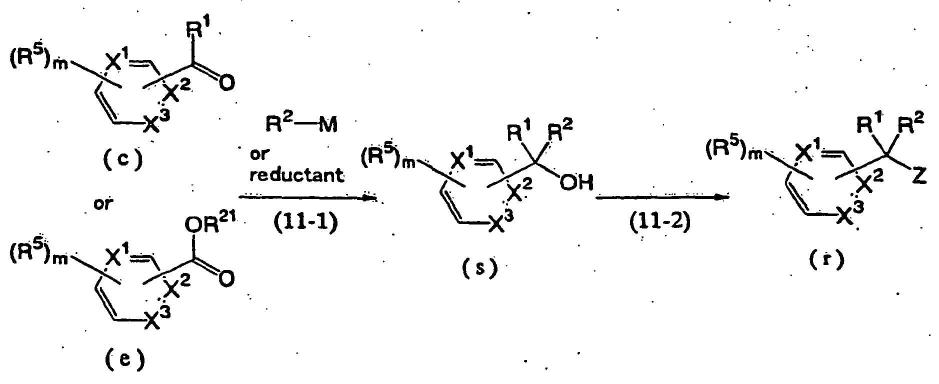 [In the formula, R¹, R², R⁵, R²¹, M, m, X¹, X², X³ and Z have the same meaning as described above.]

### (Referential Production Method 10)

Among the compound (r), the compound (r-1) may be also produced by subjecting the compound (t) to reaction with a halogenating agent. The halogenating agent used for the reaction includes, for example, N-bromosuccinimide and N-chlorosuccinimide. 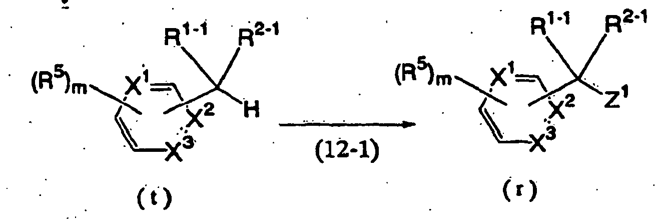 [In the formula, R⁵, m, X¹, X¹ and X³ have the same meaning as described above, each of R¹⁻¹ and R²⁻¹ represents a C1-C5 alkyl group optionally substituted by one or more halogen atoms or a hydrogen atom, and Z¹ represents a chlorine atom or a bromine atom.]

The embodiment of the compound of the present invention includes, for example, the following compounds:
a malononitrile compound represented by the formula (I) wherein R¹ is a hydrogen atom;
a malononitrile compound represented by the formula (I) wherein R² is a methyl group;
a malononitrile compound represented by the formula (I) wherein R¹ and R² are hydrogen atoms;
a malononitrile compound represented by the formula (I) wherein R¹ is a hydrogen atom and R² is a methyl group;
a malononitrile compound represented by the formula (I) wherein R³ is a hydrogen atom;
a malononitrile compound represented by the formula (I) wherein R⁴ is a C2-C5 alkenyl group optionally substituted by one or more halogen atoms;
a malononitrile compound represented by the formula (I) wherein R⁴ is a vinyl group;
a malononitrile compound represented by the formula (I) wherein R⁴ is a 2-propenyl group;
a malononitrile compound represented by the formula (I) wherein R⁴ is a 2,2-difluorovinyl group;
a malononitrile compound represented by the formula (I) wherein R⁴ is a 1-(trifluoromethyl)vinyl group;
a malononitrile compound represented by the formula (I) wherein R⁴ is a 3,3-difluoro-2-propenyl group;
a malononitrile compound represented by the formula (I) wherein R⁴ is a 2,3,3-trifluoro-2-propenyl group;
a malononitrile compound represented by the formula (I) wherein R⁴ is a 3,3,3-trifluoro-1-propenyl group;
a malononitrile compound represented by the formula (I) wherein R⁴ is a C2-C5 alkynyl group optionally substituted by one or more halogen atoms;
a malononitrile compound represented by the formula (I) wherein R⁴ is a C1-C5 fluoroalkyl group;
a malononitrile compound represented by the formula (I) wherein R⁴ is a fluoromethyl group;
a malononitrile compound represented by the formula (I) wherein R⁴ is a 2,2-difluoroethyl group;
a malononitrile compound represented by the formula (I) wherein R⁴ is a 2,2,2-trifluoroethyl group;
a malononitrile compound represented by the formula (I) wherein R⁴ is a pentafluoroethyl group;
a malononitrile compound represented by the formula (I) wherein R⁴ is a 3,3,3-trifluoropropyl group;
a malononitrile compound represented by the formula (I) wherein R⁴ is a 2,2,3,3,3-pentafluoropropyl group;
a malononitrile compound represented by the formula (I) wherein R⁴ is a C3-C5 cycloalkyl group optionally substituted by one or more halogen atoms;
a malononitrile compound represented by the formula (I) wherein R⁴ is a 2,2-dichlorocyclopropyl group;
a malononitrile compound represented by the formula (I) wherein R⁴ is a cyclopropyl group;
a malononitrile compound represented by the formula (I) wherein R⁴ is a cyclobutyl group;
a malononitrile compound represented by the formula (I) wherein R³ is a hydrogen atom and R⁴ is a C1-C5 alkyl group optionally substituted by one or more halogen atoms, a C2-C5 alkenyl group optionally substituted by one or more halogen atoms, a C2-C5 alkynyl group optionally substituted by one or more halogen atoms, a C3-C5 cycloalkyl group optionally substituted by one or more halogen atoms, or a C5-C6 cycloalkenyl group optionally substituted by one or more halogen atoms;
a malononitrile compound represented by the formula (I) wherein R³ is a hydrogen atom and R⁴ is a C2-C5 alkenyl group optionally substituted by one or more halogen atoms, a C2-C5 alkynyl group optionally substituted by one or more halogen atoms, or a C3-C5 cycloalkyl group optionally substituted by one or more halogen atoms;
a malononitrile compound represented by the formula (I) wherein R³ is a hydrogen atom, R⁴ is a C2-C5 alkenyl group optionally substituted by one or more halogen atoms, a C2-C5 alkynyl group optionally substituted by one or more halogen atoms or a C3-C5 cycloalkyl group optionally substituted by one or more halogen atoms, X¹ and X² are nitrogen atoms, and X³ is CR⁶;
a malononitrile compound represented by the formula (I) wherein R³ is a hydrogen atom, R⁴ is a C2-C5 alkenyl group optionally substituted by one or more halogen atoms, a C2-C5 alkynyl group optionally substituted by one or more halogen atoms or a C3-C5 cycloalkyl group optionally substituted by one or more halogen atoms, X¹ and X³ are nitrogen atoms, and X² is CR⁶;
a malononitrile compound represented by the formula (I) wherein R³ is a hydrogen atom, R⁴ is a C2-C5 alkenyl group optionally substituted by one or more halogen atoms, a C2-C5 alkynyl group optionally substituted by one or more halogen atoms or a C3-C5 cycloalkyl group optionally substituted by one or more halogen atoms, X² and X³ are a nitrogen atom, and X¹ is CR⁶;
a malononitrile compound represented by the formula (I) wherein R³ is a hydrogen atom and R⁴ is a vinyl group or a 2-propenyl group;
a malononitrile compound represented by the formula (I) wherein R³ is a hydrogen atom and R⁴ is a 2,2-difluorovinyl group, a 1-(trifluoromethyl)vinyl group, a 3,3-difluoro-2-propenyl group, a 2,3,3-trifluoro-2-propenyl group or a 3,3,3-trifluoro-1-propenyl group;
a malononitrile compound represented by the formula (I) wherein R³ is a hydrogen atom and R⁴ is a fluoromethyl group, a 2,2-difluoroethyl group, a 2,2,2-trifluoroethyl group, a 1,1,2,2,2-pentafluoroethyl group, a 3,3,3-trifluoropropyl group or a 2,2,3,3,3-pentafluoropropyl group;
a malononitrile compound represented by the formula (I) wherein R³ is a hydrogen atom and R⁴ is a cyclopropyl group, a cyclobutyl group or a 2,2-dichlorocyclopropyl group;
a malononitrile compound represented by the formula (I) wherein R¹, R² and R³ are hydrogens atom and R⁴ is a C2-C5 alkenyl group optionally substituted by one or more halogen atoms, a C2-C5 alkynyl group optionally substituted by one or more halogen atoms or a C3-C5 cycloalkyl group optionally substituted by one or more halogen atoms;
a malononitrile compound represented by the formula (I) wherein R¹, R² and R³ are hydrogen atoms and R⁴ is a vinyl group or a 2-propenyl group;
a malononitrile compound represented by the formula (I) wherein R¹, R² and R³ are hydrogen atoms and R⁴ is a 2,2-difluorovinyl group, a 1-(trifluoromethyl)vinyl group, a 3,3-difluoro-2-propenyl group, a 2,3,3-trifluoro-2-propenyl group or a 3,3,3-trifluoro-1-propenyl group;
a malononitrile compound represented by the formula (I) wherein R¹ and R³ are hydrogen atoms, R² is a methyl group and R⁴ is a 2,2-difluorovinyl group, a 1-(trifluoromethyl)vinyl group, a 3,3-difluoro-2-propenyl group, a 2,3,3-trifluoro-2-propenyl group, a 3,3,3-trifluoro-1-propenyl group;
a malononitrile compound represented by the formula (I) wherein R¹, R² and R³ are hydrogen atoms and R⁴ is a fluoromethyl group, a 2,2-difluoroethyl group, a 2,2,2-trifluoroethyl group, a pentafluoroethyl group, a 3,3,3-trifluoropropyl group or a 2,2,3,3,3-pentafluoropropyl group;
a malononitrile compound represented by the formula (I) wherein R¹ and R³ are hydrogen atoms, R² is a methyl group and R⁴ is a fluoromethyl group, a 2,2-difluoroethyl group, a 2,2,2-trifluoroethyl group, a pentafluoroethyl group, a 3,3,3-trifluoropropyl group or a 2,2,3,3,3-pentafluoropropyl group;
a malononitrile compound represented by the formula (I) wherein R¹, R² and R³ are hydrogen atoms and R⁴ is a cyclopropyl group, a cyclobutyl group or a 2,2-dichlorocyclopropyl group;
a malononitrile compound represented by the formula (I) wherein R¹ and R³ are hydrogen atoms and R⁴ is a C2-C5 alkenyl group optionally substituted by one or more halogen atoms, a C2-C5 alkynyl group optionally substituted by one or more halogen atoms or a C3-C5 cycloalkyl group optionally substituted by one or more halogen atoms;
a malononitrile compound represented by the formula (I) wherein X¹, X² or X³ is CR⁶ and R⁶ is a hydrogen atom;
a malononitrile compound represented by the formula (I) wherein X¹ is CR⁶ and R⁶ is a hydrogen atom;
a malononitrile compound represented by the formula (I) wherein X² is CR⁶ and R⁶ is a hydrogen atom;
a malononitrile compound represented by the formula (I) wherein X³ is CR⁶ and R⁶ is a hydrogen atom;
a malononitrile compound represented by the formula (I) wherein X³ is CR⁶ and R⁶ is R⁵;
a malononitrile compound represented by the formula (I) wherein, X¹, X² or X³ is CR⁶, R⁶ is a hydrogen atom and R⁵ is a nitro group,
a C1-C5 alkyl group optionally substituted by one or more halogen atoms, a C2-C5 alkenyl group optionally substituted by one or more halogen atoms, a C2-C5 alkynyl group optionally substituted by one or more halogen atoms, a C1-C5 alkoxy group optionally substituted by one or more halogen atoms, a C3-C6 alkenyloxy group optionally substituted by one or more halogen atoms, a C3-C6 alkynyloxy group optionally substituted by one or more halogen atoms, a C1-C5 alkylthio group optionally substituted by one or more halogen atoms, an alkylsulfinyl group optionally substituted by one or more halogen atoms or an alkylsulfonyl group optionally substituted by one or more halogen atoms;
a malononitrile compound represented by the formula (I) wherein X¹, X² or X³ is CR⁶, R⁶ is a hydrogen atom, m is 1 and R⁵ is a cyano group;
a malononitrile compound represented by the formula (I) wherein X¹, X² or X³ is CR⁶, R⁶ is a hydrogen atom, m is 1 and R⁵ is a fluorine atom;
a malononitrile compound represented by the formula (I) wherein X¹, X² or X³ is CR⁶, R⁶ is a hydrogen atom, m is 1 and R⁵ is a chlorine atom;
a malononitrile compound represented by the formula (I) wherein X¹, X² or X³ is CR⁶, R⁶ is a hydrogen atom, m is 1 and R⁵ is a bromine atom;
a malononitrile compound represented by the formula (I) wherein X¹, X² or X³ is CR⁶, R⁶ is a hydrogen atom, m is 1 and R⁵ is a methyl group;
a malononitrile compound represented by the formula (I) wherein X¹, X² or X³ is CR⁶, R⁶ is a hydrogen atom, m is 1 and R⁵ is a ethyl group;
a malononitrile compound represented by the formula (I) wherein X¹, X² or X³ is CR⁶, R⁶ is a hydrogen atom, m is 1 and R⁵ is an isopropyl group;
a malononitrile compound represented by the formula (I) wherein X¹, X² or X³ is CR⁶, R⁶ is a hydrogen atom, m is 1 and R⁵ is a t-butyl group;
a malononitrile compound represented by the formula (I) wherein X¹, X² or X³ is CR⁶, R⁶ is a hydrogen atom, m is 1 and R⁵ is a 1-fluoro-1-methylethyl group;
a malononitrile compound represented by the formula (I) wherein X¹, X² or X³ is CR⁶, R⁶ is a hydrogen atom, m is 1 and R⁵ is a trifluoromethyl group;
a malononitrile compound represented by the formula (I) wherein X¹, X² or X³ is CR⁶, R⁶ is a hydrogen atom, m is 1 and R⁵ is a pentafluoroethyl group;
a malononitrile compound represented by the formula (I) wherein X¹, X² or X³ is CR⁶, R⁶ is a hydrogen atom, m is 1 and R⁵ is a propargyl group;
a malononitrile compound represented by the formula (I) wherein X¹, X² or X³ is CR⁶, R⁶ is a hydrogen atom, m is 1 and R⁵ is a vinyl group;
a malononitrile compound represented by the formula (I) wherein X¹, X² or X³ is CR⁶, R⁶ is a hydrogen atom, m is 1 and R⁵ is a propargyloxy group;
a malononitrile compound represented by the formula (I) wherein X¹, X² or X³ is CR⁶, R⁶ is a hydrogen atom, m is 1 and R⁵ is a 2-butynyloxy group;
a malononitrile compound represented by the formula (I) wherein X¹, X² or X³ is CR⁶, R⁶ is a hydrogen atom, m is 1 and R⁵ is a 3-butynyloxy group;
a malononitrile compound represented by the formula (I) wherein X¹, X² or X³ is CR⁶, R⁶ is a hydrogen atom, m is 1 and R⁵ is a methoxy group;
a malononitrile compound represented by the formula (I) wherein X¹, X² or X³ is CR⁶, R⁶ is a hydrogen atom, m is 1 and R⁵ is a methylthio group;
a malononitrile compound represented by the formula (I) wherein X¹, X² or X³ is CR⁶, R⁶ is a hydrogen atom, m is 1 and R⁵ is a trifluoromethylthio group;
a malononitrile compound represented by the formula (I) wherein X1, X2 or X3 is CR⁶, R⁶ is a hydrogen atom, m is 1 and R⁵ is a trifluoromethylsulfinyl group;
a malononitrile compound represented by the formula (I) wherein X¹, X² or X³ is CR⁶, R⁶ is a hydrogen atom, m is 1 and R⁵ is a trifluoromethylsulfonyl group;
a malononitrile compound represented by the formula (I) wherein X¹, X² or X³ is CR⁶, R⁶ is a hydrogen atom, m is 1 and R⁵ is a nitro group;
a malononitrile compound represented by the formula (I) wherein X¹, X² or X³ is CR⁶, R⁶ is a hydrogen atom, m is 1 and R⁵ is a dimethylamino group;
a malononitrile compound represented by the formula (I) wherein X¹, X² and X³ are nitrogen atoms and R⁵ is a nitro group, a C1-C5 alkyl group optionally substituted by one or more halogen atoms,
a C2-C5 alkenyl group optionally substituted by one or more halogen atoms, a C2-C5 alkynyl group optionally substituted by one or more halogen atoms, a C1-C5 alkoxy group optionally substituted by one or more halogen atoms, a C3-C6 alkenyloxy group optionally substituted by one or more halogen atoms, a C3-C6 alkynyloxy group optionally substituted by one or more halogen atoms, a C1-C5 alkylthio group optionally substituted by one or more halogen atoms, an alkylsulfinyl group optionally substituted by one or more halogen atoms or an alkylsulfonyl group optionally substituted by one or more halogen atoms;
a malononitrile compound represented by the formula (XI), 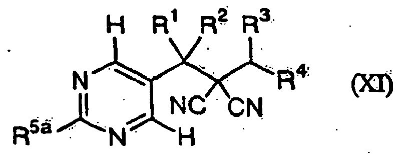 wherein, in the formula,
R¹ represents a C1-C5 alkyl group optionally substituted by one or more halogen atoms, a C2-C5 alkenyl group optionally substituted by one or more halogen atoms, a C2-C5 alkynyl group optionally substituted by one or more halogen atoms or a hydrogen atom;
R² represents a C1-C5 alkyl group optionally substituted by one or more halogen atoms, a C1-C5 alkoxy group optionally substituted by one or more halogen atoms, a C2-C5 alkenyl group optionally substituted by one or more halogen atoms, a C2-C5 alkynyl group optionally substituted by one or more halogen atoms, a cyano group or a hydrogen atom;
each of R³ and R⁴ represents a C1-C5 alkyl group optionally substituted by one or more halogen atoms, a C2-C5 alkenyl group optionally substituted by one or more halogen atoms, a C2-C5 alkynyl group optionally substituted by one or more halogen atoms, a C3-C5 cycloalkyl group optionally substituted by one or more halogen atoms, a C5-C6 cycloalkenyl group optionally substituted by one or more halogen atoms or a hydrogen atom,
or represents a C2-C6 alkanediyl group optionally substituted by one or more halogen atoms or a C4-C6 alkenediyl group optionally substituted by one or more halogen atoms in each of which R³ and R⁴ couple one another at the ends thereof;
R^{5a} represents a hydrogen atom, halogen atom, a cyano group, a nitro group, a hydroxyl group, a mercapto group, a formyl group, a SF₅ group, a carboxyl group, a C1-C5 alkyl group optionally substituted by one or more halogen atoms, a C2-C5 alkenyl group optionally substituted by one or more halogen atoms, a C2-C5 alkynyl group optionally substituted by one or more halogen atoms, a C3-C5 cycloalkyl group optionally substituted by one or more halogen atoms, a C1-C5 alkoxy group optionally substituted by one or more halogen atoms, a C3-C6 alkenyloxy group optionally substituted by one or more halogen atoms, a C3-C6 alkynyloxy group optionally substituted by one or more halogen atoms, a C1-C5 alkylthio group optionally substituted by one or more halogen atoms, a C3-C5 alkenylthio group optionally substituted by one or more halogen atoms, a C3-C5 alkynylthio group optionally substituted by one or more halogen atoms, a C1-C5 alkylsulfinyl group optionally substituted by one or more halogen atoms, a C1-C5 alkylsulfonyl group optionally substituted by one or more halogen atoms, a C2-C6 alkylcarbonyl group optionally substituted by one or more halogen atoms, a C2-C5 alkoxycarbonyl group optionally substituted by one or more halogen atoms, a group designated by -NR¹⁰R¹¹ , a group designated by -C( =X⁵)NR¹²R¹³ or a group designated by -C(=NOR¹⁴)R^{1,5}
each of R¹⁰ and R¹¹ represents a C1-C5 alkyl group optionally substituted by one or more halogen atoms, a C3-C5 alkenyl group optionally substituted by one or more halogen atoms, a C3-C5 alkynyl group optionally substituted by one or more halogen atoms , a C3-C5 cycloalkyl group optionally substituted by one or more halogen atoms, a (C1-C5 alkoxy optionally substituted by one or more halogen atoms) C1-C3 alkyl group, a C1-C5 alkylsulfonyl group optionally substituted by one or more halogen atoms, a C2-C6 alkylcarbonyl group optionally substituted by one or more halogen atoms, a C2-C5 alkoxycarbonyl group optionally substituted by one or more halogen atoms or a hydrogen atom, or represents a C2-C6 alkanediyl group optionally substituted by one or more halogen atoms or a C4-C6 alkenediyl group optionally substituted by one or more halogen atoms in each of which R¹⁰ and R¹¹ are coupled with one another at the ends thereof;
each of R¹² and R¹³ represents a C1-C5 alkyl group optionally substituted by one or more halogen atoms, a C3-C5 alkenyl group optionally substituted by one or more halogen atoms, a C3-C5 alkynyl group optionally substituted by one or more halogen atoms, a C3-C5 cycloalkyl group optionally substituted by one or more halogen atoms, a group designated by-(CH₂)ₙQ¹ or a hydrogen atom,
or represents a C2-C6 alkanediyl group optionally substituted by one or more halogen atoms or a C4-C6 alkenediyl group optionally substituted by one or more halogen atoms in each of which R¹² and R¹³ are coupled with one another at the ends thereof; each of R¹⁴ and R¹⁵ represents a C1-C5 alkyl group optionally substituted by one or more halogen atoms, a C3-C5 alkenyl group optionally substituted by one or more halogen atoms, a C3-C5 alkynyl group optionally substituted by one or more halogen atoms, a C3-C5 cycloalkyl group optionally substituted by one or more halogen atoms, a group designated by ―(CH₂)ₙQ² or a hydrogen atom; each of Q¹ and Q² represents a phenyl group which may be substituted by a group selected from the group consisting of a C1-C5 alkyl group optionally substituted by one or more halogen atoms, a C1-C5 alkoxy group optionally substituted by one or more halogen atoms, a C1-C5 alkylthio group optionally substituted by one or more halogen atoms and a halogen atom; and X⁵ represents an oxygen atom or a sulfur atom;
a malononitrile compound represented by the formula (XII), 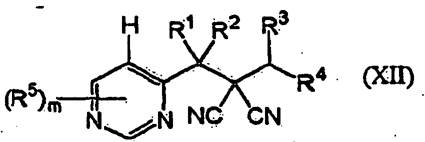 wherein, in the formula,
R¹ represents a C1-C5 alkyl group optionally substituted by one or more halogen atoms, a C2-C5 alkenyl group optionally substituted by one or more halogen atoms, a C2-C5 alkynyl group optionally substituted by one or more halogen atoms or a hydrogen atom;
R² represents a C1-C5 alkyl group optionally substituted by one or more halogen atoms, a C1-C5 alkoxy group optionally substituted by one or more halogen atoms, a C2-C5 alkenyl group optionally substituted by one or more halogen atoms, a C2-C5 alkynyl group optionally substituted by one or more halogen atoms, a cyano group or a hydrogen atom;
each of R³ and R⁴ represents a C1-C5 alkyl group optionally substituted by one or more halogen atoms, a C2-C5 alkenyl group optionally substituted by one or more halogen atoms, a C2-C5 alkynyl group optionally substituted by one or more halogen atoms, a C3-C5 cycloalkyl group optionally substituted by one or more halogen atoms, a C5-C6cycloalkenyl group optionally substituted by one or more halogen atoms or a hydrogen atom,
or represents a C2-C6 alkanediyl group optionally substituted by one or more halogen atoms or a C4-C6 alkenediyl group optionally substituted by one or more halogen atoms in each of which R³ and R⁴ couple one another at the ends thereof;
R⁵ represents a halogen atom, a cyano group, a nitro group, a hydroxyl group, a mercapto group, a formyl group, a SF₅ group, a carboxyl group, a C1-C5 alkyl group optionally substituted by one or more halogen atoms, a C2-C5 alkenyl group optionally substituted by one or more halogen atoms, a C2-C5 alkynyl group optionally substituted by one or more halogen atoms, a C3-C5 cycloalkyl group optionally substituted by one or more halogen atoms, a C1-C5 alkoxy group optionally substituted by one or more halogen atoms, a C3-C6 alkenyloxy group optionally substituted by one or more halogen atoms, a C3-C6 alkynyloxy group optionally substituted by one or more halogen atoms, a C1-C5 alkylthio group optionally substituted by one or more halogen atoms, a C3-C5 alkenylthio group optionally substituted by one or more halogen atoms, a C3-C5 alkynylthio group optionally substituted by one or more halogen atoms, a C1-C5 alkylsulfinyl group optionally substituted by one or more halogen atoms, a C1-C5 alkylsulfonyl group optionally substituted by one or more halogen atoms, a C2-C6 alkylcarbonyl group optionally substituted by one or more halogen atoms, a C2-C5 alkoxycarbonyl group optionally substituted by one or more halogen atoms, a group designated by -NR¹⁰R¹¹, a group designated by - C ( =X⁵)NR¹²R¹³ or a group designated by - C(=NOR¹⁴)R¹⁵,
each of R¹⁰ and R¹¹ represents a C1-C5 alkyl group optionally substituted by one or more halogen atoms, a C3-C5 alkenyl group optionally substituted by one or more halogen atoms, a C3-C5 alkynyl group optionally substituted by one or more halogen atoms, a C3-C5 cycloalkyl group optionally substituted by one or more halogen atoms, a (C1-C5 alkoxy optionally substituted by one or more halogen atoms) C1-C3 alkyl group, a C1-C5 alkylsulfonyl group optionally substituted by one or more halogen atoms, a C2-C6 alkylcarbonyl group optionally substituted by one or more halogen atoms, a C2-C5 alkoxycarbonyl group optionally substituted by one or more halogen atoms or a hydrogen atom, or represents a C2-C6 alkanediyl group optionally substituted by one or more halogen atoms or a C4-C6 alkenediyl group optionally substituted by one or more halogen atoms in each of which R¹⁰ and R¹¹ are coupled with one another at the ends thereof;
each of R¹² and R¹³ represents a C1-C5 alkyl group optionally substituted by one or more halogen atoms, a C3-C5 alkenyl group optionally substituted by one or more halogen atoms, a C3-C5 alkynyl group optionally substituted by one or more halogen atoms, a C3-C5 cycloalkyl group optionally substituted by one or more halogen atoms, a group designated by - (CH₂)ₙQ¹ or a hydrogen atom,
or represents a C2-C6 alkanediyl group optionally substituted by one or more halogen atoms or a C4 -C6 alkenediyl group optionally substituted by one or more halogen atoms in each of which R¹² and R¹³ are coupled with one another at the ends thereof; each of R¹⁴ and R¹⁵ represents a C1-C5 alkyl group optionally substituted by one or more halogen atoms, a C3-C5 alkenyl group optionally substituted by one or more halogen atoms, a C3-C5 alkynyl group optionally substituted by one or more halogen atoms, a C3-C5 cycloalkyl group optionally substituted by one or more halogen atoms, a group designated by -(CH₂)ₙQ² or a hydrogen atom; each of Q¹ and Q² represents a phenyl group which may be substituted by a group selected from the group consisting of a C1-C5 alkyl group optionally substituted by one or more halogen atoms, a C1-C5 alkoxy group optionally substituted by one or more halogen atoms, a C1-C5 alkylthio group optionally substituted by one or more halogen atoms and a halogen atom; and X⁵ represents an oxygen atom or a sulfur atom;
and m represents an integer from 0 to 2;
a malononitrile compound represented by the formula (XIII), 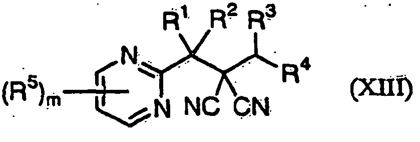 wherein, in the formula, R¹, R², R³, R⁴ , and R⁵ have the same meaning as described in the definition of the formula (XII) and m represents an integer from 0 to 3;
a malononitrile compound represented by the formula (XIV), 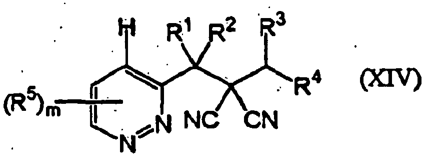 wherein, in the formula, R¹, R² , R³ , R⁴, and R⁵ have the same meaning as described in the definition of the formula (XII) and m represents an integer from 0 to 2;
a malononitrile compound represented by the formula (XV), 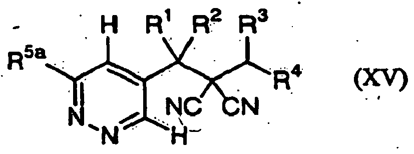 wherein, in the formula, R¹, R², R³, R⁴ and R^{5a} have the same meaning as described in the definition of the formula (XI);
a malononitrile compound represented by the formula (XVI), 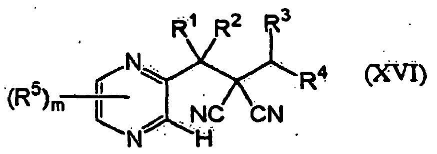 wherein, in the formula, R¹, R², R³, R⁴, and R⁵ have the same meaning as described in the definition of the formula (XII) and m represents an integer from 0 to 2;
a malononitrile compound represented by the formula (XI) wherein R³ is a hydrogen atom and R⁴ is a C1-C5 alkyl group optionally substituted by one or more halogen atoms, a C2-C5 alkenyl group optionally substituted by one or more halogen atoms, a C2-C5 alkynyl group optionally substituted by one or more halogen atoms, a C3-C5 cycloalkyl group optionally substituted by one or more halogen atoms or a C5-C6 cycloalkenyl group optionally substituted by one or more halogen atoms;
amalononitrile compound represented by the formula (XII) wherein R³ is a hydrogen atom and R⁴ is a C1-C5 alkyl group optionally substituted by one or more halogen atoms, a C2-C5 alkenyl group optionally substituted by one or more halogen atoms, a C2-C5 alkynyl group optionally substituted by one or more halogen atoms, a C3-C5 cycloalkyl group optionally substituted by one or more halogen atoms or a C5-C6 cycloalkenyl group optionally substituted by one or more halogen atoms;
a malononitrile compound represented by the formula (XIII) wherein R³ is a hydrogen atom and R⁴ is a C1-C5 alkyl group optionally substituted by one or more halogen atoms, a C2-C5 alkenyl group optionally substituted by one or more halogen atoms, a C2-C5 alkynyl group optionally substituted by one or more halogen atoms, a C3-C5 cycloalkyl group optionally substituted by one or more halogen atoms or a C5-C6 cycloalkenyl group optionally substituted by one or more halogen atoms;
amalononitrile compound represented by the formula (XIV) wherein R³is a hydrogen atom and R⁴ is a C1-C5 alkyl group optionally substituted by one or more halogen atoms, a C2-C5 alkenyl group optionally substituted by one or more halogen atoms, a C2-C5 alkynyl group optionally substituted by one or more halogen atoms, a C3-C5 cycloalkyl group optionally substituted by one or more halogen atoms or a C5-C6 cycloalkenyl group optionally substituted by one or more halogen atoms;
amalononitrile compound represented by the formula (XV) wherein R³ is a hydrogen atom and R⁴ is a C1-C5 alkyl group optionally substituted by one or more halogen atoms, a C2-C5 alkenyl group optionally substituted by one or more halogen atoms, a C2-C5 alkynyl group optionally substituted by one or more halogen atoms, a C3-C5 cycloalkyl group optionally substituted by one or more halogen atoms or a C5-C6 cycloalkenyl group optionally substituted by one or more halogen atoms;
amalononitrile compound represented by the f ormula (XVI) wherein R³ is a hydrogen atom and R⁴ is a C1-C5 alkyl group optionally substituted by one or more halogen atoms, a C2-C5 alkenyl group optionally substituted by one or more halogen atoms, a C2-C5 alkynyl group optionally substituted by one or more halogen atoms, a C3-C5 cycloalkyl group optionally substituted by one or more halogen atoms or a C5-C6 cycloalkenyl group optionally substituted by one or more halogen atoms;
a malononitrile compound represented by the formula (XI), the formula (XII) or the formula (XIII) wherein R⁵ or R^{5a} is a nitro group, a C1-C5 alkyl group optionally substituted by one or more halogen atoms, a C1-C5 alkyl group optionally substituted by one or more halogen atoms, a C2-C5 alkenyl group optionally substituted by one or more halogen atoms, a C2-C5 alkynyl group optionally substituted by one or more halogen atoms, a C1-C5 alkoxy group optionally substituted by one or more halogen atoms, a C3-C6 alkenyloxy group optionally substituted by one or more halogen atoms, a C3-C6 alkynyloxy group optionally substituted by one or more halogen atoms, a C1-C5 alkylthio group optionally substituted by one or more halogen atoms, an alkylsulfinyl group optionally substituted by one or more halogen atoms or an alkylsulfonyl group optionally substitutedby one or more halogen atoms;
a malononitrile compound represented by the formula (XI) or the formula (XII) wherein R⁵ or R^{5a} is a cyano group;
a malononitrile compound represented by the formula (XI) or the formula (XII) wherein R⁵ or R^{5a} is a fluorine atom;
a malononitrile compound represented by the formula (XI) or the formula (XII) wherein R⁵ or R^{5a} is a chlorine atom;
a malononitrile compound represented by the formula (XI) or the formula (XII) wherein R⁵ or R^{5a} is a bromine atom;
a malononitrile compound represented by the formula (XI) or the formula (XII) wherein R⁵ or R^{5a} is a methyl group;
a malononitrile compound represented by the formula (XI) or the formula (XII) wherein R⁵ or R^{5a} is an ethyl group;
a malononitrile compound represented by the formula (XI) or the formula (XII) wherein R⁵ or R^{5a} is an isopropyl group;
a malononitrile compound represented by the formula (XI) or the formula (XII) wherein R⁵ or R^{5a} is a t-butyl group;
a malononitrile compound represented by the formula (XI) or the formula (XII) wherein R⁵ or R^{5a} is a 1-fluoro-1-methylethyl group;
a malononitrile compound represented by the formula (XI) or the formula (XII) wherein R⁵ or R^{5a} is a trifluoromethyl group;
a malononitrile compound represented by the formula (XI) or the formula (XII) wherein R⁵ or R^{5a} is a pentafluoroethyl group;
a malononitrile compound represented by the formula (XI) or the formula (XII) wherein R⁵ or R^{5a} is an ethynyl group;
a malononitrile compound represented by the formula (XI) or the formula (XII) wherein R⁵ or R^{5a} is a propargyl group;
a malononitrile compound represented by the formula (XI) or the formula (XII) wherein R⁵ or R^{5a} is a vinyl group;
a malononitrile compound represented by the formula (XI) or the formula (XII) wherein R⁵ or R^{5a} is a propargyloxy group;
a malononitrile compound represented by the formula (XI) or the formula (XII) wherein R⁵ or R^{5a} is a 2-butynyloxy group;
a malononitrile compound represented by the formula (XI) or the formula (XII) wherein R⁵ or R^{5a} is a 3-butynyloxy group;
a malononitrile compound represented by the formula (XI) or the formula (XII) wherein R⁵ or R^{5a} is a methoxy group;
a malononitrile compound represented by the formula (XI) or the formula (XII) wherein R⁵ or R^{5a} is a methylthio group;
a malononitrile compound represented by the formula (XI) or the formula (XII) wherein R⁵ or R^{5a} is a trifluoromethylthio group; a malononitrile compound represented by the formula (XI) or the formula (XII) wherein R⁵ or R^{5a} is a trifluoromethylsulfinyl group;
a malononitrile compound represented by the formula (XI) or the formula (XII) wherein R⁵ or R^{5a} is a trifluoromethylsulfonyl group;
a malononitrile compound represented by the formula (XI) or the formula (XII) wherein R⁵ or R^{5a} is a nitro group; and
a malononitrile compound represented by the formula (XI) or the formula (XII) wherein R⁵ or R^{5a} is a dimethylamino group.

The pests against which the present invention compound has control activity may include, for example, arthropods such as insect pests and acarine pests and the like, and nematode pests. Specific examples are listed below:
Hemiptera:
   Delphacidae such as *Laodelphax striatellus, Nilaparvata lugens, Sogatella furcifera* and the like,
   Deltocephalidae such as *Nephotettix cincticeps, Nephotettix virescens* and the like,
   Aphididae such as *Aphis gossypii, Myzus persicae* and the like,
   Pentatomidae such *asNezara antennata, Riptortus clavetus, Eysarcoris lewisi, Eysarcoris parvus, Plautia stali, Halyomorpha mista* and the like,
   Aleyrodidae such as *Trialeurodes vaporariorum, Bemisia argentifolii* and the like,
   Coccidae such as *Aonidiella aurantii, Comstockaspis perniciosa, Unaspis citri, Ceroplastes* rubens, *Icerya purchasi* and the like,
   Tingidae,
   Psyllidae, and the like;
Lepidoptera:
   Pyralidae such as *Chilo suppressalis, Cnaphalocrocis medinalis, Notarcha derogata, Plodia interpunctella* and the like,
   Noctuidae such as Spodoptera litura, Pseudaletia separata, Thoricoplusia spp., Heliothis spp., Helicoverpa spp. and the like,
   Pieridae such as *Pieris rapae* and the like,
   Tortricidae such as Adoxophyes spp., *Grapholita molesta, Cydia pomonella* and the like,
   Carposinidae such as *Carposina niponensis* and the like,
   Lyonetiidae such as Lyonetia spp. and the like,
   Lymantriidae such as Lymantria spp. , Euproctis spp., and the like,
   Yponomeutidae such as *Plutella xylostella* and the like,
   Gelechiidae such as *Pectinophora gossypiella* and the like,
   Arctiidae such as *Hyphantria cunea* and the like,
   Tineidae such as *Tinea translucens, Tineola bisselliella* and the like;
Diptera:
   Calicidae such as *Culex pipiens pallens, Culex tritaeniorhynchus, Culex quinquefasciatus* and the like,
   Aedes spp. such as *Aedes aegypti, Aedes albopictus* and the like,
   Anopheles spp. such as *Anopheles sinensis* and the like,
   Chironomidae,
   Muscidae such as *Musca domestica, Muscina stabulans* and the like,
   Calliphoridae,
   Sarcophagidae,
   Fanniidae, .
   Anthomyiidae such as *Delia platura, Delia antiqua* and the like,
   Tephritidae,
   Drosophilidae,
   Psychodidae,
   Tabanidae,
   Simuliidae,
   Stomoxyidae,
   Agromyzidae, and the like;
Coleoptera:
   Diabrotica spp. such as *Diabrotica virgifera virgifera, Diabrotica undecimpunctata howardi* and the like,
   Scarabaeidae such as *Anomala cuprea, Anomala rufocuprea* and the like,
   Curculionidae such as *Sitophilus zeamais, Lissorhoptrus oryzophilus, Calosobruchus chinensis* and the like,
   Tenebrionidae such as *Tenebrio molitor, Tribolium castaneum* and the like,
   Chrysomelidae such as *Oulema oryzae, Aulacophora femoralis, Phyllotreta striolata, Leptinotarsa decemlineata* and the like,
   Anobiidae,
   Epilachna spp. such as *Epilachna vigintioctopunctata* and the like,
   Lyctidae,
   Bostrychidae,
   Cerambycidae,
   Paederus fuscipes;
   Blattodea: *Blattella germanica, Periplaneta fuliginosa, Periplaneta americana, Periplaneta brunnea, Blatta orientalis* and the like;
   Thysanoptera: *Thrips palmi, Thrips tabaci, Frankliniella occidentalis, Frankliniella intonsa* and the like;
   Hymenoptera: Formicidae, Vespidae, bethylid wasp, Tenthredinidae such as *Athalia japonica,* and the like;
   Orthoptera: Gryllotalpidae, Acrididae, and the like;
   Aphaniptera: *Ctenocephalides felis, Ctenocephalides canis, Pulex irritans, Xenopsylla cheopis,* and the like;
   Anoplura: *Pediculus humanus corporis, Phthirus pubis, Haematopinus eurysternus, Dalmalinia ovis,* and the like;
   Isoptera: *Reticulitermes speratus, Coptotermes formosanus,* and the like;
Acarina:
   Tetranychidae such as *Tetranychus urticae, Tetranychus kanzawai, Panonychus citri, Panonychus ulmi,* Oligonychus spp., and the like,
   Eriophyidae such as *Aculops pelekassi, Aculus schlechtendali,* and the like,
   Tarsonemidae such as *Polyphagotarsonemus latus*, and the like,
   Tenuipalpidae,
   Tuckerellidae,
   Ixodidae such as *Haemaphysalis longicornis, Haemaphysalis flava, Dermacentor taiwanicus, Ixodes ovatus, Ixodes persulcatus, Boophilus microplus,* and the like,
   Acaridae such as *Tyrophagus putrescentiae,* and the like,
   Epidermoptidae such as *Dermatophagoides farinae, Dermatophagoides ptrenyssnus,* and the like,
   Cheyletidae such as *Cheyletus eruditus, Cheyletus malaccensis, Cheyletus moorei,* and the like,
Dermanyssidae;
   Araneae: *Chiracanthium japonicum,Latrodectus hasseltii,* and the like;
   Chilopoda: *Thereuonema hilgendorfi, Scolopendra subspinipes,* and the like;
   Diplopoda: *Oxidus gracilis, Nedyopus tambanus,* and the like;
   Isopoda: *Armadillidium vulgare,* and the like;
   Gastropoda: *Limax marginatus, Limax flavus,* and the like;
   Nematoda: *Pratylenchus coffeae, Pratylenchus fallax,* *Heterodera glycines, Globodera rostochiensis, Meloidogyne hapla, Meloidogyne incognita,* and the like.

The pesticide composition of the present invention contains the compound according to the present invention and an inert carrier. Generally, it is a formulation obtained by mixing the compound according to the present invention and a carrier such as a solid carrier, a liquid carrier and a gaseous carrier, and if necessary, adding a surfactant and other adjuvant for formulation. The formulation includes, for example, an emulsifiable concentrate, an oil solution, a shampoo formulation, a flowable , a dust, a wettable powder, a granule, a paste formulation, a microcapsule, a foam, an aerosol, a carbon dioxide gas formulation, a tablet, a resin preparation and the like. These formulations can be converted for use into a poison bait, a mosqito coil, an electric mosquito mat, a smoking agent, a fumigant or sheet.

In the pesticide composition of the present invention, the compound according to the present invention is usually contained in an amount of 0.1% to 95% by weight.

The solid carrier for formulation includes, for example, a fine power and a granule of clays (e.g. , kaolin clay, diatomite, bentonite, Fubasami clay, acid clay, etc.), synthetic hydrated silicon oxide, talc, ceramic, other inorganic minerals (e.g., sericite, quartz, sulfur, activated carbon, calcium carbonate, hydrated silica) or chemical fertilizers (e.g., ammonium sulfate, ammonium phosphate, ammonium nitrate, ammonium chloride, urea).

The liquid carrier for formulation includes, for example, aromatic or aliphatic hydrocarbons (e.g., xylene, toluene, alkylnaphthalene, phenylxylylethane, kerosine, light oil, hexane, cyclohexane), halogenated hydrocarbons (e.g., chlorobenzene, dichloromethane, dichloroethane, trichloroethane), alcohols (e.g., methanol, ethanol, isopropyl alcohol, butanol, hexanol, ethylene glycol), ethers (e.g., diethyl ether, ethylene glycol dimethyl ether, diethylene glycol monomethyl ether, diethylene glycol monoethyl ether, propylene glycol monomethyl ether, tetrahydrofuran, dioxane), esters (e.g., ethyl acetate, butyl acetate), ketones (e.g., acetone, methyl ethyl ketone, methyl isobutyl ketone, cyclohexanone), nitriles (e.g., acetonitrile, isobutyronitrile), sulfoxides (e.g., dimethylsulfoxide), acid amides (e.g., N,N-dimethylformamide, N,N-dimethylacetamide), vegetable oils (e.g., soy bean oil, cotton seed oil) , vegetable essential oils (e.g., orange oil, hyssop oil, lemon oil) and water.

The gaseous carrier for formulation includes, for example, butane gas, chlorofluorocarbons, liquefied petroleum gas (LPG), dimethyl ether, carbon dioxide and the like.

The surfactant for formulation includes, for example, alkyl sulfate salts, alkylsulfonic acid salts, alkylarylsulfonic acid salts, alkyl aryl ethers and their polyoxyethylene derivatives, polyethylene glycol ethers, polyhydric alcohol esters, and sugar alcohol derivatives.

The other adjuvant for formulation includes, for example, binders, dispersants and stabilizers, and specifically, for example, casein, gelatin, polysaccharides (e.g., starch, gum arabic, cellulose derivatives, alginic acid), lignin derivatives, bentonite, sugars, synthetic water-soluble polymers (e.g., polyvinyl alcohol, polyvinylpyrrolidone, polyacrylic acid), PAP (isopropyl acid phosphate), BHT (2,6-di-t-butyl-4-methylphenol), BHA (a mixture of 2-t-butyl-4-methoxyphenol and 3-t-butyl-4-methoxyphenol), vegetable oils, mineral oils, fatty acids, and fatty acid esters.

The base for resin formulation includes, for example, polyvinyl chloride, polyurethane and the like. To these bases, if necessary, a plasticizer such as phthalate (e.g., dimethyl phthalate, dioctylphthalate), adipate and stearate may be added. The resin formulation can be obtained by kneading the compound into the base using a known kneader and then formulating by injectionmolding, extrusionmolding, pressmoldingandthelike, and further, if necessary, via a process for molding, cutting and the like, the resin formulation can be converted into a resin preparation such as board, film, tape, net, string and the like. These resin preparations can be converted into, for example, an animal collar, an animal ear tag, a sheet formulation, an attraction string, a gardening stick.

A base for the poison bait includes, for example, grain powders, vegetable oils, sugars, and crystalline cellulose, and further, if necessary, antioxidants such as dibutylhydroxytoluene and nordihydroguaiaretic acid, preservatives such as dehydroacetic acid, agents for preventing children and pets from erroneously eating such as hot pepper powder, and pest-attractive flavors such as cheese flavor, onion flavor and peanut oil may be added to the base.

Pests can be controlled by applying an effective amount of the present invention compound to pests directly and/or habitats of pests (e.g., plant, animal, soil). Usually the formulation of the pesticide composition of the present invention is used for the compound according to the present invention.

When the pesticide composition of the present invention is used for a control of pests in agriculture and forestry, the application amount is usually 1 to 10,000 g/ha, preferably 10 to 1,000 g/ha, as an active ingredient. The emulsifiable concentrates, wettable powders, flowables, and microcapsule formulations are usually applied after dilution with water to have an active ingredient concentration of 1 to 10,000 ppm, while dusts and granules are usually applied as such. These formulations may be sprayed directly to the plant to be protected from pests. The pests living in the soil can be controlled by treating the soil with these formulations, and the formulations can also be applied to treat seedbeds prior to the planting plants or to treat planting holes or plant bottoms in the planting. Furthermore, the sheet formulation of the pesticide composition of the present invention can be applied by a method such as winding around plants, stretching in the vicinity of plants and laying on the soil surface at the plant bottom.

When the pesticide composition of the present invention is used for a control of epidemic, the application amount is usually 0.001 to 10 mg/m³ as an active ingredient in case of application for open space, and 0.001 to 100 mg/m² as an active ingredient in case of application for plane surface. The emulsifiable conscentrate, wettable powders, flowables, and microcapsule formulations are usually applied after dilution with water to have an active ingredient concentration of 0.01 to 10,000 ppm, while oil solutions, aerosols, smoking agents and poison baits are usually applied as such.

When the pesticide composition of the present invention is used for a control of parasite living outside of a livestock such as caw, horse, pig, sheep, goat and chicken, and a small animal such as dog, cat, rat and mouse, the pesticide composition can be applied to said animal by a veterinarily known method. Specifically, for systemic control, the pesticide composition is administered by means of, for example, a tablet, a mixture with feed, a suppository or an injection (e.g., intramuscular, subcutaneous, intravenous, intraperitoneal), and for non-systemic control, it is applied by a method such as spraying an oil solution or an aqueous liquid formulation, carrying out pour-on treatment or spot-on treatment, washing said animal with a shampoo formulation, attaching the resin formulation on said animal as a collar or an ear-tag, and the like. When the present invention compound is administered to an animal, its amount is usually in the range of 0.1 to 1,000 mg/kg body weight of the animal.

The pesticide composition of the present invention can also be used in admixture or combination with other insecticides, nematocides, acaricides, fungicides, herbicides, plant growth regulators, synergists, fertilizers, soil conditioners, animal feeds, and the like.

The active ingredients of such other insecticide and acaricide include, for example, pyrethroid compounds such as allethrin, tetramethrin, prallethrin, phenothrin, resmethrin, cyphenothrin, permethrin, cypermethrin, alpha-cypermethrin, zeta-cypermethrin, deltamethrin, tralomethrin, cyfluthrin, beta-cyfluthrin, cyhalothrin, lambda-cyhalothrin, flumethrin, imiprothrin, etofenprox, fenvalerate, esfenvalerate, fenpropathrin, silafluofen, bifenthrin, transfluthrin, flucythrinate, tau-fluvalinate, acrinathrin and tefluthrin; organophosphorus compounds such as dichlorvos, fenitrothion, cyanophos, profenofos, sulprofos, phenthoate, isoxathion, tetrachlorvinphos,fenthion,chlorpyriphos,diazinon,acephate, terbufos, phorate, chlorethoxyfos, fosthiazate, ethoprophos, cadusafos andmethidathion; carbamate compounds such as propoxur, carbaryl, metoxadiazone, fenobucarb, methomyl, thiodicarb, alanycarb, benfuracarb, oxamyl, aldicarb and methiocarb; benzoylphenylurea compounds such as lufenuron, chlorfluazuron, hexaflumuron, diflubenzuron, triflumuron, teflubenzuron, flufenoxuron, fluazuron, novaluron and triazuron; juvenile hormone-like substances such as pyriproxyfen, methoprene, hydroprene and fenoxycarb; neonicotinoid compounds such as acetamiprid, nitenpyram, thiacloprid, thiamethoxam and dinotefuran; N-phenyl-pyrazole compounds such as acetoprole and ethiprole; benzoylhydrazine compounds such as tebufenozide, chromafenozide, methoxyfenozide and halofenozide; diafenthiuron; pymetrozine; flonicamid; triazamate; buprofezin; spinosad; emamectin benzoate; chlorfenapyr; indoxacarb MP; pyridalyl; cyromazine; fenpyroximate; tebufenpyrad; tolfenpyrad; pyridaben; pyrimidifen; fluacrypyrim;etoxazole;fenazaquin;acequinocyl;hexythiazox; clofentezine; fenbutatin oxide; dicofol, propargite; abamectin; milbemectin; amitraz; cartap; bensultap; thiocyclam; endosulfan; spirodiclofen; spiromesifen; and azadirachtin.

The active ingredients of such other fungicide include, for example, strobilurin compounds such as azoxystrobin; organophosphorus compounds such as tolclofos-methyl; azole compounds such as triflumizole, pefurazoate and difenoconazole; phthalide; flutolanil; validamycin; probenazole; diclomezine; pencycuron; dazomet; kasugamycin; IBP; pyroquilon; oxolinic acid; tricyclazole; f erimzone; mepronil; EDDP; isoprothiolane; carpropamid;diclocymet;furametpyr;fludioxonil;procymidone; and diethofencarb.

The present invention is construed in more detail by production examples, formulation examples and test examples, but should not be limited thereto. Firstly, production examples of the compound of the present invention are illustrated.

### Production Example 1

0.21 g of 2-t-butyl-5-chloromethylpyrimidine and 0:19 g of (3,3,3-trifluoropropyl)malononitrile were dissolved in 10 ml of N,N-dimethylformamide and then added with 0.16 g of potassium carbonate under ice cooling, followed by stirring at room temperature overnight. The reaction mixture was added with saturated brine, followed by extraction with t-butylmethylether. The organic phase was washed by saturated brine and then dried with an anhydrous sodium sulfate, followed by concentration under reduced pressure. The residue was subjected to a silica gel column chromatography to obtain 0.22 g of [(2-t-butylpyrimidine-5-yl)methyl](3,3,3-trifluoropropyl)malononitrile (which is referred to as the compound of the present invention (1) hereinafter).
¹H-NMR (CDCl₃, TMS) , δ (ppm) : 1.44 (9H, s), 2.28-2.32 (2H, m), 2.52-2.26 (2H, m), 3.24 (2H, s), 8.74 (2H, s)

### Production Example 2

0.15 g of 2-t-butyl-5-chloromethylpyrazine and 0.13 g of (3,3,3-trifluoropropyl)malononitrile were dissolved in 5 ml of N,N-dimethylformamide and then added with 0.11g of potassium carbonate under ice cooling, followed by stirring at room temperature overnight.
The reaction mixture was added with saturated brine, followed by extraction with t-butylmethylether. The organic phase was washed by saturated brine and then dried with an anhydrous sodium sulfate, followed by concentration under reduced pressure. The residue was subjected to a silica gel column chromatography to obtain 0.13 g of [(2-t-butylpyrazine-5-yl)methyl](3,3,3-trifluoropropyl)malononitrile (which is referred to as the compound of the present invention (2) hereinafter).
¹H-NMR (CDCl₃, TMS), δ (ppm) : 1.45 (9H, s), 2.37-2.41 (2H, m), 2.55-2.64 (2H, m), 3.47 (2H, s), 8.55 (1H, s), 8.70 (1H, s)

### Production Example 3

0.12 g of 3-t-butyl-6-chloromethylpyridazine and 0.10 g of (3,3,3-trifluoropropyl)malononitrile were dissolved in 5 ml of N,N-dimethylf ormamide and then added with 0.087 g of potassium carbonate under ice cooling, followed by stirring at room temperature overnight. The reaction mixture was added with saturated brine, followed by extraction with t-butylmethylether. The organic phase was washed by saturated brine and then dried with an anhydrous sodium sulfate, followed by concentration under reduced pressure. The residue was subjected to a silica gel column chromatography to obtain 0.15 g of [(6-t-butylpyridazine-3-yl)methyl](3,3,3-trifluoropropyl)malononitrile (which is referred to as the compound of the present invention (3) hereinafter).
¹H-NMR (CDCl₃, TMS)*,* δ (ppm) : 1.48 (9H, s), 2.37-2.41 (2H, m), 2.60-2.64 (2H, m), 3.65 (2H, s), 7.51 (1H, d), 7.61 (1H, d)

### Production Example 4

100 mg of 5-chloromethyl-2-methylthiopyrimidine and 93 mg of (3,3,3-trifluoropropyl)malononitrile were dissolved in 3 ml of N,N-dimethylformamide and then added with 80 mg of potassium carbonate under ice cooling, followed by stirring at room temperature overnight. The reaction mixture was added with saturated brine, followed by extraction with t-butylmethylether. The organic phase was washed by saturated brine and then dried with an anhydrous sodium sulfate, followed by concentration under reduced pressure. The residue was subjected to a silica gel column chromatography to obtain 0.10 g of [(2-methylthiopyrimidine-5-yl)methyl](3,3,3-trifluoropropyl) malononitrile (which is referred to as the compound of the present invention (4) hereinafter).
¹H-NMR (CDCl₃, TMS) , δ (ppm) : 2.26-2.30 (2H, m) , 2.51-2.63 (2H, m), 2.60 (3H, s), 3.20 (2H, s), 8.57 (2H, s)

### Production Example 5

0.84 g of [(2-methylthiopyrimidine-5-yl)methyl](3,3,3-trifluoropropyl) malononitrile was dissolved in 30 ml of chloroform and then added with 0.97 g of m-chloroperbenzoic acid under ice cooling, followed by stirring at room temperature for 6 hours. The reaction mixture was successively washed by 3 % aqueous solution of sodium thiosulfate and by saturated aqueous solution of sodium hydrogen carbonate. The organic phase was dried with an anhydrous sodium sulfate, followed by concentration under reduced pressure to obtain 0.76 g of [(2-methanesulfonylpyrimidine-5-yl)methyl](3,3,3-trifluoropropyl) malononitrile (which is referred to as the compound of the present invention (5) hereinafter).
¹H-NMR (DMSO-d₆, TMS), δ (ppm) : 2.50-2.56 (2H, m), 2.61-2.72 (2H, m), 3.45 (3H, s), 3.77 (2H, s), 9.12 (2H, s)

### Production Example 6

0.50 g of [(2-methanesulfonylpyrimidine-5-yl)methyl](3,3,3-trifluoropropyl) malononitrile was dissolved in 5 ml of dimethylsulfoxide and then added with 0.10 g of potassium cyanide, followed by stirring at room temperature for 8 hours. The reaction mixture was added with water, followed by extraction with t-butylmethylether. The organic phase was successively washed by saturated aqueous solution of sodium hydrogen carbonate and by saturated brine and then dried with an anhydrous sodium sulfate, followed by concentration under reduced pressure. The residue was subjected to a silica gel column chromatography to obtain 0.12 g of [(2-cyanopyrimidine-5-yl)methyl](3,3,3-trifluoropropyl)malononitrile (which is referred to as the compound of the present invention (6) hereinafter).
¹H-NMR (CDCl₃, TMS), δ (ppm) : 2.35-2.40 (2H, m), 2.55-2.64 (2H, m), 3.36 (2H, s), 8.95 (2H, s)

### Production Example 7

70 mg of propargylalcohol was dissolved in 10 ml of tetrahydrofuran and then added with 50 mg of sodium hydride (60 % oiliness) under stirring at room temperature. Then, being stirred at a room temperature for one hour. Successively, the reaction mixture was added with 400 mg of [(2-methanesulfonylpyrimidine-5-yl)methyl](3,3,3-trifluoropropyl)malononitrile and then further stirred at room temperature for 3 hours, and the reaction mixture was added with saturated aqueous solution of ammoniumchloride, followed by extraction with t-butylmethylether. The organic phase was washed by saturated brine and then dried with an anhydrous sodium sulfate, followed by concentration under reduced pressure. The residue was subjected to a silica gel column chromatography to obtain 0.10 g of {[2-(propargyloxy)pyrimidine-5-yl]methyl}(3,3,3-trifluoropropyl)malononitrile (which is referred to as the compound of the present invention (7) hereinafter).
¹H-NMR (CDCl₃, TMS), δ (ppm) : 2.26-2.31 (2H, m), 2.50 (1H, t), 2.54-2.61 (2H, m), 3.23 (2H, s), 5.07 (2H, d), 8.61 (2H, s)

### Production Example 8

50 mg of 2-butyne-1-ol was dissolved in 5 ml of tetrahydrofuran and then added with 30 mg of sodium hydride (60 % oiliness) at room temperature, followed by stirring at room temperature for one hour. Successively, the reaction mixture was added with 250 mg of [(2-methanesulfonylpyrimidine-5-yl)methyl](3,3,3-trifluoropropyl) malononitrile, followed by stirring at room temperature for 3 hours. The reaction mixture was added with saturated aqueous solution of ammoniumchloride, followed by extraction with t-butylmethylether. The organic phase was washed by saturated brine and then dried with an anhydrous sodium sulfate, followed by concentration under reduced pressure. The residue was subjected to a silica gel column chromatography to obtain 0.10 g of {[2-(2-butynyloxy)pyrimidine-5-yl]methyl}(3,3,3-trifluoropropyl)malononitrile (which is referred to as the compound of the present invention (8) hereinafter).
¹H-NMR (CDCl₃, TMS), δ (ppm) : 1.87 (3H, t), 2.26-2.30 (2H, m), 2.54-2.61 (2H, m), 3.22 (2H, s), 5.03 (2H, q), 8.60 (2H, s)

### Production Example 9

0.24 g of 5-chloromethyl-2-methylpyrimidine and 0.27 g of (3,3,3-trifluoropropyl)malononitrile were dissolved in 5 ml of N,N-dimethylformamide and then added with 0.32 g of potassium carbonate under ice cooling, followed by stirring at room temperature for overnight. The reaction mixture was added with saturated brine, followed by extraction with t-butylmethylether. The organic phase was washed by saturated brine and then dried with an anhydrous sodium sulfate, followed by concentration under reduced pressure. The residue was subjected to a silica gel column chromatography to obtain 0.11 g of [(2-methylpyrimidine-5-yl)methyl](3,3,3-trifluoropropyl)malononitrile (which is referred to as the compound of the present invention (9) hereinafter).
¹H-NMR (CDCl₃, TMS), δ (ppm) : 2.27-2.31 (2H, m), 2.54-2.61 (2H, m), 2.80 (3H, s), 3.24 (2H, s), 8.71 (2H, s)

### Production Example 10

1.0 g of 5-chloromethyl-2-isopropylpyrimidine and 0.95 g of (3,3,3-trifluoropropyl)malononitrile were dissolved in 15 ml of N,N-dimethylformamide and then added with 0.81 g of potassium carbonate under ice cooling, followed by stirring at room temperature overnight. The reaction mixture was added with saturated brine, followed by extraction with t-butylmethylether. The organic phase was washed by saturated brine and then dried with an anhydrous sodium sulfate, followed by concentration under reduced pressure. The residue was subjected to a silica gel column chromatography to obtain 0.40g of [(2-isopropylpyrimidine-5-yl)methyl](3,3,3-trifluoropropyl) malononitrile (which is referred to as the compound of the present invention (10) hereinafter).
¹H-NMR (CDCl₃, TMS), δ (ppm) : 1.38 (6H, d) , 2.28-2.32 (2H, m) , 2.55-2.61 (2H, m), 3.24 (2H, s), 3.28 (1H, m), 8.73 (2H, s)

### Production Example 11

0.75 g of 5-bromomethyl-2-phenylpyrimidine and 0.49 g of (3,3,3-trifluoropropyl)malononitrile were dissolved in 15 ml of N,N-dimethylformamide and then added with 0.42 g of potassium carbonate under ice cooling, followed by stirring at room temperature overnight. The reaction mixture was added with saturated brine, followed by extraction with t-butylmethylether. The organic phase was washed by saturated brine and then dried with an anhydrous sodium sulfate, followed by concentration under reduced pressure. The residue was subjected to a silica gel column chromatography to obtain 0.42 g of [(2-phenylpyrimidine-5-yl)methyl](3,3,3-trifluoropropyl)malononitrile (which is referred to as the compound of the present invention (11) hereinafter).
¹H-NMR (CDCl₃, TMS), δ (ppm) : 2.30-2.34 (2H, m) , 2.53-2.65 (2H, m), 3.31 (2H, s), 7.49-7.55 (3H, m), 8.47-8.50 (2H, m), 8.86 (2H, s)

### Production Example 12

0.16 g of 5-(chloromethyl)pyrimidine and 0.15 g of (3,3,3-trifluoropropyl)malononitrilewere dissolved in 5 ml of N,N-dimethylformamide and then added with 0.13 g of potassium carbonate under ice cooling, followed by stirring at room temperature overnight. The reaction mixture was added with saturated brine, followed by extraction with t-butylmethylether. The organic phase was washed by saturated brine and then dried with an anhydrous sodium sulfate, followed by concentration under reduced pressure. The residue was subjected to a silica gel column chromatography to obtain 0.11 g of [(pyrimidine-5-yl)methyl](3,3,3-trifluoropropyl)malononitrile (which is referred to as the compound of the present invention (12) hereinafter).
¹H-NMR (CDCl₃, TMS), δ (ppm) : 2.30-2.34 (2H, m) , 2.53-2.65 (2H, m), 3.29 (2H, s), 8.84 (2H, s), 9.32 (1H, s)

### Production Example 13

0.18 g of 4-t-butyl-2-chloromethylpyrimidine and 0.16 g of (3,3,3-trifluoropropyl)malononitrile were dissolved in 5 ml of N,N-dimethylformamide and then added with 0.14 g of potassium carbonate under ice cooling, followed by stirring at room temperature overnight. The reaction mixture was added with saturated brine, followed by extraction with t-butylmethylether. The organic phase was washed by saturated brine and then dried with an anhydrous sodium sulfate, followed by concentration under reduced pressure. The residue was subjected to a silica gel column chromatography to obtain 0.08 g of [(4-t-butylpyrimidine-2-yl)methyl](3,3,3-trifluoropropyl)malononitrile (which is referred to as the compound of the present invention (13) herein after).
¹H-NMR (CDCl₃, TMS), δ (ppm) : 1.39 (9H, s), 2.42-2.46 (2H, m), 2.59-2.70 (2H, m), 3.64 (2H, s), 7.29 (1H, d), 8.65 (1H, d)

### Production Example 14

0.50 g of 5-bromomethyl-2-chloropyrimidine and 0.39 g of (3,3,3-trifluoropropyl)malononitrile were dissolved in 10 ml of N,N-dimethylformamide and then added with 0.33 g of potassium carbonate under ice cooling, followed by stirring at room temperature overnight. The reaction mixture was added with saturated brine, followed by extraction with t-butylmethylether. The organic phase was washed by saturated brine and then dried with an anhydrous sodium sulfate, followed by concentration under reduced pressure. The residue was subjected to a silica gel column chromatography to obtain 0.21 g of [(2-chloropyrimidine-5-yl)methyl](3,3,3-trifluoropropyl)malononitrile represented by the formula as shown below (which is referred to as the compound of the present invention (14) hereinafter).
¹H-NMR (CDCl₃, TMS) , δ (ppm) : 2.30-2.35 (2H, m) , 2.53-2.65 (2H, m), 3.27 (2H, s), 8.71 (2H, s)

### Production Example 15

80 mg of 4-bromomethyl-2-chloropyrimidine and 63 mg of (3,3,3-trifluoropropyl)malononitrile were dissolved in 3 ml of N,N-dimethylformamide and then added with 53 mg of potassium carbonate under ice cooling, followed by stirring at room temperature overnight. The reaction mixture was added with saturated brine, followed by extraction with t-butylmethylether. The organic phase was washed by saturated brine and then dried with an anhydrous sodium sulfate, followed by concentration under reduced pressure. The residue was subjected to a silica gel column chromatography to obtain 0.07 g of [(2-chloropyrimidine-4-yl)methyl](3,3,3-trifluoropropyl)malononitrile (which is referred to as the compound of the present invention (15) hereinafter).
¹H-NMR (CDCl₃, TMS) , δ (ppm) : 2.37-2.41 (2H, m) , 2.55-2.67 (2H, m), 3.44 (2H, s), 7.36 (1H, d), 8.73 (1H, d)

### Production Example 16

0.58 g of 5-bromomethyl-2-trifluoromethylpyrimidine and 0. 39 g of (3,3,3-trifluoropropyl)malononitrile were dissolved in 10 ml of N,N-dimethylformamide and then added with 0.33 g of potassium carbonate under ice cooling, followed by stirring at room temperature overnight. The reaction mixture was added with saturated brine, followed by extraction with t-butylmethylether. The organic phase was washed by saturated brine and then dried with an anhydrous sodium sulfate, followed by concentration under reduced pressure. The residue was subjected to a silica gel column chromatography to obtain 0.55 g of [(2-trifluoromethylpyrimidine-5-yl)methyl](3,3,3-trifluoropropyl) malononitrile (which is referred to as the compound of the present invention (16) hereinafter).
¹H-NMR (CDCl₃, TMS), (δ (ppm) : 2.35-2.39 (2H, m), 2.55-2.65 (2H, m), 3.38 (2H, s), 9.00 (2H, s)

### Production Example 17

0.30 g of 5-bromomethyl-2-trifluoromethylpyrimidine and 0.13 g of allylmalononitrile were dissolved in 5 ml of N,N-dimethylformamide and then added with 0.17 g of potassium carbonate under ice cooling, followed by stirring at room temperature overnight. The reaction mixture was added with saturated brine, followed by extraction with t-butylmethylether. The organic phase was washed by saturated brine and then dried with an anhydrous sodium sulfate, followed by concentration under reduced pressure. The residue was subjected to a silica gel column chromatography to obtain 0.23 g of allyl[(2-trifluoromethylpyrimidine-5-yl)methyl]malononitrile (which is referred to as the compound of the present invention (17) hereinafter).
¹H-NMR (CDCl₃, TMS) , δ (ppm) 2.85 (2H, d), 3.30 (2H, s), 5.49-5.58 (2H, m), 5.97 (1H, m), 8.98 (2H, s)

### Production Example 18

0.30 g of 5-bromomethyl-2-trifluoromethylpyrimidine and 0.15 g of 3-butenylmalononitrile were dissolved in 5 ml of N,N-dimethylformamide and then added with 0.17 g of potassium carbonate under ice cooling, followed by stirring at room temperature overnight. The reaction mixture was added with saturated brine, followed by extraction with t-butylmethylether. The organic phase was successively washed by saturated brine and then dried with an anhydrous sodium sulfate, followed by concentration under reduced pressure. The residue was subjected to a silica gel column chromatography to obtain 0.21 g of 3-butenyl[(2-trifluoromethylpyrimidine-5-yl)methyl]malononitrile (which is referred to as the compound of the present invention (18) hereinafter).
¹H-NMR (CDCl₃, TMS), δ (ppm) : 2.14-2.18 (2H, m), 2.51-2.56 (2H, m), 3.33 (2H, s), 5.16-5.24 (2H, m), 5.83 (1H, m), 8.94 (2H, s)

### Production Example 19

0.17 g of 5-chloromethyl-2-isopropylpyrimidine and 0.11 g of allylmalononitrile were dissolved in 3 ml of N,N-dimethylformamide and then added with 0.14 g of potassium carbonate under ice cooling, followed by stirring at room temperature overnight. The reaction mixture was added with saturated brine, followed by extraction with t-butylmethylether. The organic phase was washed by saturated brine and then dried with an anhydrous sodium sulfate, followed by concentration under reduced pressure. The residue was subjected to a silica gel column chromatography to obtain 0.12 g of allyl[(2-isopropylpyrimidine-5-yl)methyl]malononitrile (which is referred to as the compound of the present invention (19) hereinafter).
¹H-NMR (CDCl₃, TMS) , δ (ppm) : 1.37 (6H, d), 2.78 (2H, d), 3.17 (2H, s), 3.27 (1H, m), 5.45-5.53 (2H, m), 5.95 (1H, m), 8.72 (2H, s)

### Production Example 20

80 mg of 2-(bromomethyl)pyrazine and 100 mg of (3,3,3-trifluoropropyl)malononitrile were dissolved in 3 ml of N,N-dimethylformamide and then added with 86 mg of potassium carbonate under ice cooling, followed by stirring at room temperature overnight. The reaction mixture was added with saturated brine, followed by extraction with t-butylmethylether. The organic phase was washed by saturated brine and then dried with an anhydrous sodium sulfate, followed by concentration under reduced pressure. The residue was subjected to a silica gel column chromatography to obtain 0.10 g of [(pyrazine-2-yl)methyl](3,3,3-trifluoropropyl)malononitrile (which is referred to as the compound of the present invention (20) hereinafter).
¹H-NMR (CDCl₃, TMS) , δ (ppm) : 2.37-2.41 (2H, m), 2.55-2.64 (2H, m), 3.52 (2H, s), 8.64-8.66 (3H, m)

### Production Example 21

0.27 g of 3-bromomethyl-6-chloropyridazine and 0.21 g of (3,3,3-trifluoropropyl)malononitrile were dissolved in 5 ml of N,N-dimethylformamide and then added with 0.18 g of potassium carbonate under ice cooling, followed by stirring at room temperature overnight. The reaction mixture was added with saturated brine, followed by extraction with t-butylmethylether. The organic phase was washed by saturated brine and then dried with an anhydrous sodium sulfate, followed by concentration under reduced pressure. The residue was subjected to a silica gel column chromatography to obtain 0.20 g of [(6-chloropyridazine-3-yl)methyl](3,3,3-trifluoropropyl)malononitrile (which is referred to as the compound of the present invention (21) hereinafter).
¹H-NMR (CDCl₃, TMS) , δ (ppm) : 2.36-2.41 (2H, m) , 2.54-2.65 (2H, m), 3.69 (2H, s), 7.59 (1H, d), 7.64 (1H, d)

### Production Example 22

0.23 g of 2-bromomethyl-5-methoxycarbonylpyrimidine and 0.16 g of (3,3, 3-trifluoropropyl)malononitrile were dissolved in 5 ml of N,N-dimethylformamide and then added with 0.14 g of potassium carbonate under ice cooling, followed by stirring at room temperature overnight. The reaction mixture was added with saturated brine, followed by extraction with t-butylmethylether. The organic phase was washed by saturated brine and then dried with an anhydrous sodium sulfate, followed by concentration under reduced pressure. The residue was subjected to a silica gel column chromatography to obtain 0.21 g of [(5-methoxycarbonyl-2-yl)methyl](3,3,3-trifluoropropyl)malononitrile (which is referred to as the compound of the present invention (22) hereinafter).
¹H-NMR (CDCl₃, TMS), δ (ppm) : 2.42-2.46 (2H, m), 2.58-2.69 (2H, m), 3.73 (2H, s), 4.01 (3H, s), 9.31 (2H, s)

### Production Example 23

0.34 g of 2-chloromethyl-6,7-dihydro-5H-cyclopentapyrimidine 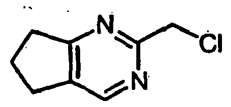 and 0.33 g of (3,3,3-trifluoropropyl)malononitrile were dissolved in 5 ml of N,N-dimethylformamide and then added with 0.28 g of potassium carbonate under ice cooling, followed by stirring at room temperature overnight. The reaction mixture was added with saturated brine, followed by t-butylmethylether. The organic phase was washed by saturated brine and then dried with an anhydrous sodium sulfate, followed by concentration under reduced pressure. The residue was subjected to a silica gel column chromatography to obtain 0.44 g of [(6,7-dihydro-5H-cyclopentapyrimidine-2-yl)methyl](3,3,3-trifluoropropyl)malononitrile 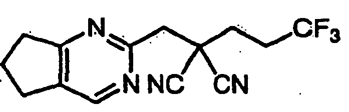 (which is referred to as the compound of the present invention (23) hereinafter).
¹H-NMR (CDCl₃, TMS), δ (ppm) : 2.15-2.23 (2H, m), 2.39-2.43 (2H, m), 2.56-2.68 (2H, m), 2.97-3.05 (4H, m), 3.59 (2H, s), 8.53 (1H,s)

### Production Example 24

138 mg of 2-chloromethyl-7,7-dimethyl-6,7-dihydro-5H-cyclopentapyrimidine 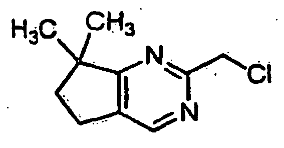 and 114 mg of (3,3,3-trifluoropropyl)malononitrile were dissolved in 3 ml of N,N-dimethylformamide and then added with 97 mg of potassium carbonate under ice cooling, followed by stirring at room temperature overnight. The reaction mixture was added with saturated brine, followed by extraction with t-butylmethylether. The organic phase was washed by saturated brine and then dried with an anhydrous sodium sulfate, followed by concentration under reduced pressure. The residue was subjected to a silica gel column chromatography to obtain 0.19 g of [(7,7-dimethyl-6,7-dihydro-5H-cyclopentapyrimidine-2-yl)methyl](3,3,3-trifluoropropyl)malononitrile 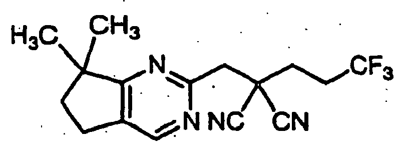 (which is referred to as the compound of the present invention (24) hereinafter).
¹H-NMR (CDCl₃, TMS), δ (ppm) : 1.31 (6H, s), 2.03 (2H, t), 2.41-2.45 (2H, m), 2.59-2.66 (2H, m), 2.92 (2H, t), 3.62 (2H, s), 8.51 (1H, s)

### Production Example 25

0.30 g of 5-bromomethyl-2-trifluoromethylpyrimidine and 0.13 g of propargylmalononitrile were dissolved in 5 ml of N,N-dimethylformamide and then added with 0.17 g of potassium carbonate under ice cooling, followed by stirring at room temperature overnight. The reaction mixture was added with saturated brine, followed by extraction with t-butylmethylether. The organic phase was successively washed by saturated brine and then dried with an anhydrous sodium sulfate, followed by concentration under reduced pressure. The residue was subjected to a silica gel column chromatography to obtain 0.17 g of (propargyl)[(2-trifluoromethylpyrimidine-5-yl)methyl]malononitrile (which is referred to as the compound of the present invention (25) hereinafter).
¹H-NMR (CDCl₃, TMS), δ (ppm) : 2.59 (1H, t), 3.03 (2H, d), 3.50 (2H, s), 9.02 (2H, s)

### Production Example 26

0.20 g of 5-bromomethyl-2-trifluoromethylpyrimidine and 0.30 g of 2,2,3,3-tetrafluoropropylmalononitrile were dissolved in 5 ml of N,N-dimethylformamide and then added with 0.23 g of potassium carbonate under ice cooling, followed by stirring at room temperature overnight. The reaction mixture was added with saturated brine, followed by extraction with t-butylmethylether. The organic phase was successively washed by saturated brine and then dried with an anhydrous sodium sulfate, followed by concentration under reduced pressure. The residue was subjected to a silica gel column chromatography to obtain 0.24 g of (2,2,3,3-tetrafluoropropyl)[(2-trifluoromethylpyrimidine-5-yl)methyl]malononitrile (, which is referred to as the compound of the present invention (26) hereinafter).
¹H-NMR (CDCl₃, TMS) , δ (ppm) 3.00 (2H, t), 3.60 (2H, s), 5.96 (1H, tt), 9.06 (2H, s)

### Production Example 27

0.40 g of 2-chloromethyl-7,7-dimethyl-6,7-dihydro-5H-cyclopentapyrimidine and 0.22 g of allylmalononitrile were dissolved in 10 ml of N,N-dimethylformamide and then added with 0.28 g of potassium carbonate under ice cooling, followed by stirring at room temperature overnight. The reaction mixture was added with saturated brine, followed by extraction with t-butylmethylether.
The organic phase was washed by saturated brine and then dried with an anhydrous sodium sulf ate, followedby concentration under reduced pressure. The residue was subjected to a silica gel column chromatography to obtain 0.34 g of allyl[(7,7-dimethyl-6,7-dihydro-5H-cyclopentapyrimidine-2- yl)-methyl]malononitrile (which is referred to as the compound of the present invention (27) hereinafter). 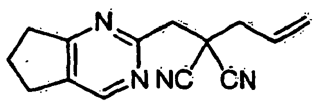 ¹H-NMR (CDCl₃, TMS), δ (ppm) : 1.31 (6H, s), 2.02 (2H, t), 2.90-2.93 (4H, m), 3.55 (2H, s), 5.42-5.47 (2H, m), 6.00 (1H, m), 8.51 (1H, s)

### Production Example 28

0.40 g of 2-chloromethyl-7,7-dimethyl-6,7-dihydro-5H-cyclopentapyrimidine and 0.24 g of 3-butenylmalononitrile were dissolved in 3 ml of N, N-dimethylformamide and then said mixed liquid was added with 0.28 g of potassium carbonate along with agitation under ice cooling, followed by stirring at room temperature overnight. The reaction mixture was added with saturated brine, followed by extraction with t-butylmethylether. The organic phase was washed by saturated brine and then dried with an anhydrous sodium sulfate, followed by concentration under reduced pressure. The residue was subjected to a silica gel column chromatography to obtain 0.29 g of 3-butenyl[(7,7-dimethyl-6,7-dihydro-5H-cyclopentapyrimidine-2-yl)methyl] malononitrile 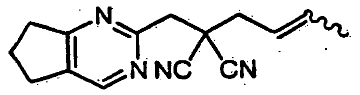 (which is referred to as the compound of the present invention (28) hereinafter).
¹H-NMR(CDCl₃,TMS), δ (ppm) : 1.31 (6H, s), 2.02 (2H, t), 2.19-2.23 (2H, m), 2.52-2.58(2H, m), 2.91 (2H, t), 3.58 (2H, s), 5.10-5.21 (2H, m), 5.84 (1H, m), 8.51 (1H, s)

Specific examples of the compound of the present invention are listed with their compound numbers in Table 1 to Table 13. The compounds are shown with respect to formula (I):

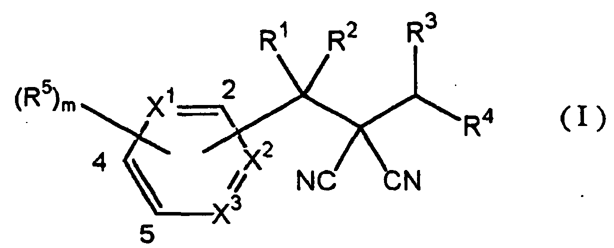

**Table 1**

| Compound Number | R¹ | R² | R³ | R⁴ | (R⁵)ₘ | X¹ | X² | X³ | bonding site |
|---|---|---|---|---|---|---|---|---|---|
| 1 | H | H | H | CH₂CF₃ | 2-C(CH₃)₃ | N | N | CH | 5 |
| 2 | H | H | H | CH₂CF₃ | 2-C(CH₃)₃ | N | CH | N | 5 |
| 3 | H | H | H | CH₂CF₃ | 2-C(CH₃)₃ | CH | N | N | 5 |
| 4 | H | H | H | CH₂CF₃ | 2-SCH₃ | N | N | CH | 5 |
| 5 | H | H | H | CH₂CF₃ | 2-S(O)₂CH₃ | N | N | CH | 5 |
| 6 | H | H | H | CH₂CF₃ | 2-CN | N | N | CH | 5 |
| 7 | H | H | H | CH₂CF₃ | 2-OCH₂CCH | N | N | CH | 5 |
| 8 | H | H | H | CH₂CF₃ | 2-OCH₂CCCH₃ | N | N | CH | 5 |
| 9 | H | H | H | CH₂CF₃ | 2-CH₃ | N | N | CH | 5 |
| 10 | H | H | H | CH₂CF₃ | 2-CH(CH₃)₂ | N | N | CH | 5 |
| 11 | H | H | H | CH₂CF₃ | 2-C₆H₅ | N | N | CH | 5 |
| 12 | H | H | H | CH₂CF₃ | | N | N | CH | 5 |
| 13 | H | H | H | CH₂CF₃ | 4-C(CH₃)₃ | N | N | CH | 2 |
| 14 | H | H | H | CH₂CF₃ | 2-Cl | N | N | CH | 5 |
| 15 | H | H | H | CH₂CF₃ | 2-Cl | N | N | CH | 4 |
| 16 | H | H | H | CH₂CF₃ | 2-CF₃ | N | N | CH | 5 |
| 17 | H | H | H | CH=CH₂ | 2-CF₃ | N | N | CH | 5 |
| 18 | H | H | H | CH₂CH=CH₂ | 2-CF₃ | N | N | CH | 5 |
| 19 | H | H | H | CH=CH₂ | 2-CH(CH₃)₂ | N | N | CH | 5 |
| 20 | H | H | H | CH₂CF₃ | | N | CH | N | 2 |
| 21 | H | H | H | CH₂CF₃ | 2-Cl | CH | N | N | 5 |
| 22 | H | H | H | CH₂CF₃ | 5-CO₂CH₃ | N | N | CH | 2 |
| 23 | H | H | H | CH₂CF₃ | 4,5-(CH₂)₃- | N | N | CH | 2 |
| 24 | H | H | H | CH₂CF₃ | 4,5-C(CH₃)₂(CH₂)₂- | N | N | CH | 2 |
| 25 | H | H | H | CCH | 2-CF₃ | N | N | CH | 5 |

**Table 2**

| Compound Number | R¹ | R² | R³ | R⁴ | (R⁵)ₘ | X¹ | X² | X³ | bonding site |
|---|---|---|---|---|---|---|---|---|---|
| 26 | H | H | H | CF₂CF₂H | 2-CF₃ | N | N | CH | 5 |
| 27 | H | H | H | CH=CH₂ | 4,5-C(CH₃)₂(CH₂)₂- | N | N | CH | 2 |
| 28 | H | H | H | CH₂CH=CH₂ | 4,5-C(CH₃)₂(CH₂)₂- | N | N | CH | 2 |
| 29 | H | H | H | CH=CF₂ | 2-CF₃ | N | N | CH | 5 |
| 30 | H | H | H | CH=CH(CF₃) | 2-CF₃ | N | N | CH | 5 |
| 31 | H | H | H | CH(Br)CF₃ | 2-CF₃ | N | N | CH | 5 |
| 32 | H | H | H | CF₂CF₃ | 2-CF₃ | N | N | CH | 5 |
| 33 | H | H | H | CH₂CH₂CF₃ | 2-CF₃ | N | N | CH | 5 |
| | | | | | | | | | |
| 34 | H | H | H | 2,2-dichloro-1-cyclopropyl | 2-CF₃ | N | N | CH | 5 |
| 35 | H | CH₃ | H | CH=CH₂ | 2-CF₃ | N | N | CH | 5 |
| 36 | H | CH₃ | H | CH₂CH=CH₂ | 2-CF₃ | N | N | CH | 5 |
| 37 | H | CH₃ | H | CH=CF₂ | 2-CF₃ | N | N | CH | 5 |
| 38 | H | CH₃ | H | CH=CH(CF₃) | 2-CF₃ | N | N | CH | 5 |
| 39 | H | CH₃ | H | CH(Br)CF₃ | 2-CF₃ | N | N | CH | 5 |
| 40 | H | CH₃ | H | CF₂CF₃ | 2-CF₃ | N | N | CH | 5 |
| 41 | H | CH₃ | H | CH₂CF₂H | 2-CF₃ | N | N | CH | 5 |
| 42 | H | CH₃ | H | CH₂CH₂CF₃ | 2-CF₃ | N | N | CH | 5 |
| | | | | | | | | | |
| 43 | H | CH₃ | H | 2,2-dichloro-1-cyclopropyl | 2-CF₃ | N | N | CH | 5 |
| 44 | H | H | H | CH=CH₂ | 2-C(CH₃)₃ | N | N | CH | 5 |
| 45 | H | H | H | CH₂CH=CH₂ | 2-C(CH₃)₃ | N | N | CH | 5 |
| 46 | H | H | H | CH=CF₂ | 2-C(CH₃)₃ | N | N | CH | 5 |
| 47 | H | H | H | CH=CH(CF₃) | 2-C(CH₃)₃ | N | N | CH | 5 |
| 48 | H | H | H | CH(Br)CF₃ | 2-C(CH₃)₃ | N | N | CH | 5 |
| 49 | H | H | H | CF₂CF₃ | 2-C(CH₃)₃ | N | N | CH | 5 |
| 50 | H | H | H | CH₂CF₂H | 2-C(CH₃)₃ | N | N | CH | 5 |

**Table 3**

| Compound Number | R¹ | R² | R³ | R⁴ | (R⁵)ₘ | X¹ | X² | X³ | bonding site |
|---|---|---|---|---|---|---|---|---|---|
| 51 | H | H | H | CH₂CH₂CF₃ | 2-C(CH₃)₃ | N | N | CH | 5 |
| | | | | | | | | | |
| 52 | H | H | H | 2,2-dichloro-1-cyclopropyl | 2-C(CH₃)₃ | N | N | CH | 5 |
| 53 | H | H | H | CH₂CH=CH₂ | 2-CH(CH₃)₂ | N | N | CH | 5 |
| 54 | H | H | H | CH=CF₂ | 2-CH(CH₃)₂ | N | N | CH | 5 |
| 55 | H | H | H | CH=CH(CF₃) | 2-CH(CH₃)₂ | N | N | CH | 5 |
| 56 | H | H | H | CH(Br)CF₃ | 2-CH(CH₃)₂ | N | N | CH | 5 |
| 57 | H | H | H | CF₂CF₃ | 2-CH(CH₃)₂ | N | N | CH | 5 |
| 58 | H | H | H | CH₂CF₂H | 2-CH(CH₃)₂ | N | N | CH | 5 |
| 59 | H | H | H | CH₂CH₂CF₃ | 2-CH(CH₃)₂ | N | N | CH | 5 |
| | | | | | | | | | |
| 60 | H | H | H | 2,2-dichloro-1-cyclopropyl | 2-CH(CH₃)₂ | N | N | CH | 5 |
| 61 | H | H | H | CH=CH₂ | 2-Cl | N | N | CH | 5 |
| 62 | H | H | H | CH₂CH=CH₂ | 2-Cl | N | N | CH | 5 |
| 63 | H | H | H | CH=CF₂ | 2-Cl | N | N | CH | 5 |
| 64 | H | H | H | CH=CH(CF₃) | 2-Cl | N | N | CH | 5 |
| 65 | H | H | H | CH(Br)CF₃ | 2-Cl | N | N | CH | 5 |
| 66 | H | H | H | CF₂CF₃ | 2-Cl | N | N | CH | 5 |
| 67 | H | H | H | CH₂CF₂H | 2-Cl | N | N | CH | 5 |
| 68 | H | H | H | CH₂CH₂CF₃ | 2-Cl | N | N | CH | 5 |
| | | | | | | | | | |
| 69 | H | H | H | 2,2-dichloro-1-cyclopropyl | 2-Cl | N | N | CH | 5 |
| 70 | H | H | H | CH₂CF₃ | 2-CH₂CH₃ | N | N | CH | 5 |
| 71 | H | H | H | CH₂CF₃ | 2-CF₂CF₃ | N | N | CH | 5 |
| 72 | H | H | H | CH₂CF₃ | 2-CF₂H | N | N | CH | 5 |
| 73 | H | H | H | CH₂CF₃ | 2-CH₂F | N | N | CH | 5 |
| 74 | H | H | H | CH₂CF₃ | 2-Br | N | N | CH | 5 |
| 75 | H | H | H | CH₂CF₃ | 2-F | N | N | CH | 5 |

**Table 4**

| Compound Number | R¹ | R² | R³ | R⁴ | (R⁵)ₘ | X¹ | X² | X³ | bonding site |
|---|---|---|---|---|---|---|---|---|---|
| 76 | H | H | H | CH₂CF₃ | 2-CCH | N | N | CH | 5 |
| 77 | H | H | H | CH₂CF₃ | 2-SCF₃ | N | N | CH | 5 |
| 78 | H | H | H | CH₂CF₃ | 2-S(O)CF₃ | N | N | CH | 5 |
| 79 | H | H | H | CH₂CF₃ | 2-S(O)₂CF₃ | N | N | CH | 5 |
| 80 | H | H | H | CH₂CF₃ | 2-NO₂ | N | N | CH | 5 |
| 81 | H | CH₃ | H | CH₂CF₃ | 2-C(CH₃)₃ | N | N | CH | 5 |
| 82 | H | CH₃ | H | CH₂CF₃ | 2-CH(CH₃)₂ | N | N | CH | 5 |
| 83 | H | CH₃ | H | CH₂CF₃ | 2-CH₂CH₃ | N | N | CH | 5 |
| 84 | H | CH₃ | H | CH₂CF₃ | 2-CH₃ | N | N | CH | 5 |
| 85 | H | CH₃ | H | CH₂CF₃ | 2-CF₃ | N | N | CH | 5 |
| 86 | H | CH₃ | H | CH₂CF₃ | 2-CF₂CF₃ | N | N | CH | 5 |
| 87 | H | CH₃ | H | CH₂CF₃ | 2-CF₂H | N | N | CH | 5 |
| 88 | H | CH₃ | H | CH₂CF₃ | 2-CH₂F | N | N | CH | 5 |
| 89 | H | CH₃ | H | CH₂CF₃ | 2-Cl | N | N | CH | 5 |
| 90 | H | CH₃ | H | CH₂CF₃ | 2-Br | N | N | CH | 5 |
| 91 | H | CH₃ | H | CH₂CF₃ | 2-F | N | N | CH | 5 |
| 92 | H | CH₃ | H | CH₂CF₃ | 2-CN | N | N | CH | 5 |
| 93 | H | CH₃ | H | CH₂CF₃ | 2-CCH | N | N | CH | 5 |
| 94 | H | CH₃ | H | CH₂CF₃ | 2-SCF₃ | N | N | CH | 5 |
| 95 | H | CH₃ | H | CH₂CF₃ | 2-S(O)CF₃ | N | N | CH | 5 |
| 96 | H | CH₃ | H | CH₂CF₃ | 2-S(O)₂CF₃ | N | N | CH | 5 |
| 97 | H | CH₃ | H | CH₂CF₃ | 2-NO₂ | N | N | CH | 5 |
| 98 | H | CH₃ | H | CH₂CF₃ | | N | N | CH | 5 |
| 99 | H | H | H | CH₂CF₃ | 2-C(CH₃)₃ | N | N | CH | 4 |
| 100 | H | H | H | CH₂CF₃ | 2-CH(CH₃)₂ | N | N | CH | 4 |

**Table 5**

| Compound Number | R¹ | R² | R³ | R⁴ | (R⁵)ₘ | X¹ | X² | X³ | bonding site |
|---|---|---|---|---|---|---|---|---|---|
| 101 | H | H | H | CH₂CF₃ | 2-CH₂CH₃ | N | N | CH | 4 |
| 102 | H | H | H | CH₂CF₃ | 2-CH₃ | N | N | CH | 4 |
| 103 | H | H | H | CH₂CF₃ | 2-CF₃ | N | N | CH | 4 |
| 104 | H | H | H | CH₂CF₃ | 2-CF₂CF₃ | N | N | CH | 4 |
| 105 | H | H | H | CH₂CF₃ | 2-CF₂H | N | N | CH | 4 |
| 106 | H | H | H | CH₂CF₃ | 2-CH₂F | N | N | CH | 4 |
| 107 | H | H | H | CH₂CF₃ | 2-Br | N | N | CH | 4 |
| 108 | H | H | H | CH₂CF₃ | 2-F | N | N | CH | 4 |
| 109 | H | H | H | CH₂CF₃ | 2-CN | N | N | CH | 4 |
| 110 | H | H | H | CH₂CF₃ | 2-CCH | N | N | CH | 4 |
| 111 | H | H | H | CH₂CF₃ | 2-SCF₃ | N | N | CH | 4 |
| 112 | H | H | H | CH₂CF₃ | 2-S(O)CF₃ | N | N | CH | 4 |
| 113 | H | H | H | CH₂CF₃ | 2 - S(O)₂CF₃ | N | N | CH | 4 |
| 114 | H | H | H | CH₂CF₃ | 2-NO₂ | N | N | CH | 4 |
| 115 | H | H | H | CH₂CF₃ | | N | N | CH | 4 |
| 116 | H | H | H | CH₂CF₃ | 5-C(CH₃)₃ | N | N | CH | 2 |
| 117 | H | H | H | CH₂CF₃ | 5-CH(CH₃)₂ | N | N | CH | 2 |
| 118 | H | H | H | CH₂CF₃ | 5-CH₂CH₃ | N | N | CH | 2 |
| 119 | H | H | H | CH₂CF₃ | 5-CH₃ | N | N | CH | 2 |
| 120 | H | H | H | CH₂CF₃ | 5-CF₃ | N | N | CH | 2 |
| 121 | H | H | H | CH₂CF₃ | 5-CF₂CF₃ | N | N | CH | 2 |
| 122 | H | H | H | CH₂CF₃ | 5-CF₂H | N | N | CH | 2 |
| 123 | H | H | H | CH₂CF₃ | 5-CH₂F | N | N | CH | 2 |
| 124 | H | H | H | CH₂CF₃ | 5-Cl | N | N | CH | 2 |
| 125 | H | H | H | CH₂CF₃ | 5-Br | N | N | CH | 2 |

**Table 6**

| Compound Number | R¹ | R² | R³ | R⁴ | (R⁵)ₘ | X¹ | X² | X³ | bonding site |
|---|---|---|---|---|---|---|---|---|---|
| 126 | H | H | H | CH₂CF₃ | 5-F | N | N | CH | 2 |
| 127 | H | H | H | CH₂CF₃ | 5-CN | N | N | CH | 2 |
| 128 | H | H | H | CH₂CF₃ | 5-CCH | N | N | CH | 2 |
| 129 | H | H | H | CH₂CF₃ | 5-SCF₃ | N | N | CH | 2 |
| 130 | H | H | H | CH₂CF₃ | 5-S(O)CF₃ | N | N | CH | 2 |
| 131 | H | H | H | CH₂CF₃ | 5-S(O)₂CF₃ | N | N | CH | 2 |
| 132 | H | H | H | CH₂CF₃ | 5-NO₂ | N | N | CH | 2 |
| 133 | H | CH₃ | H | CH₂CF₃ | 5-C(CH₃)₃ | N | N | CH | 2 |
| 134 | H | CH₃ | H | CH₂CF₃ | 5-CH(CH₃)₂ | N | N | CH | 2 |
| 135 | H | CH₃ | H | CH₂CF₃ | 5-CH₂CH₃ | N | N | CH | 2 |
| 136 | H | CH₃ | H | CH₂CF₃ | 5-CH₃ | N | N | CH | 2 |
| 137 | H | CH₃ | H | CH₂CF₃ | 5-CF₃ | N | N | CH | 2 |
| 138 | H | CH₃ | H | CH₂CF₃ | 5-CF₂CF₃ | N | N | CH | 2 |
| 139 | H | CH₃ | H | CH₂CF₃ | 5-CF₂H | N | N | CH | 2 |
| 140 | H | CH₃ | H | CH₂CF₃ | 5-CH₂F | N | N | CH | 2 |
| 141 | H | CH₃ | H | CH₂CF₃ | 5-Cl | N | N | CH | 2 |
| 142 | H | CH₃ | H | CH₂CF₃ | 5-Br | N | N | CH | 2 |
| 143 | H | CH₃ | H | CH₂CF₃ | 5-F | N | N | CH | 2 |
| 144 | H | CH₃ | H | CH₂CF₃ | 5-CN | N | N | CH | 2 |
| 145 | H | CH₃ | H | CH₂CF₃ | 5-CCH | N | N | CH | 2 |
| 146 | H | CH₃ | H | CH₂CF₃ | 5-SCF₃ | N | N | CH | 2 |
| 147 | H | CH₃ | H | CH₂CF₃ | 5-S(O)CF₃ | N | N | CH | 2 |
| 148 | H | CH₃ | H | CH₂CF₃ | 5-S(O)₂CF₃ | N | N | CH | 2 |
| 149 | H | CH₃ | H | CH₂CF₃ | 5-NO₂ | N | N | CH | 2 |
| 150 | H | CH₃ | H | CH₂CF₃ | | N | N | CH | 2 |

**Table 7**

| Compound Number | R¹ | R² | R³ | R⁴ | (R⁵)ₘ | X¹ | X² | X³ | bonding site |
|---|---|---|---|---|---|---|---|---|---|
| 151 | H | H | H | CH₂CF₃ | 4-CH(CH₃)₂ | N | N | CH | 2 |
| 152 | H | H | H | CH₂CF₃ | 4-CH₂CH₃ | N | N | CH | 2 |
| 153 | H | H | H | CH₂CF₃ | 4-CH₃ | N | N | CH | 2 |
| 154 | H | H | H | CH₂CF₃ | 4-CF₃ | N | N | CH | 2 |
| 155 | H | H | H | CH₂CF₃ | 4-CF₂CF₃ | N | N | CH | 2 |
| 156 | H | H | H | CH₂CF₃ | 4-CF₂H | N | N | CH | 2 |
| 157 | H | H | H | CH₂CF₃ | 4-CH₂F | N | N | CH | 2 |
| 158 | H | H | H | CH₂CF₃ | 4-Cl | N | N | CH | 2 |
| 159 | H | H | H | CH₂CF₃ | 4-Br | N | N | CH | 2 |
| 160 | H | H | H | CH₂CF₃ | 4-F | N | N | CH | 2 |
| 161 | H | H | H | CH₂CF₃ | 4-CN | N | N | CH | 2 |
| 162 | H | H | H | CH₂CF₃ | 4-CCH | N | N | CH | 2 |
| 163 | H | H | H | CH₂CF₃ | 4-SCF₃ | N | N | CH | 2 |
| 164 | H | H | H | CH₂CF₃ | 4-S(O)CF₃ | N | N | CH | 2 |
| 165 | H | H | H | CH₂CF₃ | 4-S(O)₂CF₃ | N | N | CH | 2 |
| 166 | H | H | H | CH₂CF₃ | 4-NO₂ | N | N | CH | 2 |
| 167 | H | H | H | CH₂CF₃ | 2-CH(CH₃)₂ | N | CH | N | 5 |
| 168 | H | H | H | CH₂CF₃ | 2-CH₂CH₃ | N | CH | N | 5 |
| 169 | H | H | H | CH₂CF₃ | 2-CH₃ | N | CH | N | 5 |
| 170 | H | H | H | CH₂CF₃ | 2-CF₃ | N | CH | N | 5 |
| 171 | H | H | H | CH₂CF₃ | 2-CF₂CF₃ | N | CH | N | 5 |
| 172 | H | H | H | CH₂CF₃ | 2-CF₂H | N | CH | N | 5 |
| 173 | H | H | H | CH₂CF₃ | 2-CH₂F | N | CH | N | 5 |
| 174 | H | H | H | CH₂CF₃ | 2-Cl | N | CH | N | 5 |
| 175 | H | H | H | CH₂CF₃ | 2-Br | N | CH | N | 5 |

**Table 8**

| Compound Number | R¹ | R² | R³ | R⁴ | (R⁵)ₘ | X¹ | X² | X³ | bonding site |
|---|---|---|---|---|---|---|---|---|---|
| 176 | H | H | H | CH₂CF₃ | 2-F | N | CH | N | 5 |
| 177 | H | H | H | CH₂CF₃ | 2-CN | N | CH | N | 5 |
| 178 | H | H | H | CH₂CF₃ | 2-CCH | N | CH | N | 5 |
| 179 | H | H | H | CH₂CF₃ | 2-SCF₃ | N | CH | N | 5 |
| 180 | H | H | H | CH₂CF₃ | 2-S(O)CF₃ | N | CH | N | 5 |
| 181 | H | H | H | CH₂CF₃ | 2-S(O)₂CF₃ | N | CH | N | 5 |
| 182 | H | H | H | CH₂CF₃ | 2-NO₂ | N | CH | N | 5 |
| 183 | H | CH₃ | H | CH₂CF₃ | 2-C(CH₃)₃ | N | CH | N | 5 |
| 184 | H | CH₃ | H | CH₂CF₃ | 2-CH(CH₃) | N | CH | N | 5 |
| 185 | H | CH₃ | H | CH₂CF₃ | 2-CH₂CH₃ | N | CH | N | 5 |
| 186 | H | CH₃ | H | CH₂CF₃ | 2-CH₃ | N | CH | N | 5 |
| 187 | H | CH₃ | H | CH₂CF₃ | 2-CF₃ | N | CH | N | 5 |
| 188 | H | CH₃ | H | CH₂CF₃ | 2-CF₂CF₃ | N | CH | N | 5 |
| 189 | H | CH₃ | H | CH₂CF₃ | 2-CF₂H | N | CH | N | 5 |
| 190 | H | CH₃ | H | CH₂CF₃ | 2-CH₂F | N | CH | N | 5 |
| 191 | H | CH₃ | H | CH₂CF₃ | 2-Cl | N | CH | N | 5 |
| 192 | H | CH₃ | H | CH₂CF₃ | 2-Br | N | CH | N | 5 |
| 193 | H | CH₃ | H | CH₂CF₃ | 2-F | N | CH | N | 5 |
| 194 | H | CH₃ | H | CH₂CF₃ | 2-CN | N | CH | N | 5 |
| 195 | H | CH₃ | H | CH₂CF₃ | 2-CCH | N | CH | N | 5 |
| 196 | H | CH₃ | H | CH₂CF₃ | 2-SCF₃ | N | CH | N | 5 |
| 197 | H | CH₃ | H | CH₂CF₃ | 2-S(O)CF₃ | N | CH | N | 5 |
| 198 | H | CH₃ | H | CH₂CF₃ | 2-S(O)₂CF₃ | N | CH | N | 5 |
| 199 | H | CH₃ | H | CH₂CF₃ | 2-NO₂ | N | CH | N | 5 |
| 200 | H | CH₃ | H | CH₂CF₃ | | N | CH | N | 5 |

**Table 9**

| Compound Number | R¹ | R² | R³ | R⁴ | (R⁵)ₘ | X¹ | X² | X³ | bonding site |
|---|---|---|---|---|---|---|---|---|---|
| 201 | H | H | H | CH₂CF₃ | 2-C(CH₃)₃ | N | CH | N | 4 |
| 202 | H | H | H | CH₂CF₃ | 2-CH(CH₃)₂ | N | CH | N | 4 |
| 203 | H | H | H | CH₂CF₃ | 2-CH₂CH₃ | N | CH | N | 4 |
| 204 | H | H | H | CH₂CF₃ | 2-CH₃ | N | CH | N | 4 |
| 205 | H | H | H | CH₂CF₃ | 2-CF₃ | N | CH | N | 4 |
| 206 | H | H | H | CH₂CF₃ | 2-CF₂CF₃ | N | CH | N | 4 |
| 207 | H | H | H | CH₂CF₃ | 2-CF₂H | N | CH | N | 4 |
| 208 | H | H | H | CH₂CF₃ | 2-CH₂F | N | CH | N | 4 |
| 209 | H | H | H | CH₂CF₃ | 2-Cl | N | CH | N | 4 |
| 210 | H | H | H | CH₂CF₃ | 2-Br | N | CH | N | 4 |
| 211 | H | H | H | CH₂CF₃ | 2-F | N | CH | N | 4 |
| 212 | H | H | H | CH₂CF₃ | 2-CN | N | CH | N | 4 |
| 213 | H | H | H | CH₂CF₃ | 2-CCH | N | CH | N | 4 |
| 214 | H | H | H | CH₂CF₃ | 2-SCF₃ | N | CH | N | 4 |
| 215 | H | H | H | CH₂CF₃ | 2-S(O)CF₃ | N | CH | N | 4 |
| 216 | H | H | H | CH₂CF₃ | 2-5(O)₂CF₃ | N | CH | N | 4 |
| 217 | H | H | H | CH₂CF₃ | 2-NO₂ | N | CH | N | 4 |
| 218 | H | H | H | CH₂CF₃ | 2-CH(CH₃)₂ | CH | N | N | 5 |
| 219 | H | H | H | CH₂CF₃ | 2-CH₂CH₃ | CH | N | N | 5 |
| 220 | H | H | H | CH₂CF₃ | 2-CH₃ | CH | N | N | 5 |
| 221 | H | H | H | CH₂CF₃ | 2-CF₃ | CH | N | N | 5 |
| 222 | H | H | H | CH₂CF₃ | 2-CF₂CF₃ | CH | N | N | 5 |
| 223 | H | H | H | CH₂CF₃ | 2-CF₂H | CH | N | N | 5 |
| 224 | H | H | H | CH₂CF₃ | 2-CH₂F | CH | N | N | 5 |
| 225 | H | H | H | CH₂CF₃ | 2-Br | CH | N | N | 5 |

**Table 10**

| Compound Number | R¹ | R² | R³ | R⁴ | (R⁵)ₘ | X¹ | X² | X³ | bonding site |
|---|---|---|---|---|---|---|---|---|---|
| 226 | H | H | H | CH₂CF₃ | 2-F | CH | N | N | 5 |
| 227 | H | H | H | CH₂CF₃ | 2-CN | CH | N | N | 5 |
| 228 | H | H | H | CH₂CF₃ | 2-CCH | CH | N | N | 5 |
| 229 | H | H | H | CH₂CF₃ | 2-SCF₃ | CH | N | N | 5 |
| 230 | H | H | H | CH₂CF₃ | 2-S(O)CF₃ | CH | N | N | 5 |
| 231 | H | H | H | CH₂CF₃ | 2-S(O)₂CF₃ | CH | N | N | 5 |
| 232 | H | H | H | CH₂CF₃ | 2-NO₂ | CH | N | N | 5 |
| 233 | H | H | H | CH₂CF₃ | | CH | N | N | 5 |
| 234 | H | CH₃ | H | CH₂CF₃ | 2-C(CH₃)₃ | CH | N | N | 5 |
| 235 | H | CH₃ | H | CH₂CF₃ | 2-CH(CH₃)₂ | CH | N | N | 5 |
| 236 | H | CH₃ | H | CH₂CF₃ | 2-CH₂CH₃ | CH | N | N | 5 |
| 237 | H | CH₃ | H | CH₂CF₃ | 2-CH₃ | CH | N | N | 5 |
| 238 | H | CH₃ | H | CH₂CF₃ | 2-CF₃ | CH | N | N | 5 |
| 239 | H | CH₃ | H | CH₂CF₃ | 2-CF₂CF₃ | CH | N | N | 5 |
| 240 | H | CH₃ | H | CH₂CF₃ | 2-CF₂H | CH | N | N | 5 |
| 241 | H | CH₃ | H | CH₂CF₃ | 2-CH₂F | CH | N | N | 5 |
| 242 | H | CH₃ | H | CH₂CF₃ | 2-Cl | CH | N | N | 5 |
| 243 | H | CH₃ | H | CH₂CF₃ | 2-Br | CH | N | N | 5 |
| 244 | H | CH₃ | H | CH₂CF₃ | 2-F | CH | N | N | 5 |
| 245 | H | CH₃ | H | CH₂CF₃ | 2-CN | CH | N | N | 5 |
| 246 | H | CH₃ | H | CH₂CF₃ | 2-CCH | CH | N | N | 5 |
| 247 | H | CH₃ | H | CH₂CF₃ | 2-SCF₃ | CH | N | N | 5 |
| 248 | H | CH₃ | H | CH₂CF₃ | 2-S(O)CF₃ | CH | N | N | 5 |
| 249 | H | CH₃ | H | CH₂CF₃ | 2-S(O)₂CF₃ | CH | N | N | 5 |
| 250 | H | CH₃ | H | CH₂CF₃ | 2-NO₂ | CH | N | N | 5 |

**Table 11**

| Compound Number | R¹ | R² | R³ | R⁴ | (R⁵)ₘ | X¹ | X² | X³ | bonding site |
|---|---|---|---|---|---|---|---|---|---|
| 251 | H | CH₃ | H | CH₂CF₃ | | CH | N | N | 5 |
| 252 | H | H | H | CH₂CF₃ | 2-C(CH₃)₃ | CH | N | N | 4 |
| 253 | H | H | H | CH₂CF₃ | 2-CH(CH₃)₂ | CH | N | N | 4 |
| 254 | H | H | H | CH₂CF₃ | 2-CH₂CH₃ | CH | N | N | 4 |
| 255 | H | H | H | CH₂CF₃ | 2-CH₃ | CH | N | N | 4 |
| 256 | H | H | H | CH₂CF₃ | 2-CF₃ | CH | N | N | 4 |
| 257 | H | H | H | CH₂CF₃ | 2-CF₂CF₃ | CH | N | N | 4 |
| 258 | H | H | H | CH₂CF₃ | 2-CF₂H | CH | N | N | 4 |
| 259 | H | H | H | CH₂CF₃ | 2-CH₂F | CH | N | N | 4 |
| 260 | H | H | H | CH₂CF₃ | 2-Cl | CH | N | N | 4 |
| 261 | H | H | H | CH₂CF₃ | 2-Br | CH | N | N | 4 |
| 262 | H | H | H | CH₂CF₃ | 2-F | CH | N | N | 4 |
| 263 | H | H | H | CH₂CF₃ | 2-CN | CH | N | N | 4 |
| 264 | H | H | H | CH₂CF₃ | 2-CCH | CH | N | N | 4 |
| 265 | H | H | H | CH₂CF₃ | 2-SCF₃ | CH | N | N | 4 |
| 266 | H | H | H | CH₂CF₃ | 2-S(O)CF₃ | CH | N | N | 4 |
| 267 | H | H | H | CH₂CF₃ | 2-S(O)₂CF₃ | CH | N | N | 4 |
| 268 | H | H | H | CH₂CF₃ | 2-NO₂ | CH | N | N | 4 |
| 269 | H | H | H | CH₂CF₃ | | CH | N | N | 4 |
| 270 | H | H | H | CH₂CF₃ | 4-C(CH₃)₃ | CH | N | N | 2 |
| 271 | H | H | H | CH₂CF₃ | 4-CH(CH₃)₂ | CH | N | N | 2 |
| 272 | H | H | H | CH₂CF₃ | 4-CH₂CH₃ | CH | N | N | 2 |
| 273 | H | H | H | CH₂CF₃ | 4-CH₃ | CH | N | N | 2 |
| 274 | H | H | H | CH₂CF₃ | 4-CF₃ | CH | N | N | 2 |
| 275 | H | H | H | CH₂CF₃ | 4-CF₂CF₃ | CH | N | N | 2 |

**Table 12**

| Compound Number | R¹ | R² | R³ | R⁴ | (R⁵)ₘ | X¹ | X² | X³ | bonding site |
|---|---|---|---|---|---|---|---|---|---|
| 276 | H | H | H | CH₂CF₃ | 4-CF₂H | CH | N | N | 2 |
| 277 | H | H | H | CH₂CF₃ | 4-CH₂F | CH | N | N | 2 |
| 278 | H | H | H | CH₂CF₃ | 4-Cl | CH | N | N | 2 |
| 279 | H | H | H | CH₂CF₃ | 4-Br | CH | N | N | 2 |
| 280 | H | H | H | CH₂CF₃ | 4-F | CH | N | N | 2 |
| 281 | H | H | H | CH₂CF₃ | 4-CN | CH | N | N | 2 |
| 282 | H | H | H | CH₂CF₃ | 4-CCH | CH | N | N | 2 |
| 283 | H | H | H | CH₂CF₃ | 4-SCF3 | CH | N | N | 2 |
| 284 | H | H | H | CH₂CF₃ | 4-S(O)CF₃ | CH | N | N | 2 |
| 285 | H | H | H | CH₂CF₃ | 4-S(O)₂CF₃ | CH | N | N | 2 |
| 286 | H | H | H | CH₂CF₃ | 4-NO₂ | CH | N | N | 2 |
| 287 | H | H | H | CH₂CF₃ | | CH | N | N | 2 |
| 288 | H | H | H | CH₂CF₃ | 2-C(CH₃)₃ | N | N | N | 5 |
| 289 | H | H | H | CH₂CF₃ | 2-CH(CH₃)₂ | N | N | N | 5 |
| 290 | H | H | H | CH₂CF₃ | 2-CH₂CH₃ | N | N | N | 5 |
| 291 | H | H | H | CH₂CF₃ | 2-CH₃ | N | N | N | 5 |
| 292 | H | H | H | CH₂CF₃ | 2-CF₃ | N | N | N | 5 |
| 293 | H | H | H | CH₂CF₃ | 2-CF₂CF₃ | N | N | N | 5 |
| 294 | H | H | H | CH₂CF₃ | 2-CF₂H | N | N | N | 5 |
| 295 | H | H | H | CH₂CF₃ | 2-CH₂F | N | N | N | 5 |
| 296 | H | H | H | CH₂CF₃ | 2-Cl | N | N | N | 5 |
| 297 | H | H | H | CH₂CF₃ | 2-Br | N | N | N | 5 |
| 298 | H | H | H | CH₂CF₃ | 2-F | N | N | N | 5 |
| 299 | H | H | H | CH₂CF₃ | 2-CN | N | N | N | 5 |
| 300 | H | H | H | CH₂CF₃ | 2-CCH | N | N | N | 5 |

**Table 13**

| Compound Number | R¹ | R² | R³ | R⁴ | (R⁵)ₘ | X¹ | X² | X³ | bonding site |
|---|---|---|---|---|---|---|---|---|---|
| 301 | H | H | H | CH₂CF₃ | 2-SCF₃ | N | N | N | 5 |
| 302 | H | H | H | CH₂CF₃ | 2-S(O)CF₃ | N | N | N | 5 |
| 303 | H | H | H | CH₂CF₃ | 2-S(O)₂CF₃ | N | N | N | 5 |
| 304 | H | H | H | CH₂CF₃ | 2-NO₂ | N | N | N | 5 |
| 305 | H | H | H | CH₂CF₃ | | N | N | N | 5 |
| 306 | H | H | H | CH₂CF₃ | 5-C(CH₃)₃ | N | N | N | 2 |
| 307 | H | H | H | CH₂CF₃ | 5-CH(CH₃)₂ | N | N | N | 2 |
| 308 | H | H | H | CH₂CF₃ | 5-CH₂CH₃ | N | N | N | 2 |
| 309 | H | H | H | CH₂CF₃ | 5-CH₃ | N | N | N | 2 |
| 310 | H | H | H | CH₂CF₃ | 5-CF₃ | N | N | N | 2 |
| 311 | H | H | H | CH₂CF₃ | 5-CF₂CF₃ | N | N | N | 2 |
| 312 | H | H | H | CH₂CF₃ | 5-CF₂H | N | N | N | 2 |
| 313 | H | H | H | CH₂CF₃ | 5-CH₂F | N | N | N | 2 |
| 314 | H | H | H | CH₂CF₃ | 5-Cl | N | N | N | 2 |
| 315 | H | H | H | CH₂CF₃ | 5-Br | N | N | N | 2 |
| 316 | H | H | H | CH₂CF₃ | 5-F | N | N | N | 2 |
| 317 | H | H | H | CH₂CF₃ | 5-CN | N | N | N | 2 |
| 318 | H | H | H | CH₂CF₃ | 5-CCH | N | N | N | 2 |
| 319 | H | H | H | CH₂CF₃ | 5-SCF₃ | N | N | N | 2 |
| 320 | H | H | H | CH₂CF₃ | 5-S(O)CF₃ | N | N | N | 2 |
| 321 | H | H | H | CH₂CF₃ | 5-S(O)₂CF₃ | N | N | N | 2 |
| 322 | H | H | H | CH₂CF₃ | 5-NO₂ | N | N | N | 2 |
| 323 | H | H | H | CH₂CF₃ | | N | N | N | 2 |

The term "bonding site" as above described in (Table 1) to (Table 13) means the position of the carbon atom on the ring at which the group of the formula 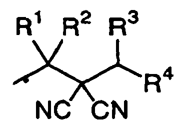 is coupled.

In addition, a blank in (R)ₘ represents m=0 , and each of the remarks of "4,5-(CH₂)₃-" and "4,5-(CH₃)₂(CH₂)₂-" designates a group bound at the position (left end) of the carbon atom represented by ┌4┘ and at the position (right end) of the carbon atom represented by ┌5┘ in the formula (I).

Secondary, production examples of the intermediate compound are illustrated as Referential Production Examples.

### Referential Production Example 1

### 2-t-butyl-5-pyrimidinecarboxylic acid methyl ester (represented by the following formula)

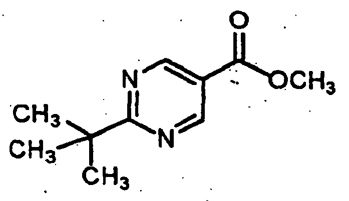

The above compound can be produced by the method described in Synthesis, 2002, p.720-722.

### Referential Production Example 2

### (2-t-butylpyrimidine-5-yl)methanol (represented by the following formula)

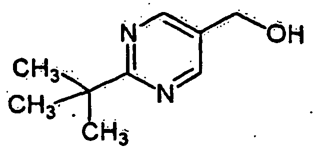

0.27 g of 2-t-butyl-5-pyrimidinecarboxylic acid methyl ester was dissolved in 10 ml of tetrahydrofuran under a nitrogen atmosphere and then dropped with 3.0 ml of toluene solution of diisobutylaluminum hydride (1 mole/L) under ice cooling, followed by stirring as itself for one hour. The reaction mixture was poured into saturated brine, followed by extraction with ethylacetate. The organic phase was dried with an anhydrous sodium sulf ate, followed by concentration under reduced pressure. The residue was subjected to a silica gel column chromatography to obtain 0.09 g of (2-t-butylpyrimidine-5-yl)methanol represented by the above formula.
¹H-NMR (CDCl₃, TMS), δ (ppm) : 1.45 (9H, s), 4.72 (2H, brs), 8.69 (2H, s)

### Referential Production Example 3

### 2-t-butyl-5-chloromethylpyrimidine (represented by the following formula)

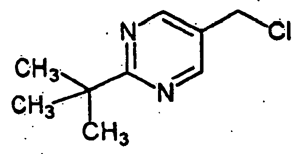

0.25 g of (2-t-butylpyrimidine-5-yl)methanol was dissolved with 5 ml of chloroform and then added with 0.2 g of thionylchloride, followed by heating under reflux for one hour. The reaction mixture was cooled down to room temperature, followed by concentration under reduced pressure. The residue was added with water and saturated aqueous solution of sodium hydrogen carbonate, followed by extraction with ethylacetate. The organic phase was dried with an anhydrous sodium sulfate, followed by concentration under reduced pressure to obtain 0.21 g of 2-t-butyl-5-chloromethylpyrimidine.

### Referential Production Example 4

### 2-chloro-5-methylpyrimidine (represented by the following formula)

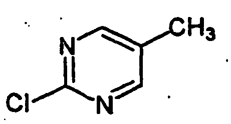

The above compound can be produced by the method described in Aust. J. Chem., Vol. 26, p.443-447.

### Referential Production Example 5

### 5-bromomethyl-2-chloropyrimidine (represented by the following formula)

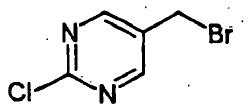

0.40 g of 2-chloro-5-methylpyrimidine was dissolved in 15 ml of carbontetrachloride and then added with 0.83 g of N-bromosuccinimide and 0.05 g of azobisisobutyronitrile, followed by heating under reflux for 8 hours. The reaction mixture was cooled down to room temperature and then filtrated, followed by the filtrate being concentrated under reduced pressure. The residue was subjected to a silica gel column chromatography to obtain 0.27 g of 5-bromomethyl-2-chloropyrimidine represented by above formula.
¹H-NMR (CDCl₃, TMS) , δ (ppm) : 4.43 (2H, s), 8.67 (2H, s)

### Referential Production Example 6

### 3-chloro-6-methylpyridazine (represented by the following formula)

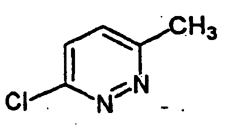

5.0 g of 6-methyl-2H-pyridazine-3-one and 28 g of phosphorus oxychloride were mixed, followed by heating under reflux for 2 hours. The reaction mixture was cooled down to room temperature and then poured into ice water, followed by extraction with ethylacetate. The organic phase was dried with an anhydrous sodium sulfate, followed by concentration under reduced pressure to obtain 1.6 g of 3-chloro-6-methylpyridazine.

### Referential Production Example 7

### 3-bromomethyl-6-chloropyridazine (represented by the following formula)

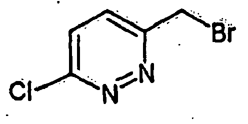

1.0 g of 3-chloro-6-methylpyridazine was dissolved in 20 ml of carbontetrachloride and then added with 1.4 g of N-bromosuccinimide and 0.13 g of azobisisobutyronitrile, followed by heating under ref lux for 8 hours. The reaction mixture was cooled down to room temperature and then filtrated, followed by the filtrate being concentrated under reduced pressure. The residue was subjected to a silica gel column chromatography to obtain 0.27 g of 3-bromomethyl-6-chloropyridazine represented by above formula.
¹H-NMR (CDCl₃, TMS) , δ (ppm) : 4.56 (2H, s), 7.38 (1H, d) , 7.44 (1H, d)

Formulation Examples are exemplified below. In addition, "part" means part by weight. The compounds according to the present invention are designated by their compound numbers shown in Tables 1 to 13.

### Formulation Example 1

9 Parts of each of the compounds according to the present invention (1) to (28) are dissolved in 37.5 parts of xylene and 37.5 parts of dimethylformamide, and 10 parts of polyoxyethylene styryl phenyl ether and 6 parts of calcium dodecylbenzenesulfonate are added thereto, followed by stirring and mixing well, to give an emulsifiable concentrate for each compound.

### Formulation Example 2

To 40 parts of each of the compounds according to the present invention (1) to (28) are added 5 parts of SORPOL 5060 (registered trade name for TOHO KAGAKU KOGYO), followed by mixing well. To the mixture are added 32 parts of CARPLEX #80 (registered trade name for SHIONOGI & Co., synthetic hydrated silicon oxide fine powder) and 23 parts of 300 mesh diatomaceous earth, followed by mixing with a juice mixer, to give a wettable powder for each compound.

### Formulation Example 3

To 3 parts of each of the compounds according to the present invention (1) to (28) are added 5 parts of synthetic hydrated silicon oxide fine powder, 5 parts of sodium dodecylbenzenesulfonate, 30 parts of bentonite, and 57 parts of clay, followed by stirring and mixing well. Then an appropriate amount of water is added to this mixture, followed by further stirring, granulating with a granulator, and air drying, to give a granule for each compound.

### Formulation Example 4

4.5 Parts of each of the present invention compounds (1) to (28), 1 part of synthetic hydrated silicon oxide fine powder, 1 part of Doriresu B (Sankyo Co., Ltd.) as a flocculant and 7 parts of clay are well mixed with a mortar, followed by stirring and mixing with a juice mixer. To the resulting mixture are added 86.5 parts of cut clay, followed by stirring and mixing well, to give a dust for each compound.

### Formulation Example 5

10 Parts of each of the present invention compounds (1) to (28), 35 parts of white carbon containing 50 parts of polyoxyethylene alkyl ether sulfate ammonium salt and 55 parts of water are mixed and pulverized by the wet grinding method to give a formulation for each compound.

### Formulation Example 6

0.5 Parts of each of the present invention compounds (1) to (28) are dissolved in 10 parts of dichloromethane, and the resulting solution is mixed with 89.5 parts of Iso-Par M (isoparaffine: registered trade name for EXXON CHEMICAL LTD) to give an oil solution.

### Formulation Example 7

0.1 Parts of each of the present invention compounds (1) to (28) and 49.9 parts of NEO-CHIOZOL (CHUO KASEI Co. , LTD) are charged into aerosol can, and aerosol valve is fixed to the can. Then 25 parts of dimethyl ether and 25 parts of LPG are filled in the can, followed by shaking and fitting an actuator on it, to give an oil aerosol.

### Formulation Example 8

0.6 Parts of each of the present invention compounds (1) to (28), 0.01 parts of BHT, 5 parts of xylene, 3.39 parts of deodorized kerosene and 1 part of emulsifier [Atmos 300(registered trade name for ATMOS CHEMICAL LTD)] are mixed and dissolved. The solution obtained and 50 parts of distilled water are charged into aerosol container, and a valve is fixed to the container. 40 Parts of propellant (LPG) are charged under pressure through the valve to give an aqueous aerosol.

The following test examples will demonstrate that the present invention compounds have a pesticidal activity as active ingredient of a composition for controlling pests. The present invention compounds are designated by their compound numbers shown in Tables.

### Test Example 1

The formulation obtained according to Formulation Example 5 using the compounds according to the present invention (1), (2), (3), (7), (8), (9), (10), (11), (13), (14), (15), (16), (17), (18), (19), (24), (26) and (28) respectively, was diluted with water so that the active ingredient concentration came to 500 ppm to prepare a pesticidal solution for test.

Fifty grams of molding Bonsoru 2 (available from Sumitomo Chemical Co., Ltd.) was put into a polyethylene cup, and 10 to 15 seeds of rice were planted in the polyethylene cup. Then rice plants were grown until the second foliage leaves developed and then cut into the same height of 5 cm. The pesticidal solution for test prepared above was sprayed at the rate of 20 ml/cup to these rice plants. After the pesticidal solution sprayed onto the rice plants was dried, they were put into a plastic cup for escape prevention of test pests, and thirty first-instar larvae of *Nilaparvata lugens* were set free on the rice plants, followed by covering the plastic cup with a lid. Then the plastic cup was left in a greenhouse (25ºC). On the sixth day after the release of larvae of *Nilaparvata lugens,* the number of parasitic *Nilaparvata lugens* on the rice plants was examined. As a result, in the treatment with each of the compounds according to the present invention (1), (2), (3), (7), (8), (9), (10), (11), (13), (14), (15), (16), (17), (18), (19), (24), (26) and (28), the number of parasitic *Nilaparvata lugens* was not greater than 3.

### Test Example 2

The formulation obtained according to Formulation Example 5 using the present invention compounds (1), (7), (8), (9), (10), (14), (16), (18) and (26) respectively, was diluted with water so that the active ingredient concentration came to 500 ppm to prepare a pesticidal solution for test.

A polyethylene cup was seeded with cucumber and a plant was grown until the first true leaf was developed, on which about twenty *Aphis gossypii* are allowed to be parasitic. On the next day, the above pesticidal solution for test was sprayed at a ratio of 20 ml/cup to the cucumber plant. On the sixth day after the application, the number of *Aphis gossypii* was examined.

As a result, in the treatment with each of the present invention compounds (1), (7), (8), (9), (10), (14), (16), (18) and (26), the number of parasitic *Nilaparvata lugens* on the sixth day after the treatment was not greater than 3.

### Test Example 3

The formulation obtained according to Formulation Example 5 using the compounds according to the present invention (1), (2), (3), (7), (8), (9), (10), (11), (13), (14), (16), (17), (18) and (19) respectively, was diluted with water so that the active ingredient concentration came to 500 ppm to prepare a pesticidal solution for test.

On the bottom of a polyethylene cup having a diameter of 5.5 cm, a filter paper having the same diameter was laid, and 0.7 ml of the above pesticidal solution for test was added dropwise on the filter paper, followed by putting 30 mg of sucrose on it uniformly as a bait. Ten female *Musca domestica* imagoes were set free in the polyethylene cup and covered it with a lid. After 24 hours, the number of surviving *Musca domestica* was examined and the rate of dead pests was calculated.
As a result, in the treatment with each of the compounds according to the present invention (1), (2), (3), (7), (8), (9), (10), (11), (13), (14), (16), (17), (18) and (19) the rate of dead pests was 90% or more.

### Test Example 4

The formulation obtained according to Formulation Example 5 using the compounds according to the present invention (1), (6), (9), (10), (12), (13), (14), (16), (17), (18), (19) and (20) respectively, was diluted with water so that the active ingredient concentration came to 500 ppm to prepare a pesticidal solution for test.

On the bottom of a polyethylene cup having a diameter of 5.5 cm, a filter paper having the same diameter was laid, and 0. 7 ml of the above pesticidal solution for test was added dropwise on the filter paper, followed by putting 30 mg of sucrose on it uniformly as a bait. Two male *Blattella germanica* imagoes were set free in the polyethylene cup and covered it with a lid. After 6 days, the number of surviving *Blattella germanica* was examined and the rate of dead pests was calculated. As a result, in the treatment with each of the compounds according to the present invention (1), (6), (9), (10), (12), (13), (14), (16), (17), (18), (19) and (20), the rate of dead pests was 100%.

### Test Example 5

The formulation obtained according to Formulation Example 5 using the compounds according to the present invention (1), (2), (3), (4), (6), (7), (8), (9), (10), (11), (13), (14), (16), (17), (18), (19), (21), (22), (23), (25) and (26) respectively, was diluted with water so that the active ingredient concentration came to 500 ppm to prepare a pesticidal solution for test.

0.7 ml of above pesticidal solution for test was added to 100 ml of ion exchanged water (active ingredient concentration: 3.5 ppm). Twenty last-instar larvae of *Culex pipiens pallens* were set free in the solution. After one day, the number of surviving *Culex pipiens pallens* was examined and the rate of dead pests was calculated.

As a result, in the treatment with each of the compounds according to the present invention (1), (2), (3), (4), (6), (7), (8), (9), (10), (11), (13), (14), (16), (17), (18), (19), (21), (22), (23), (25) and (26), the rate of dead pests was 100%.

Pests can be controlled by using the pesticide composition containing the compound of the present invention as an active ingredient.

## Claims

1. A malononitrile compound represented by the formula (I): 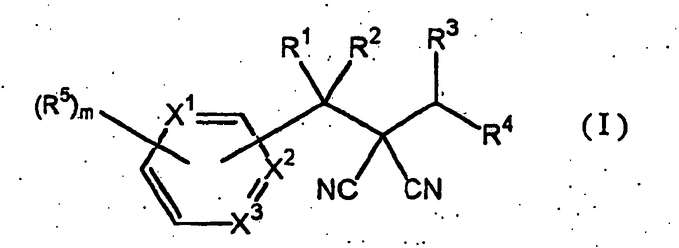 wherein, in the formula,
R¹ represents a C1-C5 alkyl group optionally substituted by one or more halogen atoms, a C2-C5 alkenyl group optionally substituted by one or more halogen atoms, a C2-C5 alkynyl group optionally substituted by one or more halogen atoms or a hydrogen atom;
R² represents a C1-C5 alkyl group optionally substituted by one or more halogen atoms, a C1-C5 alkoxy group optionally substituted by one or more halogen atoms, a C2-C5 alkenyl group optionally substituted by one or more halogen atoms, a C2-C5 alkynyl group optionally substituted by one or more halogen atoms, a cyano group or a hydrogen atom;
each of R³ and R⁴ represents a C1-C5 alkyl group optionally substituted by one or more halogen atoms, a C2-C5 alkenyl group optionally substituted by one or more halogen atoms, a C2-C5 alkynyl group optionally substituted by one or more halogen atoms, a C3-C5 cycloalkyl group optionally substituted by one or more halogen atoms, a C5-C6 cycloalkenyl group optionally substituted by one or more halogen atoms or a hydrogen atom,
or represents a C2-C6 alkanediyl group optionally substituted by one or more halogen atoms or a C4-C6 alkenediyl group optionally substituted by one or more halogen atoms in each of which R³ and R⁴ couple one another at the ends thereof;
each of X¹, X² and X³ represents a nitrogen atom or CR⁶, with the proviso that two or three of X¹, X² and X³ represent a nitrogen atom;
R⁶ represents a hydrogen atom or R⁵ ;
each of R⁵s represents a halogen atom, a cyano group, a nitro group, a hydroxyl group, a mercapto group, a formyl group, a SF₅ group, a carboxyl group, a C1-C5 alkyl group optionally substituted by one or more halogen atoms, a C2-C5 alkenyl group optionally substituted by one or more halogen atoms, a C2-C5 alkynyl group optionally substituted by one or more halogen atoms, a C3-C5 cycloalkyl group optionally substituted by one or more halogen atoms, a C1-C5 alkoxy group optionally substituted by one or more halogen atoms, a C3-C6 alkenyloxy group optionally substituted by one or more halogen atoms, a C3-C6 alkynyloxy group optionally substituted by one or more halogen atoms, a C1-C5 alkylthio group optionally substituted by one or more halogen atoms, a C3-C5 alkenylthio group optionally substituted by one or more halogen atoms, a C3-C5 alkynylthio group optionally substituted by one or more halogen atoms, a C1-C5 alkylsulfinyl group optionally substituted by one or more halogen atoms, a C1-C5 alkylsulfonyl group optionally substituted by one or more halogen atoms, a C2-C6 alkylcarbonyl group optionally substituted by one or more halogen atoms, a C2-C5 alkoxycarbonyl group optionally substituted by one or more halogen atoms, a group designated by -NR¹⁰R¹¹, a group designated by -C(=X⁵) NR¹²R¹³ or a group designated by - C(=NOR¹⁴)R¹⁵, or
where m is 2 and two R⁵s are coupled to two adjacent carbon atoms may represent a C3-C6 alkanediyl group or a C3-C6 alkenediyl group each of which is formed by coupling of the two R⁵s at the ends thereof, furthermore, in this condition, a methylene group contained in said C3-C6 alkanediyl group or said C3-C6 alkenediyl group may be replaced by O, S(O)ᵣ or NR¹⁶ and a carbon atom contained in said C3-C6 alkanediyl group or said C3-C6 alkenediyl group may be coupled with a halogen atom or a C1-C3 alkoxy group optionally substituted by one or more halogen atoms instead of hydrogen atom(s);
each of R¹⁰ and R¹¹ represents a C1-C5 alkyl group optionally substituted by one or more halogen atoms, a C3-C5 alkenyl group optionally substituted by one or more halogen atoms, a C3-C5 alkynyl group optionally subst ituted by one or more halogen atoms, a C3-C5 cycloalkyl group optionally substituted by one or more halogen atoms, a (C1 -C5 alkoxy optionally substituted by one or more halogen atoms) C1-C3 alkyl group, a C1-C5 alkylsulfonyl group optionally substituted by one or more halogen atoms, a C2-C6 alkylcarbonyl group optionally substituted by one or more halogen atoms, a C2-C5 alkoxycarbonyl group optionally substituted by one or more halogen atoms or a hydrogen atom, or represents a C2-C6 alkanediyl group optionally substituted by one or more halogen atoms or aC4-C6 alkenediyl group optionally substituted by one or more halogen atoms in each of which R¹⁰ and R¹¹ are coupled with one another at the ends thereof;
each of R¹² and R¹³ represents a C1-C5 alkyl group optionally substituted by one or more halogen atoms, a C3-C5 alkenyl group optionally substituted by one or more halogen atoms, a C3-C5 alkynyl group optionally substituted by one or more halogen atoms, a C3-C5 cycloalkyl group optionally substituted by one or more halogen atoms, a group designated by -(CH₂)ₙQ² or a hydrogen atom,
or represents a C2-C6 alkanediyl group optionally substituted by one or more halogen atoms or a C4-C6 alkenediyl group optionally substituted by one or more halogen atoms in each of which R¹² and R¹³ are coupled with one another at the ends thereof; each of R¹⁴ and R¹⁵ represents a C1-C5 alkyl group optionally substituted by one or more halogen atoms, a C3-C5 alkenyl group optionally substituted by one or more halogen atoms, a C3-C5 alkynyl group optionally substituted by one or more halogen atoms, a C3-C5 cycloalkyl group optionally substituted by one or more halogen atoms, a group designated by -(CH₂)ₙQ² or a hydrogen atom;
each of Q¹ and Q² represents a phenyl group which may be substituted by a group selected from the group consisting of a C1-C5 alkyl group optionally substituted by one or more halogen atoms, a C1-C5 alkoxy group optionally substituted by one or more halogen atoms, a C1-C5 alkylthio group optionally substituted by one or more halogen atoms and a halogen atom;
R¹⁶ represents a C1-C3 alkyl group; m represents an integer of from 0 to 2; n represents an integer of from 0 to 3; r represents
an integer of from 0 to 2; and
X⁵ represents an oxygen atom or a sulfur atom.

2. The malononitrile compound according to claim 1, wherein R³ is a hydrogen atom and R⁴ is a C1-C5 alkyl group optionally substituted by one or more halogen atoms, a C2-C5 alkenyl group optionally substituted by one or more halogen atoms, a C2-C5 alkynyl group optionally substituted by one or more halogen atoms , a C3-C5 cycloalkyl group optionally substituted by one or more halogen atoms or a C5-C6 cycloalkenyl group optionally substituted by one or more halogen atoms.

3. The malononitrile compound according to claim 1, which is represented by the formula (XI): 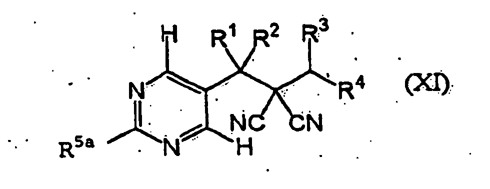 wherein R¹ represents a C1-C5 alkyl group optionally substituted by one or more halogen atoms, a C2-C5 alkenyl group optionally substituted by one or more halogen atoms, a C2-C5 alkynyl group optionally substituted by one or more halogen atoms or a hydrogen atom;
R² represents a C1-C5 alkyl group optionally substituted by one or more halogen atoms, a C1-C5 alkoxy group optionally substituted by one or more halogen atoms, a C2-C5 alkenyl group optionally substituted by one or more halogen atoms, a C2-C5 alkynyl group optionally substituted by one or more halogen atoms, a cyano group or a hydrogen atom;
each of R³ and R⁴ represents a C1-C5 alkyl group optionally substituted by one or more halogen atoms, a C2-C5 alkenyl group optionally substituted by one or more halogen atoms, a C2-C5 alkynyl group optionally substituted by one or more halogen atoms, a C3-C5 cycloalkyl group optionally substituted by one or more halogen atoms, a C5-C6 cycloalkenyl group optionally substituted by one or more halogen atoms or a hydrogen atom,
or represents a C2-C6 alkanediyl group optionally substituted by one or more halogen atoms or a C4-C6 alkenediyl group optionally substituted by one or more halogen atoms in each of which R³ and R⁴ couple one another at the ends thereof;
R^{5a} represents a hydrogen atom, halogen atom, a cyano group, a nitro group, a hydroxyl group, a mercapto group, a formyl group, a SF₅ group, a carboxyl group, a C1-C5 alkyl group optionally substituted by one or more halogen atoms, a C2-C5 alkenyl group optionally substituted by one or more halogen atoms, a C2-C5 alkynyl group optionally substituted by one or more halogen atoms, a C3-C5 cycloalkyl group optionally substituted by one or more halogen atoms, a C1-C5 alkoxy group optionally substituted by one or more halogen atoms, a C3-C6 alkenyloxy group optionally substituted by one or more halogen atoms, a C3-C6 alkynyloxy group optionally substituted by one or more halogen atoms, a C1-C5 alkylthio group optionally substituted by one or more halogen atoms, a C3-C5 alkenylthio group optionally substituted by one or more halogen atoms, a C3 -C5 alkynylthio group optionally substituted by one or more halogen atoms, a C1-C5 alkylsulfinyl group optionally substituted by one or more halogen atoms, a C1-C5 alkylsulfonyl group optionally substituted by one or more halogen atoms, a C2-C6 alkylcarbonyl group optionally substituted by one or more halogen atoms, a C2-C5 alkoxycarbonyl group optionally substituted by one or more halogen atoms, a group designated by -NR¹⁰R¹¹, a group designated by -C( =X⁵) NR¹²R¹³ or a group designated by -C(=NOR¹⁴)R¹⁵,
each of R¹⁰ and R¹¹ represents a C1-C5 alkyl group optionally substituted by one or more halogen atoms, a C3-C5 alkenyl group optionally substituted by one or more halogen atoms, a C3-C5 alkynyl group optionally substituted by one or more halogen atoms, a C3-C5 cycloalkyl group optionally substituted by one or more halogen atoms, a (C1-C5 alkoxy optionally substituted by one or more halogen atoms) C1-C3 alkyl group, a C1-C5 alkylsulfonyl group optionally substituted by one or more halogen atoms, a C2-C6 alkylcarbonyl group optionally substituted by one or more halogen atoms, a C2-C5 alkoxycarbonyl group optionally substituted by one or more halogen atoms or a hydrogen atom,
or represents a C2-C6 alkanediyl group optionally substituted by one or more halogen atoms or a C4-C6 alkenediyl group optionally substituted by one or more halogen atoms in each of which R¹⁰ and R¹¹ are coupled with one another at the ends thereof;
each of R¹² and R¹³ represents a C1-C5 alkyl group optionally substituted by one or more halogen atoms, a C3-C5 alkenyl group optionally substituted by one or more halogen atoms, a C3-C5 alkynyl group optionally substituted by one or more halogen atoms, a C3-C5 cycloalkyl group optionally substituted by one or more halogen atoms, a group designated by -(CH₂)ₙQ¹ or a hydrogen atom,
or represents a C2-C6 alkanediyl group optionally substituted by one or more halogen atoms or a C4-C6 alkenediyl group optionally substituted by one or more halogen atoms in each of which R¹² and R¹³ are coupled with one another at the ends thereof;
each of R¹⁴ and R¹⁵ represents a C1-C5 alkyl group optionally substituted by one or more halogen atoms, a C3-C5 alkenyl group optionally substituted by one or more halogen atom, a C3-C5 alkynyl group optionally substituted by one or more halogen atoms, a C3-C5 cycloalkyl group optionally substituted by one or more halogen atoms, a group designated by - (CH₂)ₙQ² or a hydrogen atom; each of Q¹ and Q² represents a phenyl group which may be substituted by a group selected from the group consisting of a C1-C5 alkyl group optionally substituted by one or more halogen atoms, a C1-C5 alkoxy group optionally substituted by one or more halogen atoms, a C1-C5 alkylthio group optionally substituted by one or more halogen atoms and a halogen atom; and X⁵ represents an oxygen atom or a sulfur atom.

4. The malononitrile compound according to Claim 1, which is represented by the formula (XII): 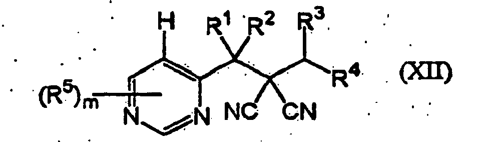 wherein R¹ represents a C1-C5 alkyl group optionally substituted by one or more halogen atoms, a C2-C5 alkenyl group optionally substituted by one or more halogen atoms, a C2-C5 alkynyl group optionally substituted by one or more halogen atoms or a hydrogen atom;
R² represents a C1-C5 alkyl group optionally substituted by one or more halogen atoms, a C1-C5 alkoxy group optionally substituted by one or more halogen atoms, a C2-C5 alkenyl group optionally substituted by one or more halogen atoms, a C2-C5 alkynyl group optionally substituted by one or more halogen atoms, a cyano group or a hydrogen atom;
each of R³ and R⁴ represents a C1-C5 alkyl group optionally substituted by one or more halogen atoms, a C2-C5 alkenyl group optionally substituted by one or more halogen atoms; a C2-C5 alkynyl group optionally substituted by one or more halogen atoms, a C3-C5 cycloalkyl group optionally substituted by one or more halogen atoms, a C5-C6 cycloalkenyl group optionally substituted by one or more halogen atoms or a hydrogen atom,
or represents a C2-C6 alkanediyl group optionally substituted by one or more halogen atoms or a C4-C6 alkenediyl group optionally substituted by one or more halogen atoms in each of which R³ and R⁴ couple one another at the ends thereof;
R⁵ represents a halogen atom, a cyano group, a nitro group, a hydroxyl group, a mercapto group, a formyl group, a SF₅ group, a carboxyl group, a C1-C5 alkyl group optionally substituted by one or more halogen atoms, a C2-C5 alkenyl group optionally substituted by one or more halogen atoms, a C2-C5 alkynyl group optionally substituted by one or more halogen atoms, a C3-C5 cycloalkyl group optionally substituted by one or more halogen atoms, a C1-C5 alkoxy group optionally substituted by one or more halogen atoms, a C3-C6 alkenyloxy group optionally substituted by one or more halogen atoms, a C3-C6 alkynyloxy group optionally substituted by one or more halogen atoms, a C1-C5 alkylthio group optionally substituted by one or more halogen atoms, a C3 -C5 alkenylthio group optionally substituted by one or more halogen atoms, aC3-C5 alkynylthio group optionally substituted by one or more halogen atoms, a C1-C5 alkylsulfinyl group optionally substituted by one or more halogen atoms, a C1-C5 alkylsulfonyl group optionally substituted by one or more halogen atoms, a C2-C6 alkylcarbonyl group optionally substituted by one or more halogen atoms, a C2-C5 alkoxycarbonyl group optionally substituted by one or more halogen atoms, a group designated by -NR¹⁰R¹¹, a group designated by -C(=X⁵) NR¹²R¹³ or a group designated by -C(=NOR¹⁴)R¹⁵,
each of R¹⁰ and R¹¹ represents a C1-C5 alkyl group optionally substituted by one or more halogen atoms, a C3-C5 alkenyl group optionally substituted by one or more halogen atoms, a C3-C5 alkynyl group optionally substituted by one or more halogen atoms, a C3-C5 cycloalkyl group optionally substituted by one or more halogen atoms, a (C1-C5 alkoxy optionally substituted by one or more halogen atoms) C1-C3 alkyl group, a C1-C5 alkylsulfonyl group optionally substituted by one or more halogen atoms, a C2-C6 alkylcarbonyl group optionally substituted by one or more halogen atoms, a C2-C5 alkoxycarbonyl group optionally substituted by one or more halogen atoms or a hydrogen atom, or represents a C2-C6 alkanediyl group optionally substituted by one or more halogen atoms or a C4-C6 alkenediyl group optionally substituted by one or more halogen atoms in each of which R¹⁰ and R¹¹ are coupled with one another at the ends thereof;
each of R¹² and R¹³ represents a C1-C5 alkyl group optionally substituted by one or more halogen atoms, a C3-C5 alkenyl group optionally substituted by one or more halogen atoms, a C3-C5 alkynyl group optionally substituted by one or more halogen atoms, a C3-C5 cycloalkyl group optionally substituted by one or more halogen atoms, a group designated by -(CH₂)ₙQ¹ or a hydrogen atom,
or represents a C2-C6 alkanediyl group optionally substituted by one or more halogen atoms or a C4 -C6 alkenediyl group optionally substituted by one or more halogen atoms in each of which R¹² and R¹³ are coupled with one another at the ends thereof;
each of R¹⁴ and R¹⁵ represents a Cl-C5 alkyl group optionally substituted by one or more halogen atoms, a C3-C5 alkenyl group optionally substituted by one or more halogen atoms, a C3-C5 alkynyl group optionally substituted by one or more halogen atoms , a C3-C5 cycloalkyl group optionally substituted by one or more halogen atoms, a group designated by -(CH₂)ₙQ² or a hydrogen atom; each of Q¹ and Q² represents a phenyl group which may be substituted by a group selected from the group consisting of a C1-C5 alkyl group optionally substituted by one or more halogen atoms, a C1-C5 alkoxy group optionally substituted by one or more halogen atoms, a C1-C5 alkylthio group optionally substituted by one or more halogen atoms and a halogen atom; X⁵ represents an oxygen atom or a sulfur atom;
and m represents an integer from 0 to 2.

5. The malononitrile compound according to Claim 1, which is represented by the formula (XIII): 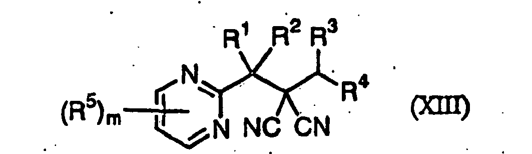 wherein R¹, R², R³, R⁴, and R⁵ have the same meaning as described in claim 4, and m represents an integer from 0 to 3.

6. The malononitrile compound according to Claim 1, which is represented by the formula (XIV): 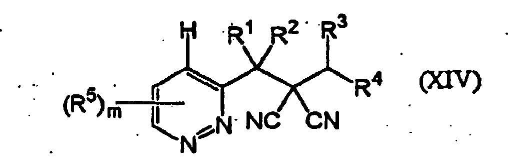 wherein R¹, R², R³, R⁴, R⁵ and m have the same meaning as described in claim 4.

7. The malononitrile compound according to Claim 1, which is represented by the formula (XV): 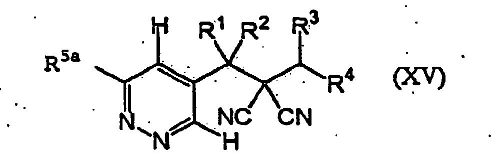 wherein R¹, R², R³, R⁴ and R^{5a} have the same meaning as described in claim 3.

8. A pesticide composition comprising the malononitrile compound according to any of claims 1 to 7 as an active ingredient and an inert carrier.

9. A method for controlling pests excluding a method for the treatment of the human or animal body by therapy comprising applying an effective amount of the malononitrile compound according to any of claims 1 to 7 to pests or at a habitat of pests.

10. Use of the malononitrile compound according to any of claims 1 to 7 for the preparation of a veterinary medicament for systemic or non-systemic control of parasites.

11. A malononitrile compound as defined in any of claims 1 to 7 for use in the therapy of animals infested by pests.

## Patentansprüche

1. Malonsäuredinitrilverbindung der Formel (I): 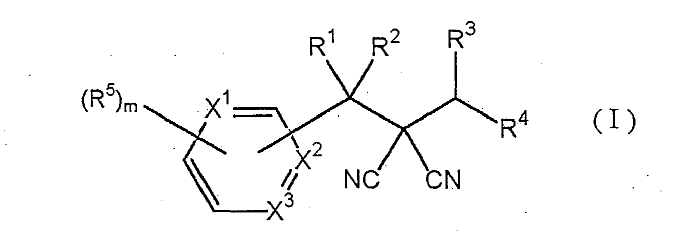
wobei in der Formel
R¹ einen gegebenenfalls mit einem oder mehreren Halogenatomen substituierten C1-C5-Alkylrest, einen gegebenenfalls mit einem oder mehreren Halogenatomen substituierten C2-C5-Alkenylrest, einen gegebenenfalls mit einem oder mehreren Halogenatomen substituierten C2-C5-Alkinylrest oder ein Wasserstoffatom darstellt;
R² einen gegebenenfalls mit einem oder mehreren Halogenatomen substituierten C1-C5-Alkylrest, einen gegebenenfalls mit einem oder mehreren Halogenatomen substituierten C1-C5-Alkoxyrest, einen gegebenenfalls mit einem oder mehreren Halogenatomen substituierten C2-C5-Alkenylrest, einen gegebenenfalls mit einem oder mehreren Halogenatomen substituierten C2-C5-Alkinylrest, eine Cyanogruppe oder ein Wasserstoffatom darstellt;
R³ und R⁴ jeweils einen gegebenenfalls mit einem oder mehreren Halogenatomen substituierten C1-C5-Alkylrest, einen gegebenenfalls mit einem oder mehreren Halogenatomen substituierten C2-C5-Alkenylrest, einen gegebenenfalls mit einem oder mehreren Halogenatomen substituierten C2-C5-Alkinylrest, einen gegebenenfalls mit einem oder mehreren Halogenatomen substituierten C3-CS-Cycloalkylrest, einen gegebenenfalls mit einem oder mehreren Halogenatomen substituierten C5-C6-Cycloalkenylrest oder ein Wasserstoffatom darstellen,
oder einen gegebenenfalls mit einem oder mehreren Halogenatomen substituierten C2-C6-Alkandiylrest oder einen gegebenenfalls mit einem oder mehreren Halogenatomen substituierten C4-C6-Alkendiylrest darstellen, wobei R³ und R⁴ jeweils an ihren Enden miteinander verknüpft sind;
X¹, X² und X³ jeweils ein Stickstoffatom oder CR⁶ darstellen, mit der Maßgabe, dass zwei oder drei von X¹, X² und X³ ein Stickstoffatom darstellen;
R⁶ ein Wasserstoffatom oder R⁵ darstellt;
jedes R⁵ ein Halogenatom, eine Cyanogruppe, eine Nitrogruppe, eine Hydroxylgruppe, eine Mercaptogruppe, eine Formylgruppe, eine SF₅-Gruppe, eine Carboxylgruppe, einen gegebenenfalls mit einem oder mehreren Halogenatomen substituierten C1-C5-Alkylrest, einen gegebenenfalls mit einem oder mehreren Halogenatomen substituierten C2-C5-Alkenylrest, einen gegebenenfalls mit einem oder mehreren Halogenatomen substituierten C2-C5-Alkinylrest, einen gegebenenfalls mit einem oder mehreren Halogenatomen substituierten C3-C5-Cycloalkylrest, einen gegebenenfalls mit einem oder mehreren Halogenatomen substituierten C1-C5-Alkoxyrest, einen gegebenenfalls mit einem oder mehreren Halogenatomen substituierten C3-C6-Alkenyloxyrest, einen gegebenenfalls mit einem oder mehreren Halogenatomen substituierten C3-C6-Alkinyloxyrest, einen gegebenenfalls mit einem oder mehreren Halogenatomen substituierten C1-C5-Alkylthiorest, einen gegebenenfalls mit einem oder mehreren Halogenatomen substituierten C3-C5-Alkenylthiorest, einen gegebenenfalls mit einem oder mehreren Halogenatomen substituierten C3-C5-Alkinylthiorest, einen gegebenenfalls mit einem oder mehreren Halogenatomen substituierten C1-C5-Alkylsulfinylrest, einen gegebenenfalls mit einem oder mehreren Halogenatomen substituierten C1-C5-Alkylsulfonylrest, einen gegebenenfalls mit einem oder mehreren Halogenatomen substituierten C2-C6-Alkylcarbonylrest, einen gegebenenfalls mit einem oder mehreren Halogenatomen substituierten C2-C5-Alkoxycarbonylrest, einen als -NR¹⁰R¹¹ bezeichneten Rest, einen als -C(=X⁵)NR¹²R¹³ bezeichneten Rest oder einen als -C(=NOR¹⁴)R¹⁵ bezeichneten Rest darstellt, oder
wenn m 2 ist und zwei R⁵ an benachbarte Kohlenstoffatome gebunden sind, diese einen C3-C6-Alkandiylrest oder einen C3-C6-Alkendiylrest darstellen können, der durch Verknüpfung der zwei R⁵ an ihren Enden gebildet wird, weiterhin unter dieser Bedingung eine in dem C3-C6-Alkandiylrest oder dem C3-C6-Alkendiylrest enthaltene Methylengruppe durch O, S(O)ᵣ oder N-R¹⁶ ersetzt sein kann und ein in dem C3-C6-Alkandiylrest oder dem C3-C6-Alkendiylrest enthaltenes Kohlenstoffatom mit einem Halogenatom oder einem gegebenenfalls mit einem oder mehreren Halogenatomen substituierten C1-C3-Alkoxyrest anstelle von (einem) Wasserstoffatom(en) verbunden sein kann;
R¹⁰ und R¹¹ jeweils einen gegebenenfalls mit einem oder mehreren Halogenatomen substituierten C1-C5-Alkylrest, einen gegebenenfalls mit einem oder mehreren Halogenatomen substituierten C3-C5-Alkenylrest, einen gegebenenfalls mit einem oder mehreren Halogenatomen substituierten C3-C5-Alkinylrest, einen gegebenenfalls mit einem oder mehreren Halogenatomen substituierten C3-C5-Cycloalkylrest, einen (gegebenenfalls mit einem oder mehreren Halogenatomen substituierten C1-C5-Alkoxy-)C1-C3-Alkylrest, einen gegebenenfalls mit einem oder mehreren Halogenatomen substituierten C1-C5-Alkylsulfonylrest, einen gegebenenfalls mit einem oder mehreren Halogenatomen substituierten C2-C6-Alkylcarbonylrest, einen gegebenenfalls mit einem oder mehreren Halogenatomen substituierten C2-C5-Alkoxycarbonylrest oder ein Wasserstoffatom darstellen,
oder einen gegebenenfalls mit einem oder mehreren Halogenatomen substituierten C2-C6-Alkandiylrest oder einen gegebenenfalls mit einem oder mehreren Halogenatomen substituierten C4-C6-Alkendiylrest darstellen, wobei R¹⁰ und R¹¹ jeweils an ihren Enden miteinander verknüpft sind;
R¹² und R¹³ jeweils einen gegebenenfalls mit einem oder mehreren Halogenatomen substituierten C1-C5-Alkylrest, einen gegebenenfalls mit einem oder mehreren Halogenatomen substituierten C3-C5-Alkenylrest, einen gegebenenfalls mit einem oder mehreren Halogenatomen substituierten C3-C5-Alkinylrest, einen gegebenenfalls mit einem oder mehreren Halogenatomen substituierten C3-C5-Cycloalkylrest, einen als -(CH₂)ₙQ¹ bezeichneten Rest oder ein Wasserstoffatom darstellen,
oder einen gegebenenfalls mit einem oder mehreren Halogenatomen substituierten C2-C6-Alkandiylrest oder einen gegebenenfalls mit einem oder mehreren Halogenatomen substituierten C4-C6-Alkendiylrest darstellen, wobei R¹² und R¹³ jeweils an ihren Enden miteinander verknüpft sind;
R¹⁴ und R¹⁵ jeweils einen gegebenenfalls mit einem oder mehreren Halogenatomen substituierten C1-C5-Alkylrest, einen gegebenenfalls mit einem oder mehreren Halogenatomen substituierten C3-C5-Alkenylrest, einen gegebenenfalls mit einem oder mehreren Halogenatomen substituierten C3-C5-Alkinylrest, einen gegebenenfalls mit einem oder mehreren Halogenatomen substituierten C3-C5-Cycloalkylrest, einen als -(CH₂)ₙQ² bezeichneten Rest oder ein Wasserstoffatom darstellen;
Q¹ und Q² jeweils eine Phenylgruppe darstellen, welche mit einem Rest ausgewählt aus einem gegebenenfalls mit einem oder mehreren Halogenatomen substituierten C1-C5-Alkylrest, einem gegebenenfalls mit einem oder mehreren Halogenatomen substituierten C1-C5-Alkoxyrest, einem gegebenenfalls mit einem oder mehreren Halogenatomen substituierten C1-C5-Alkylthiorest und einem Halogenatom substituiert sein kann;
R¹⁶ einen C1-C3-Alkylrest darstellt; m eine ganze Zahl von 0 bis 2 darstellt; n eine ganze Zahl von 0 bis 3 darstellt; r eine ganze Zahl von 0 bis 2 darstellt; und
X⁵ ein Sauerstoffatom oder ein Schwefelatom darstellt.

2. Malonsäuredinitrilverbindung gemäß Anspruch 1, wobei R³ ein Wasserstoffatom ist und R⁴ ein gegebenenfalls mit einem oder mehreren Halogenatomen substituierter C1-C5-Alkylrest, ein gegebenenfalls mit einem oder mehreren Halogenatomen substituierter C2-C5-Alkenylrest, ein gegebenenfalls mit einem oder mehreren Halogenatomen substituierter C2-C5-Alkinylrest, ein gegebenenfalls mit einem oder mehreren Halogenatomen substituierter C3-C5-Cycloalkylrest oder ein gegebenenfalls mit einem oder mehreren Halogenatomen substituierter C5-C6-Cycloalkenylrest ist.

3. Malonsäuredinitrilverbindung gemäß Anspruch 1 der Formel (XI): 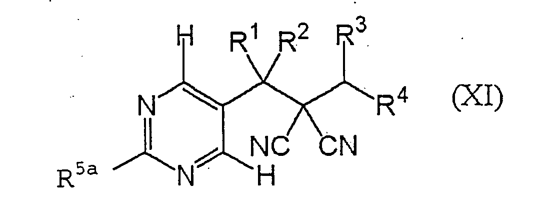
wobei R¹ einen gegebenenfalls mit einem oder mehreren Halogenatomen substituierten C1-C5-Alkylrest, einen gegebenenfalls mit einem oder mehreren Halogenatomen substituierten C2-C5-Alkenylrest, einen gegebenenfalls mit einem oder mehreren Halogenatomen substituierten C2-C5-Alkinylrest oder ein Wasserstoffatom darstellt;
R² einen gegebenenfalls mit einem oder mehreren Halogenatomen substituierten C1-C5-Alkylrest, einen gegebenenfalls mit einem oder mehreren Halogenatomen substituierten C1-C5-Alkoxyrest, einen gegebenenfalls mit einem oder mehreren Halogenatomen substituierten C2-C5-Alkenylrest, einen gegebenenfalls mit einem oder mehreren Halogenatomen substituierten C2-C5-Alkinylrest, eine Cyanogruppe oder ein Wasserstoffatom darstellt;
R³ und R⁴ jeweils einen gegebenenfalls mit einem oder mehreren Halogenatomen substituierten C1-C5-Alkylrest, einen gegebenenfalls mit einem oder mehreren Halogenatomen substituierten C2-C5-Alkenylrest, einen gegebenenfalls mit einem oder mehreren Halogenatomen substituierten C2-C5-Alkinylrest, einen gegebenenfalls mit einem oder mehreren Halogenatomen substituierten C3-C5-Cycloalkylrest, einen gegebenenfalls mit einem oder mehreren Halogenatomen substituierten C5-C6-Cycloalkenylrest oder ein Wasserstoffatom darstellen,
oder einen gegebenenfalls mit einem oder mehreren Halogenatomen substituierten C2-C6-Alkandiylrest oder einen gegebenenfalls mit einem oder mehreren Halogenatomen substituierten C4-C6-Alkendiylrest darstellen, wobei R³ und R⁴ jeweils an ihren Enden miteinander verknüpft sind;
R^{5a} ein Wasserstoffatom, Halogenatom, eine Cyanogruppe, eine Nitrogruppe, eine Hydroxylgruppe, eine Mercaptogruppe, eine Formylgruppe, eine SF₅-Gruppe, eine Carboxylgruppe, einen gegebenenfalls mit einem oder mehreren Halogenatomen substituierten C1-C5-Alkylrest, einen gegebenenfalls mit einem oder mehreren Halogenatomen substituierten C2-C5-Alkenylrest, einen gegebenenfalls mit einem oder mehreren Halogenatomen substituierten C2-C5-Alkinylrest, einen gegebenenfalls mit einem oder mehreren Halogenatomen substituierten C3-C5-Cycloalkylrest, einen gegebenenfalls mit einem oder mehreren Halogenatomen substituierten C1-C5-Alkoxyrest, einen gegebenenfalls mit einem oder mehreren Halogenatomen substituierten C3-C6-Alkenyloxyrest, einen gegebenenfalls mit einem oder mehreren Halogenatomen substituierten C3-C6-Alkinyloxyrest, einen gegebenenfalls mit einem oder mehreren Halogenatomen substituierten C1-C5-Alkylthiorest, einen gegebenenfalls mit einem oder mehreren Halogenatomen substituierten C3-C5-Alkenylthiorest, einen gegebenenfalls mit einem oder mehreren Halogenatomen substituierten C3-C5-Alkinylthiorest, einen gegebenenfalls mit einem oder mehreren Halogenatomen substituierten C1-C5-Alkylsulfinylrest, einen gegebenenfalls mit einem oder mehreren Halogenatomen substituierten C1-C5-Alkylsulfonylrest, einen gegebenenfalls mit einem oder mehreren Halogenatomen substituierten C2-C6-Alkylcarbonylrest, einen gegebenenfalls mit einem oder mehreren Halogenatomen substituierten C2-C5-Alkoxycarbonylrest, einen als -NR¹⁰R¹¹ bezeichneten Rest, einen als -C(=X⁵)NR¹²R¹³ bezeichneten Rest oder einen als -C(=NOR¹⁴)R¹⁵ bezeichneten Rest darstellt;
R¹⁰ und R¹¹ jeweils einen gegebenenfalls mit einem oder mehreren Halogenatomen substituierten C1-C5-Alkylrest, einen gegebenenfalls mit einem oder mehreren Halogenatomen substituierten C3-C5-Alkenylrest, einen gegebenenfalls mit einem oder mehreren Halogenatomen substituierten C3-C5-Alkinylrest, einen gegebenenfalls mit einem oder mehreren Halogenatomen substituierten C3-C5-Cycloalkylrest, einen (gegebenenfalls mit einem oder mehreren Halogenatomen substituierten C1-C5-Alkoxy-)C1-C3-Alkylrest, einen gegebenenfalls mit einem oder mehreren Halogenatomen substituierten C1-C5-Alkylsulfonylrest, einen gegebenenfalls mit einem oder mehreren Halogenatomen substituierten C2-C6-Alkylcarbonylrest, einen gegebenenfalls mit einem oder mehreren Halogenatomen substituierten C2-C5-Alkoxycarbonylrest oder ein Wasserstoffatom darstellen,
oder einen gegebenenfalls mit einem oder mehreren Halogenatomen substituierten C2-C6-Alkandiylrest oder einen gegebenenfalls mit einem oder mehreren Halogenatomen substituierten C4-C6-Alkendiylrest darstellen, wobei R¹⁰ und R¹¹ jeweils an ihren Enden miteinander verknüpft sind;
R¹² und R¹³ jeweils einen gegebenenfalls mit einem oder mehreren Halogenatomen substituierten C1-C5-Alkylrest, einen gegebenenfalls mit einem oder mehreren Halogenatomen substituierten C3-C5-Alkenylrest, einen gegebenenfalls mit einem oder mehreren Halogenatomen substituierten C3-C5-Alkinylrest, einen gegebenenfalls mit einem oder mehreren Halogenatomen substituierten C3-C5-Cycloalkylrest, einen als -(CH₂)ₙQ¹ bezeichneten Rest oder ein Wasserstoffatom darstellen,
oder einen gegebenenfalls mit einem oder mehreren Halogenatomen substituierten C2-C6-Alkandiylrest oder einen gegebenenfalls mit einem oder mehreren Halogenatomen substituierten C4-C6-Alkendiylrest darstellen, wobei R¹² und R¹³ jeweils an ihren Enden miteinander verknüpft sind;
R¹⁴ und R¹⁵ jeweils einen gegebenenfalls mit einem oder mehreren Halogenatomen substituierten C1-C5-Alkylrest, einen gegebenenfalls mit einem oder mehreren Halogenatomen substituierten C3-C5-Alkenylrest, einen gegebenenfalls mit einem oder mehreren Halogenatomen substituierten C3-C5-Alkinylrest, einen gegebenenfalls mit einem oder mehreren Halogenatomen substituierten C3-C5-Cycloalkylrest, einen als -(CH₂)ₙQ² bezeichneten Rest oder ein Wasserstoffatom darstellen;
Q¹ und Q² jeweils eine Phenylgruppe darstellen, welche mit einem Rest, ausgewählt aus einem gegebenenfalls mit einem oder mehreren Halogenatomen substituierten C1-C5-Alkylrest, einem gegebenenfalls mit einem oder mehreren Halogenatomen substituierten C1-C5-Alkoxyrest, einem gegebenenfalls mit einem oder mehreren Halogenatomen substituierten C1-C5-Alkylthiorest und einem Halogenatom, substituiert sein kann; und X⁵ ein Sauerstoffatom oder ein Schwefelatom darstellt.

4. Malonsäuredinitrilverbindung gemäß Anspruch 1 der Formel (XII): 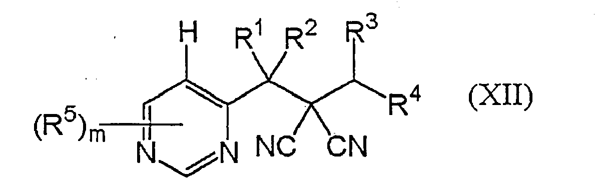
wobei R¹ einen gegebenenfalls mit einem oder mehreren Halogenatomen substituierten C1-C5-Alkylrest, einen gegebenenfalls mit einem oder mehreren Halogenatomen substituierten C2-C5-Alkenylrest, einen gegebenenfalls mit einem oder mehreren Halogenatomen substituierten C2-C5-Alkinylrest oder ein Wasserstoffatom darstellt;
R² einen gegebenenfalls mit einem oder mehreren Halogenatomen substituierten C1-C5-Alkylrest, einen gegebenenfalls mit einem oder mehreren Halogenatomen substituierten C1-C5-Alkoxyrest, einen gegebenenfalls mit einem oder mehreren Halogenatomen substituierten C2-C5-Alkenylrest, einen gegebenenfalls mit einem oder mehreren Halogenatomen substituierten C2-C5-Alkinylrest, eine Cyanogruppe oder ein Wasserstoffatom darstellt;
R³ und R⁴ jeweils einen gegebenenfalls mit einem oder mehreren Halogenatomen substituierten C1-C5-Alkylrest, einen gegebenenfalls mit einem oder mehreren Halogenatomen substituierten C2-C5-Alkenylrest, einen gegebenenfalls mit einem oder mehreren Halogenatomen substituierten C2-C5-Alkinylrest, einen gegebenenfalls mit einem oder mehreren Halogenatomen substituierten C3-C5-Cycloalkylrest, einen gegebenenfalls mit einem oder mehreren Halogenatomen substituierten C5-C6-Cycloalkenylrest oder ein Wasserstoffatom darstellen,
oder einen gegebenenfalls mit einem oder mehreren Halogenatomen substituierten C2-C6-Alkandiylrest oder einen gegebenenfalls mit einem oder mehreren Halogenatomen substituierten C4-C6-Alkendiylrest darstellen, wobei R³ und R⁴ jeweils an ihren Enden miteinander verknüpft sind;
R⁵ ein Halogenatom, eine Cyanogruppe, eine Nitrogruppe, eine Hydroxylgruppe, eine Mercaptogruppe, eine Formylgruppe, einen SF₅-Gruppe, eine Carboxylgruppe, einen gegebenenfalls mit einem oder mehreren Halogenatomen substituierten C1-C5-Alkylrest, einen gegebenenfalls mit einem oder mehreren Halogenatomen substituierten C2-C5-Alkenylrest, einen gegebenenfalls mit einem oder mehreren Halogenatomen substituierten C2-C5-Alkinylrest, einen gegebenenfalls mit einem oder mehreren Halogenatomen substituierten C3-C5-Cycloalkylrest, einen gegebenenfalls mit einem oder mehreren Halogenatomen substituierten C1-C5-Alkoxyrest, einen gegebenenfalls mit einem oder mehreren Halogenatomen substituierten C3-C6-Alkenyloxyrest, einen gegebenenfalls mit einem oder mehreren Halogenatomen substituierten C3-C6-Alkinyloxyrest, einen gegebenenfalls mit einem oder mehreren Halogenatomen substituierten C1-C5-Alkylthiorest, einen gegebenenfalls mit einem oder mehreren Halogenatomen substituierten C3-C5-Alkenylthiorest, einen gegebenenfalls mit einem oder mehreren Halogenatomen substituierten C3-C5-Alkinylthiorest, einen gegebenenfalls mit einem oder mehreren Halogenatomen substituierten C1-C5-Alkylsulfinylrest, einen gegebenenfalls mit einem oder mehreren Halogenatomen substituierten C1-C5-Alkylsulfonylrest, einen gegebenenfalls mit einem oder mehreren Halogenatomen substituierten C2-C6-Alkylcarbonylrest, einen gegebenenfalls mit einem oder mehreren Halogenatomen substituierten C2-C5-Alkoxycarbonylrest, einen als -NR¹⁰R¹¹ bezeichneten Rest, einen als -C(=X⁵)NR¹²R¹³ bezeichneten Rest oder einen als -C(=NOR¹⁴)R¹⁵ bezeichneten Rest darstellt;
R¹⁰ und R¹¹ jeweils einen gegebenenfalls mit einem oder mehreren Halogenatomen substituierten C1-C5-Alkylrest, einen gegebenenfalls mit einem oder mehreren Halogenatomen substituierten C3-C5-Alkenylrest, einen gegebenenfalls mit einem oder mehreren Halogenatomen substituierten C3-C5-Alkinylrest, einen gegebenenfalls mit einem oder mehreren Halogenatomen substituierten C3-C5-Cycloalkylrest, einen (gegebenenfalls mit einem oder mehreren Halogenatomen substituierten C1-C5-Alkoxy-)C1-C3-Alkylrest, einen gegebenenfalls mit einem oder mehreren Halogenatomen substituierten C1-C5-Alkylsulfonylrest, einen gegebenenfalls mit einem oder mehreren Halogenatomen substituierten C2-C6-Alkylcarbonylrest, einen gegebenenfalls mit einem oder mehreren Halogenatomen substituierten C2-C5-Alkoxycarbonylrest oder ein Wasserstoffatom darstellen,
oder einen gegebenenfalls mit einem oder mehreren Halogenatomen substituierten C2-C6-Alkandiylrest oder einen gegebenenfalls mit einem oder mehreren Halogenatomen substituierten C4-C6-Alkendiylrest darstellen, wobei R¹⁰ und R¹¹ jeweils an ihren Enden miteinander verknüpft sind;
R¹² und R¹³ jeweils einen gegebenenfalls mit einem oder mehreren Halogenatomen substituierten C1-C5-Alkylrest, einen gegebenenfalls mit einem oder mehreren Halogenatomen substituierten C3-C5-Alkenylrest, einen gegebenenfalls mit einem oder mehreren Halogenatomen substituierten C3-C5-Alkinylrest, einen gegebenenfalls mit einem oder mehreren Halogenatomen substituierten C3-C5-Cycloalkylrest, einen als -(CH₂)ₙQ¹ bezeichneten Rest oder ein Wasserstoffatom darstellen,
oder einen gegebenenfalls mit einem oder mehreren Halogenatomen substituierten C2-C6-Alkandiylrest oder einen gegebenenfalls mit einem oder mehreren Halogenatomen substituierten C4-C6-Alkendiylrest darstellen, wobei R¹² und R¹³ jeweils an ihren Enden miteinander verknüpft sind;
R¹⁴ und R¹⁵ jeweils einen gegebenenfalls mit einem oder mehreren Halogenatomen substituierten C1-C5-Alkylrest, einen gegebenenfalls mit einem oder mehreren Halogenatomen substituierten C3-C5-Alkenylrest, einen gegebenenfalls mit einem oder mehreren Halogenatomen substituierten C3-C5-Alkinylrest, einen gegebenenfalls mit einem oder mehreren Halogenatomen substituierten C3-C5-Cycloalkylrest, einen als -(CH₂)ₙQ² bezeichneten Rest oder ein Wasserstoffatom darstellen;
Q¹ und Q² jeweils eine Phenylgruppe darstellen, welche mit einem Rest, ausgewählt aus einem gegebenenfalls mit einem oder mehreren Halogenatomen substituierten C1-C5-Alkylrest, einem gegebenenfalls mit einem oder mehreren Halogenatomen substituierten Cl-C5-Alkoxyrest, einem gegebenenfalls mit einem oder mehreren Halogenatomen substituierten C1-C5-Alkylthiorest und einem Halogenatom, substituiert sein kann;
X⁵ ein Sauerstoffatom oder ein Schwefelatom darstellt; und m eine ganze Zahl von 0 bis 2 darstellt.

5. Malonsäuredinitrilverbindung gemäß Anspruch 1 der Formel (XIII): 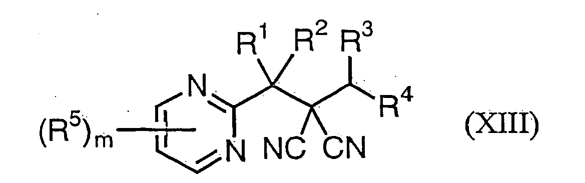
wobei R¹, R², R³, R⁴ und R⁵ die gleiche Bedeutung wie in Anspruch 4 haben, und m eine ganze Zahl von 0 to 3 darstellt.

6. Malonsäuredinitrilverbindung gemäß Anspruch 1 der Formel (XIV): 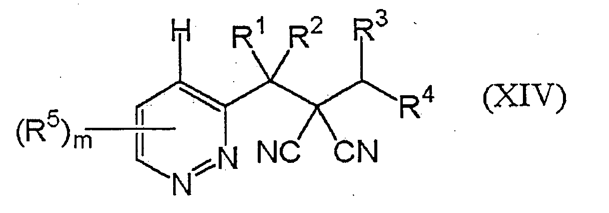 wobei R¹, R², R³, R⁴ , R⁵ und m die gleiche Bedeutung wie in Anspruch 4 haben.

7. Malonsäuredinitrilverbindung gemäß Anspruch 1 der Formel (XV): 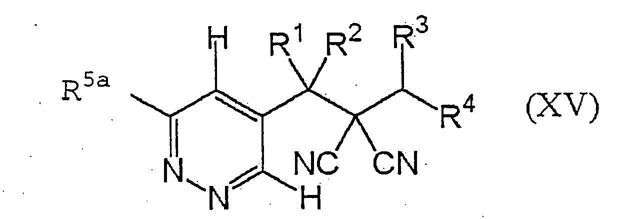 wobei R¹, R² , R³, R⁴ und R^{5a} die gleiche Bedeutung wie in Anspruch 3 haben.

8. Pestizidzusammensetzung umfassend die Malonsäuredinitrilverbindung gemäß einem der Ansprüche 1 bis 7 als aktiven Bestandteil und einen inerten Träger.

9. Verfahren zur Schädlingsbekämpfung, wobei Verfahren zur therapeutischen Behandlung des menschlichen oder tierischen Körpers ausgeschlossen sind, umfassend das Anwenden einer wirksamen Menge der Malonsäuredinitrilverbindung gemäß einem der Ansprüche 1 bis 7 auf Schädlinge oder den Lebensraum von Schädlingen.

10. Verwendung der Malonsäuredinitrilverbindung gemäß einem der Ansprüche 1 bis 7 zur Herstellung eines Tierheilmittels für die systemische oder nicht-systemische Kontrolle von Parasiten.

11. Malonsäuredinitrilverbindung gemäß einem der Ansprüche 1 bis 7 zur Verwendung in der Therapie von mit Schädlingen befallenen Tieren.

## Revendications

1. Composé malononitrile représenté par la formule (I) : 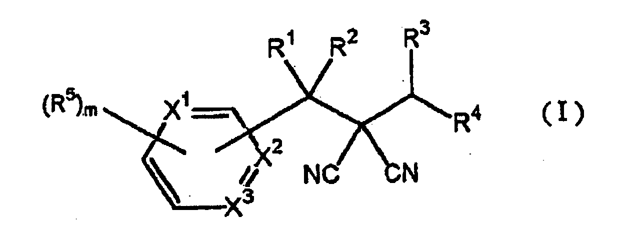 où, dans la formule,
R¹ représente un groupe alkyle en C₁ à C₅ facultativement substitué par un ou plusieurs atomes d'halogène, un groupe alcényle en C₂ à C₅ facultativement substitué par un ou plusieurs atomes d'halogène, un groupe alcynyle en C₂ à C₅ facultativement substitué par un ou plusieurs atomes d'halogène ou un atome d'hydrogène ;
R² représente un groupe alkyle en C₁ à C₅ facultativement substitué par un ou plusieurs atomes d'halogène, un groupe alcoxy en C₁ à C₅ facultativement substitué par un ou plusieurs atomes d'halogène, un groupe alcényle en C₂ à C₅ facultativement substitué par un ou plusieurs atomes d'halogène, un groupe alcynyle en C₂ à C₅ facultativement substitué par un ou plusieurs atomes d'halogène, un groupe cyano ou un atome d'hydrogène ;
chacun de R³ et R⁴ représente un groupe alkyle en C₁ à C₅ facultativement substitué par un ou plusieurs atomes d'halogène, un groupe alcényle en C₂ à C₅ facultativement substitué par un ou plusieurs atomes d'halogène, un groupe alcynyle en C₂ à C₅ facultativement substitué par un ou plusieurs atomes d'halogène, un groupe cycloalkyle en C₃ à C₅ facultativement substitué par un ou plusieurs atomes d'halogène, un groupe cycloalcényle en C₅ à C₆ facultativement substitué par un ou plusieurs atomes d'halogène ou un atome d'hydrogène,
ou représente un groupe alcanediyle en C₂ à C₆ facultativement substitué par un ou plusieurs atomes d'halogène ou un groupe alcènediyle en C₄ à C₆ facultativement substitué par un ou plusieurs atomes d'halogène dans chacun desquels R³ et R⁴ se couple l'un à l'autre aux extrémités de celui-ci ;
chacun X¹, X² et X³ représente un atome d'azote ou CR⁶, à condition que deux ou trois parmi X¹, X² et X³ représentent un atome d'azote ;
R⁶ représente un atome d'hydrogène ou R⁵ ;
chacun des R⁵ représente un atome d'halogène, un groupe cyano, un groupe nitro, un groupe hydroxyle, un groupe mercapto, un groupe formyle, un groupe SF₅, un groupe carboxyle, un groupe alkyle en C₁ à C₅ facultativement substitué par un ou plusieurs atomes d'halogène, un groupe alcényle en C₂ à C₅ facultativement substitué par un ou plusieurs atomes d'halogène, un groupe alcynyle en C₂ à C₅ facultativement substitué par un ou plusieurs atomes d'halogène, un groupe cycloalkyle en C₃ à C₅ facultativement substitué par un ou plusieurs atomes d'halogène, un groupe alcoxy en C₁ à C₅ facultativement substitué par un ou plusieurs atomes d'halogène, un groupe alcényloxy en C₃ à C₆ facultativement substitué par un ou plusieurs atomes d'halogène, un groupe alcynyloxy en C₃ à C₆ facultativement substitué par un ou plusieurs atomes d'halogène, un groupe alkylthio en C₁ à C₅ facultativement substitué par un ou plusieurs atomes d'halogène, un groupe alcénylthio en C₃ à C₅ facultativement substitué par un ou plusieurs atomes d'halogène, un groupe alcynylthio en C₃ à C₅ facultativement substitué par un ou plusieurs atomes d'halogène, un groupe alkylsulfinyle en C₁ à C₅ facultativement substitué par un ou plusieurs atomes d'halogène, un groupe alkylsulfonyle en C₁ à C₅ facultativement substitué par un ou plusieurs atomes d'halogène, un groupe alkylcarbonyle en C₂ à C₆ facultativement substitué par un ou plusieurs atomes d'halogène, un groupe alcoxycarbonyle en C₂ à C₅ facultativement substitué par un ou plusieurs atomes d'halogène, un groupe désigné par -NR¹⁰R¹¹, un groupe désigné par -C(=X⁵)NR¹²R¹³ ou un groupe désigné .par -C(=NOR¹⁴)R¹⁵, ou
où m vaut 2 et deux R⁵ sont couplés à deux atomes de carbone adjacents peut représenter un groupe alcanediyle en C₃ à C₆ ou un groupe alcènediyle en C₃ à C₆ dont chacun est formé en couplant les deux R⁵ aux extrémités de celui-ci, en outre, dans cette condition, un groupe méthylène contenu dans ledit groupe alcanediyle en C₃ à C₆ ou ledit groupe alcènediyle en C₃ à C₆ peut être remplacé par O, S(O)ᵣ ou NR¹⁶ et un atome de carbone contenu dans ledit groupe alcanediyle en C₃ à C₆ ou ledit groupe alcènediyle en C₃ à C₆ peut être couplé avec un atome d'halogène ou un groupe alcoxy en C₁ à C₃ facultativement substitué par un ou plusieurs atomes d'halogène au lieu de un ou des atomes d'hydrogène ;
chacun de R¹⁰ et R¹¹ représente un groupe alkyle en C₁ à C₅ facultativement substitué par un ou plusieurs atomes d'halogène, un groupe alcényle en C₃ à C₅ facultativement substitué par un ou plusieurs atomes d'halogène, un groupe alcynyle en C₃ à C₅ facultativement substitué par un ou plusieurs atomes d'halogène, un groupe cycloalkyle en C₃ à C₅ facultativement substitué par un ou plusieurs atomes d'halogène, un groupe alkyle en C₁ à C₃ (alcoxy en C₁ à C₅ facultativement substitué par un ou plusieurs atomes d'halogène), un groupe alkylsulfonyle en C₁ à C₅ facultativement substitué par un ou plusieurs atomes d'halogène, un groupe alkylcarbonyle en C₂ à C₆ facultativement substitué par un ou plusieurs atomes d'halogène, un groupe alcoxycarbonyle en C₂ à C₅ facultativement substitué par un ou plusieurs atomes d'halogène ou un atome d'hydrogène, ou représente un groupe alcanediyle en C₂ à C₆ facultativement substitué par un ou plusieurs atomes d'halogène ou un groupe alcènediyle en C₄ à C₆ facultativement substitué par un ou plusieurs atomes d'halogène dans chacun desquels R¹⁰ et R¹¹ sont couplés l'un avec l'autre aux extrémités de celui-ci ; chacun de R¹² et R¹³ représente un groupe alkyle en C₁ à C₅ facultativement substitué par un ou plusieurs atomes d'halogène, un groupe alcényle en C₃ à C₅ facultativement substitué par un ou plusieurs atomes d'halogène, un groupe alcynyle en C₃ à C₅ facultativement substitué par un ou plusieurs atomes d'halogène, un groupe cycloalkyle en C₃ à C₅ facultativement substitué par un ou plusieurs atomes d'halogène, un groupe désigné par -(CH₂)ₙQ¹ ou un atome d'hydrogène,
ou représente un groupe alcanediyle en C₂ à C₆ facultativement substitué par un ou plusieurs atomes d'halogène ou groupe alcènediyle en C₄ à C₆ facultativement substitué par un ou plusieurs atomes d'halogène dans chacun desquels R¹² et R¹³ sont couplés l'un avec l'autre aux extrémités de celui-ci ;
chacun de R¹⁴ et R¹⁵ représente un groupe alkyle en C₁ à C₅ facultativement substitué par un ou plusieurs atomes d'halogène, un groupe alcényle en C₃ à C₅ facultativement substitué par un ou plusieurs atomes d'halogène, un groupe alcynyle en C₃ à C₅ facultativement substitué par un ou plusieurs atomes d'halogène, un groupe cycloalkyle en C₃ à C₅ facultativement substitué par un ou plusieurs atomes d'halogène,
un groupe désigné par -(CH₂)ₙQ² ou un atome d'hydrogène ;
chacun de Q¹ et Q² représente un groupe phényle qui peut être substitué par un groupe choisi dans le groupe constitué d'un groupe alkyle en C₁ à C₅ facultativement substitué par un ou plusieurs atomes d'halogène, un groupe alcoxy en C₁ à C₅ facultativement substitué par un ou plusieurs atomes d'halogène, un groupe alkylthio en C₁ à C₅ facultativement substitué par un ou plusieurs atomes d'halogène et un atome d'halogène ;
R¹⁶ représente un groupe alkyle en C₁ à C₃ ; m représente un entier de 0 à 2 ; n représente un entier de 0 à 3 ; r représente un entier de 0 à 2 ; et
X⁵ représente un atome d'oxygène ou un atome de soufre.

2. Composé malononitrile selon la revendication 1, dans lequel R³ est un atome d'hydrogène et R⁴ est un groupe alkyle en C₁ à C₅ facultativement substitué par un ou plusieurs atomes d'halogène, une groupe alcényle en C₂ à C₅ facultativement substitué par un ou plusieurs atomes d'halogène, un groupe alcynyle en C₂ à C₅ facultativement substitué par un ou plusieurs atomes d'halogène, un groupe cycloalkyle en C₃ à C₅ facultativement substitué par un ou plusieurs atomes d'halogène ou un groupe cycloalcényle en C₅ à C₆ facultativement substitué par un ou plusieurs atomes d'halogène.

3. Composé malononitrile selon la revendication 1, qui est représenté par la formule (XI) : 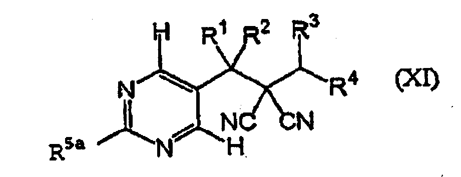
dans laquelle R¹ représente un groupe alkyle en C₁ à C₅ facultativement substitué par un ou plusieurs atomes d'halogène, un groupe alcényle en C₂ à C₅ facultativement substitué par un ou plusieurs atomes d'halogène, un groupe alcynyle en C₂ à C₅ facultativement substitué par un ou plusieurs atomes d'halogène ou un atome d'hydrogène;
R² représente un groupe alkyle en C₁ à C₅ facultativement substitué par un ou plusieurs atomes d'halogène, un groupe alcoxy en C₁ à C₅ facultativement substitué par un ou plusieurs atomes d'halogène, un groupe alcényle en C₂ à C₅ facultativement substitué par un ou plusieurs atomes d'halogène, un groupe alcynyle en C₂ à C₅ facultativement substitué par un ou plusieurs atomes d'halogène, un groupe cyano ou un atome d'hydrogène;
chacun de R³ et R⁴ représente un groupe alkyle en C₁ à C₅ facultativement substitué par un ou plusieurs atomes d'halogène, un groupe alcényle en C₂ à C₅ facultativement substitué par un ou plusieurs atomes d'halogène, un groupe alcynyle en C₂ à C₅ facultativement substitué par un ou plusieurs atomes d'halogène, un groupe cycloalkyle en C₃ à C₅ facultativement, substitué par un ou plusieurs atomes d'halogène, un groupe cycloalcényle en C₅ à C₆ facultativement substitué par un ou plusieurs atomes d'halogène ou un atome d'hydrogène,
ou représente un groupe alcanediyle en C₂ à C₆ facultativement substitué par un ou plusieurs atomes d'halogène ou un groupe alcènediyle en C₄ à C₆ facultativement substitué par un ou plusieurs atomes d'halogène dans chacun desquels R³ et R⁴ se couplent l'un à l'autre aux extrémités de celui-ci ;
R^{5a} représente un atome d'hydrogène, un atome d'halogène, un groupe cyano, un groupe nitro, un groupe hydroxyle, un groupe mercapto, un groupe formyle, un groupe SF₅, un groupe carboxyle, un groupe alkyle en C₁ à C₅ facultativement substitué par un ou plusieurs atomes d'halogène, un groupe alcényle en C₂ à C₅ facultativement substitué par un ou plusieurs atomes d'halogène, un groupe alcynyle en C₂ à C₅ facultativement substitué par un ou plusieurs atomes d'halogène, un groupe cycloalkyle en C₃ à C₅ facultativement substitué par un ou plusieurs atomes d'halogène, un groupe alcoxy en C₁ à C₅ facultativement substitué par un ou plusieurs atomes d'halogène, un groupe alcényloxy en C₃ à C₆ facultativement substitué par un ou plusieurs atomes d'halogène, un groupe alcynyloxy en C₃ à C₆ facultativement substitué par un ou plusieurs atomes d'halogène, un groupe alkylthio en C₁ à C₅ facultativement substitué par un ou plusieurs atomes d'halogène, un groupe alcénylthio en C₃ à C₅ facultativement substitué par un ou plusieurs atomes d'halogène, un groupe alcynylthio en C₃ à C₅ facultativement substitué par un ou plusieurs atomes d'halogène, un groupe alkylsulfinyle en C₁ à C₅ facultativement substitué par un ou plusieurs atomes d'halogène, un groupe alkylsulfonyle en C₁ à C₅ facultativement substitué par un ou plusieurs atomes d'halogène, un groupe alkylcarbonyle en C₂ à C₆ facultativement substitué par un ou plusieurs atomes d'halogène, un groupe alcoxycarbonyle en C₂ à C₅ facultativement substitué par un ou plusieurs atomes d'halogène, un groupe désigné par -NR¹⁰R¹¹, un groupe désigné par -C(=X⁵)NR¹²R¹³ ou un groupe désigné par -C(=NOR¹⁴)R¹⁵,
chacun de R¹⁰ et R¹¹ représente un groupe alkyle en C₁ à C₅ facultativement substitué par un ou plusieurs atomes d'halogène, un groupe alcényle en C₃ à C₅ facultativement substitué par un ou plusieurs atomes d'halogène, un groupe alcynyle en C₃ à C₅ facultativement substitué par un ou plusieurs atomes d'halogène, un groupe cycloalkyle en C₃ à C₅ facultativement substitué par un ou plusieurs atomes d'halogène, un groupe alkyle en C₁ à C₃ (alcoxy en C₁ à C₅ facultativement substitué par un ou plusieurs atomes d'halogène), un groupe alkylsulfonyle en C₁ à C₅ facultativement substitué par un ou plusieurs atomes d'halogène, un groupe alkylcarbonyle en C₂ à C₆ facultativement substitué par un ou plusieurs atomes d'halogène, un groupe alcoxycarbonyle en C₂ à C₅ facultativement substitué par un ou plusieurs atomes d'halogène ou un atome d'hydrogène, ou représente un groupe alcanediyle en C₂ à C₆ facultativement substitué par un ou plusieurs atomes d'halogène ou un groupe alcènediyle en C₄ à C₆ facultativement substitué par un ou plusieurs atomes d'halogène dans chacun desquels R¹⁰ et R¹¹ sont couplés l'un avec l'autre aux extrémités de celui-ci ;
chacun de R¹² et R¹³ représente un groupe alkyle en C₁ à C₅ facultativement substitué par un ou plusieurs atomes d'halogène, un groupe alcényle en C₃ à C₅ facultativement substitué par un ou plusieurs atomes d'halogène, un groupe alcynyle en C₃ à C₅ facultativement substitué par un ou plusieurs atomes d'halogène, un groupe cycloalkyle en C₃ à C₅ facultativement substitué par un ou plusieurs atomes d'halogène, un groupe désigné par -(CH₂)ₙQ¹ ou un atome d'hydrogène,
ou représente un groupe alcanediyle en C₂ à C₆ facultativement substitué par un ou plusieurs atomes d'halogène ou groupe alcènediyle en C₄ à C₆ facultativement substitué par un ou plusieurs atomes d'halogène dans chacun desquels R¹² et R¹³ sont couplés l'un avec l'autre aux extrémités de celui-ci ;
chacun de R¹⁴ et R¹⁵ représente un groupe alkyle en C₁ à C₅ facultativement substitué par un ou plusieurs atomes d'halogène, un groupe alcényle en C₃ à C₅ facultativement substitué par un ou plusieurs atomes d'halogène, un groupe alcynyle en C₃ à C₅ facultativement substitué par un ou plusieurs atomes d'halogène, un groupe cycloalkyle en C₃ à C₅ facultativement substitué par un ou plusieurs atomes d'halogène, un groupe désigné par -(CH₂)ₙQ² ou un atome d'hydrogène ; chacun de Q¹ et Q² représente un groupe phényle qui peut être substitué par un groupe choisi dans le groupe constitué d'un groupe alkyle en C₁ à C₅ facultativement substitué par un ou plusieurs atomes d'halogène, un groupe alcoxy en C₁à C₅ facultativement substitué par un ou plusieurs atomes d'halogène, un groupe alkylthio en C₁ à C₅ facultativement substitué par un ou plusieurs atomes d'halogène et un atome d'halogène ; et X⁵ représente un atome d'oxygène ou un atome de soufre.

4. Composé malononitrile selon la revendication 1, qui est représenté par la formule (XII) : 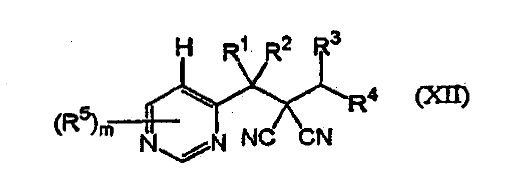
dans laquelle R¹ représente un groupe alkyle en C₁ à C₅ facultativement substitué par un ou plusieurs atomes d'halogène, un groupe alcényle en C₂ à C₅ facultativement substitué par un ou plusieurs atomes d'halogène, un groupe alcynyle en C₂ à C₅ facultativement substitué par un ou plusieurs atomes d'halogène ou un atome d'hydrogène ;
R² représente un groupe alkyle en C₁ à C₅ facultativement substitué par un ou plusieurs atomes d'halogène, un groupe alcoxy en C₁ à C₅ facultativement substitué par un ou plusieurs atomes d'halogène, un groupe alcényle en C₂ à C₅ facultativement substitué par un ou plusieurs atomes d'halogène, un groupe alcynyle en C₂ à C₅ facultativement substitué par un ou plusieurs atomes d'halogène, un groupe cyano ou un atome d'hydrogène ;
chacun de R³ et R⁴ représente un groupe alkyle en C₁ à C₅ facultativement substitué par un ou plusieurs atomes d'halogène, un groupe alcényle en C₂ à C₅ facultativement substitué par un ou plusieurs atomes d'halogène, un groupe alcynyle en C₂ à C₅ facultativement substitué par un ou plusieurs atomes d'halogène, un groupe cycloalkyle en C₃ à C₅ facultativement substitué par un ou plusieurs atomes d'halogène, un groupe cycloalcényle en C₅ à C₆ facultativement substitué par un ou plusieurs atomes d'halogène ou un atome d'hydrogène,
ou représente un groupe alcanediyle en C₂ à C₆ facultativement substitué par un ou plusieurs atomes d'halogène ou un groupe alcènediyle en C₄ à C₆ facultativement substitué par un ou plusieurs atomes d'halogène dans chacun desquels R³ et R⁴ se couplent l'un à l'autre aux extrémités de celui-ci ;
R⁵ représente un atome d'halogène, un groupe cyano, un groupe nitro, un groupe hydroxyle, un groupe mercapto, un groupe formyle, un groupe SF₅, un groupe carboxyle, un groupe alkyle en C₁ à C₅ facultativement substitué par un ou plusieurs atomes d'halogène, un groupe alcényle en C₂ à C₅ facultativement substitué par un ou plusieurs atomes d'halogène, un groupe alcynyle en C₂ à C₅ facultativement substitué par un ou plusieurs atomes d'halogène, un groupe cycloalkyle en C₃ à C₅ facultativement substitué par un ou plusieurs atomes d'halogène, un groupe alcoxy en C₁ à C₅ facultativement substitué par un ou plusieurs atomes d'halogène, un groupe alcényloxy en C₃ à C₆ facultativement substitué par un ou plusieurs atomes d'halogène, un groupe alcynyloxy en C₃ à C₆ facultativement substitué par un ou plusieurs atomes d'halogène, un groupe alkylthio en C₁ à C₅ facultativement substitué par un ou plusieurs atomes d'halogène, un groupe alcénylthio en C₃ à C₅ facultativement substitué par un ou plusieurs atomes d'halogène, un groupe alcynylthio en C₃ à C₅ facultativement substitué par un ou plusieurs atomes d'halogène, un groupe alkylsulfinyle en C₁ à C₅ facultativement substitué par un ou plusieurs atomes d'halogène, un groupe alkylsulfonyle en C₁ à C₅ facultativement substitué par un ou plusieurs atomes d'halogène, un groupe alkylcarbonyle en C₂ à C₆ facultativement substitué par un ou plusieurs atomes d'halogène, un groupe alcoxycarbonyle en C₂ à C₅ facultativement substitué par un ou plusieurs atomes d'halogène, un groupe désigné par -NR¹⁰R¹¹, un groupe désigné par -C(=X⁵)NR¹²R¹³ ou un groupe désigné par -C(=NOR¹⁴)R¹⁵,
chacun de R¹⁰ et R¹¹ représente un groupe alkyle en C₁ à C₅ facultativement substitué par un ou plusieurs atomes d'halogène, un groupe alcényle en C₃ à C₅ facultativement substitué par un ou plusieurs atomes d'halogène, un groupe alcynyle en C₃ à C₅ facultativement substitué par un ou plusieurs atomes d'halogène, un groupe cycloalkyle en C₃ à C₅ facultativement substitué par un ou plusieurs atomes d'halogène, un groupe alkyle en C₁ à C₃ (alcoxy en C₁ à C₅ facultativement substitué par un ou plusieurs atomes d'halogène), un groupe alkylsulfonyle en C₁ à C₅ facultativement substitué par un ou plusieurs atomes d'halogène, un groupe alkylcarbonyle en C₂ à C₆ facultativement substitué par un ou plusieurs atomes d'halogène, un groupe alcoxycarbonyle en C₂ à C₅ facultativement substitué par un ou plusieurs atomes d'halogène ou un atome d'hydrogène,
ou représente un groupe alcanediyle en C₂ à C₆ facultativement substitué par un ou plusieurs atomes d'halogène ou un groupe alcènediyle en C₄ à C₆ facultativement substitué par un ou plusieurs atomes d'halogène dans chacun desquels R¹⁰ et R¹¹ sont couplés l'un à l'autre aux extrémités de celui-ci ; chacun de R¹² et R¹³ représente un groupe alkyle en C₁ à C₅ facultativement substitué par un ou plusieurs atomes d'halogène, un groupe alcényle en C₃ à C₅ facultativement substitué par un ou plusieurs atomes d'halogène, un groupe alcynyle en C₃ à C₅ facultativement substitué par un ou plusieurs atomes d'halogène, un groupe cycloalkyle en C₃ à C₅ facultativement substitué par un ou plusieurs atomes d'halogène, un groupe désigné par -(CH₂)ₙQ¹ ou un atome d'hydrogène,
ou représente un groupe alcanediyle en C₂ à C₆ facultativement substitué par un ou plusieurs atomes d'halogène ou groupe alcènediyle en C₄ à C₆ facultativement substitué par un ou plusieurs atomes d'halogène dans chacun desquels R¹² et R¹³ sont couplés l'un avec l'autre aux extrémités de celui-ci ;
chacun de R¹⁴ et R¹⁵ représente un groupe alkyle en C₁ à C₅ facultativement substitué par un ou plusieurs atomes d'halogène, un groupe alcényle en C₃ à C₅ facultativement substitué par un ou plusieurs atomes d'halogène, un groupe alcynyle en C₃ à C₅ facultativement substitué par un ou plusieurs atomes d'halogène, un groupe cycloalkyle en C₃ à C₅ facultativement substitué par un ou plusieurs atomes d'halogène, un groupe désigné par -(CH₂)ₙQ² ou un atome d'hydrogène ; chacun de Q¹ et Q² représente un groupe phényle qui peut être substitué par un groupe choisi dans le groupe constitué d'un groupe alkyle en C₁ à C₅ facultativement substitué par un ou plusieurs atomes d'halogène, un groupe alcoxy en C₁ à C₅ facultativement substitué par un ou plusieurs atomes d'halogène, un groupe alkylthio en C₁ à C₅ facultativement substitué par un ou plusieurs atomes d'halogène et un atome d'halogène ;
X⁵ représente un atome d'oxygène ou un atome de soufre ;
et m représente un entier de 0 à 2.

5. Composé malononitrile selon la revendication 1, qui est représenté par la formule (XIII) : 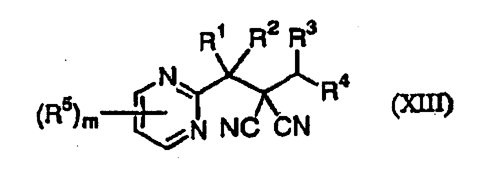 dans laquelle R¹, R², R³, R⁴ et R⁵ ont la même signification que décrite dans la revendication 4, et m représente un entier de 0 à 3.

6. Composé malononitrile selon la revendication 1, qui est représenté par la formule (XIV) : 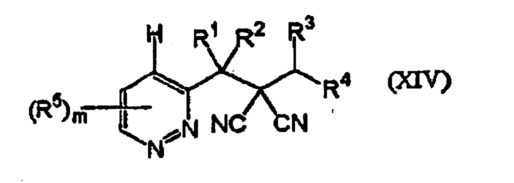 dans laquelle R¹, R², R³, R⁴, R⁵ et m ont la même signification décrite dans la revendication 4.

7. Composé malononitrile selon la revendication 1, qui est représenté par la formule (XV) : 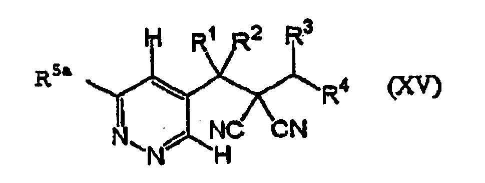 dans laquelle R¹, R², R³, R⁴ et R^{5a} ont la même signification décrite dans la revendication 3.

8. Composition de pesticide comprenant le composé malononitrile selon l'une quelconque des revendications 1 à 7 en tant qu'ingrédient actif et entraîneur inerte.

9. Procédé de protection contre les organismes nuisibles excluant un procédé pour le traitement du corps humain ou animal par thérapie comprenant l'application d'une quantité efficace du composé malononitrile selon l'une quelconque des revendications 1 à 7 à des organismes nuisibles ou à un habitat d'organismes nuisibles.

10. Utilisation du composé malononitrile selon l'une quelconque des revendications 1 à 7 pour la préparation d'un médicament vétérinaire pour un contrôle systémique ou non systémique de parasites.

11. Composé malononitrile comme défini selon l'une quelconque des revendications 1 à 7, pour l'utilisation dans la thérapie d'animaux infestés d'organismes nuisibles.
